(19) Europäisches Patentamt
European Patent Office
Office européen des brevets

(11) **EP 3 838 280 A1**

(12) **EUROPEAN PATENT APPLICATION**

(43) Date of publication:
**23.06.2021 Bulletin 2021/25**

(51) Int Cl.:
*A61K 35/50* (2015.01)    *A61K 35/12* (2015.01)
*A61P 17/02* (2006.01)    *C07K 14/78* (2006.01)

(21) Application number: **20204692.6**

(22) Date of filing: **06.12.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2012 US 201261734665 P**

(62) Document number(s) of the earlier application(s) in accordance with Art. 76 EPC:
**13861269.2 / 2 928 481**

(71) Applicant: **Celularity Inc.**
**Florham Park, NJ 07932 (US)**

(72) Inventors:
• **HARIRI, Robert, J.**
**Bernardsville, NJ New Jersey 07924 (US)**

• **GURNEY, Jodi, P.**
**New Jersey, 07932 (US)**
• **BHATIA, Mohit, B.**
**Manalapan, NJ New Jersey 07726 (US)**
• **HOFGARTNER, Wolfgang**
**Florham Park, NJ New Jersey 07932 (US)**

(74) Representative: **Mewburn Ellis LLP**
**Aurora Building**
**Counterslip**
**Bristol BS1 6BX (GB)**

Remarks:
•This application was filed on 29.10.2020 as a divisional application to the application mentioned under INID code 62.
•Claims filed after the date of receipt of the application (Rule 68(4) EPC).

(54) **TREATING ORAL LESIONS USING PLACENTAL EXTRACELLULAR MATRIX**

(57)    Provided herein are uses of compositions comprising extracellular matrix (ECM) and compositions comprising ECM components in the treatment of non-dental oral lesions.

FIG. 1

EP 3 838 280 A1

## Description

This application claims priority to U.S. Provisional Patent Application No. 61/734,665, filed December 7, 2012, the disclosure of which is herein incorporated by reference in its entirety.

## 1. FIELD

Provided herein are uses of compositions comprising extracellular matrix (ECM) and compositions comprising ECM components in the treatment of non-dental oral lesions.

## 2. BACKGROUND

Collagen is an extracellular matrix protein component that forms many structures in the body including tendons, bones, teeth and sheets that support skin and internal organs. There remains a need in the art for collagen compositions for the treatment of oral lesions.

## 3. SUMMARY

In one aspect, provided herein are methods of treating individuals having an oral lesion, *e.g.*, an oral lesion not caused by a dental procedure, comprising administering to said individual a composition comprising extracellular matrix (ECM), e.g., human placental ECM, or a composition comprising one or more ECM components. In certain embodiments, the composition is administered directly to said oral lesion. In other embodiments, the composition is administered adjacent to or at the periphery of at least a part of the oral lesion. In certain embodiments, the composition is administered as a paste. In certain other embodiments, the composition is administered in the form of a spray or aerosol. In certain other embodiments, the composition is administered in the form of a solution, e.g., in a mouthwash. In certain other embodiments, the composition is administered as a sheet or patch.

In one embodiment, the oral lesion is caused by or associated with graft-versus-host disease. In another embodiment, the oral lesion is, results from, or is associated with desquamation, e.g., is a desquamating oral disorder. In another specific embodiment, the oral lesion is an aphthous ulcer. In a specific embodiment, the aphthous ulcer is caused by, or is a part of, Behçet's disease. In another specific embodiment, the aphthous ulcer is idiopathic.

In certain embodiments, said individual having said oral lesion is undergoing, or has undergone, hematopoietic stem cell therapy. In another specific embodiment, said individual is receiving, or has received, a bone marrow transplant. In a more specific embodiments, said hematopoietic stem cell therapy comprises partial or complete hematopoietic ablation. In a specific embodiment, said ablation comprises partial or full-body radiation. In another specific embodiment, the individual having the oral lesion is undergoing, or has undergone, radiotherapy to the head or neck.

In another embodiment, the oral lesion is caused by or associated with chemotherapy, e.g., chemotherapy that has been administered to the individual to treat a tumor, blood cancer, or other type of cancer. In a specific embodiment, the oral lesion is caused by or associated with use of a chemotherapy drug, e.g., an alkylating agent, for example a nitrogen mustard alkylating agent, such as melphalan, by the individual having the oral lesion. In another specific embodiment, the oral lesion is post-chemotherapy oral mucositis or chemotherapy-induced oral mucositis. In another specific embodiment, the oral lesion is, or is diagnosed as, aphthous stomatitis, *e.g.*, idiopathic aphthous stomatitis. In a specific embodiment, the oral lesion is caused by or associated with use of an mTOR (mammalian target of rapamycin) inhibitor by the individual having the oral lesion. In another specific embodiment, the oral lesion is caused by or associated with use of 5-fluorouracil by the individual having the oral lesion. In another specific embodiment, development of said oral lesion in said individual, wherein said individual is receiving or has received a course therapy, *e.g.*, chemotherapy, has caused a premature termination of said course of therapy. In this context, "premature termination" means termination of the course of therapy prior to what has been prescribed for said individual, partially or wholly as a result of said oral lesion.

In another embodiment, the oral lesion is caused by or associated with administration of an antibody to said individual. In certain specific embodiments, the antibody is an anti-CD20 antibody. In a more specific embodiment, the antibody is rituximab (e.g., RITUXAN®), ofatumumab (e.g., ARZERRA®), veltuzumab or ocrelizumab. In another specific embodiment, the antibody is an anti-tumor necrosis factor antibody. In more specific embodiments, the antibody is adalimumab (e.g., HUMIRA®), etanercept (e.g., ENBREL®), infliximab (e.g., REMICADE®), certolizumab pegol (e.g., CIMZIA®), natalizumab (e.g., TYSABRI®) or golimumab (e.g., SIMPONI®). In another specific embodiment, development of said oral lesion in said individual, wherein said individual is receiving or has received a course antibody therapy has caused a premature termination of said course of antibody therapy. In this context, "premature termination" means termination of the course of antibody therapy prior to what has been prescribed for said individual, partially or wholly as a result of said oral lesion.

In one embodiment, the individual having an oral lesion is diagnosed or evaluated using the World Health Organization Oral Toxicity (WHO-OT) score. In specific embodiments, administration of said ECM to said individual results in a reduction in the WHO-OT score from Grade 4 to Grade 3, from Grade 3 to Grade 2, from Grade 2 to Grade 1, from Grade 4 to Grade 2, from Grade 4 to Grade 1, or from Grade 3 to Grade 1, *e.g.*, within 1, 2, 3, 4, 5, 6, or 7 days post-administration.

In another embodiment, the individual having an oral lesion is diagnosed or evaluated using the National Cancer Institute Common Toxicity Criteria (NCI-CTC) for Oral Mucositis score. In specific embodiments, administration of said ECM to said individual results in a reduction in the NCI-CTC score (for stomatitis/pharyngitis [oral/pharyngeal mucositis]) from Grade 4 to Grade 3, from Grade 3 to Grade 2, from Grade 2 to Grade 1, from grade 1 to Grade 0, from Grade 4 to Grade 2, from Grade 4 to Grade 1, from Grade 4 to Grade 0, from Grade 3 to Grade 1, from Grade 3 to Grade 0, or from Grade 2 to Grade 0, *e.g.*, within 1, 2, 3, 4, 5, 6, or 7 days post-administration.

In another embodiment, the individual having an oral lesion is diagnosed or evaluated using the Oral Mucositis Assessment Scale (OMAS), wherein said OMAS comprises a mean mucositis score, a weighted mean mucositis score, an extent of mucositis score, and a worst site score. In specific embodiments, administration of said ECM to said individual results in a reduction of the mean mucositis score, the weighted mean mucositis score, the extent of mucositis score, or the worst site score of at least 1, at least 2, at least 3, at least 4, or at least 5 points. *See* Sonis et al., Cancer 85(10):2103-2113 (1999), *e.g.,* within 1, 2, 3, 4, 5, 6, or 7 days post-administration.

In another embodiment, the individual having an oral lesion is diagnosed or evaluated using the Western Consortium for Cancer Nursing Research (WCCNR) score. In specific embodiments, administration of said ECM to said individual results in a reduction in the ACCRN score from Stage 3 to Stage 2, from Stage 3 to Stage 1, from Stage 3 to Stage 0, from stage 2 to Stage 1, from Stage 2 to Stage 0, or from stage 1 to Stage 0, *e.g.*, within 1, 2, 3, 4, 5, 6, or 7 days post-administration.

In another embodiment, the individual having an oral lesion is diagnosed or evaluated using the Radiation Therapy Oncology Group (RTOG) score (for mucous membranes). In specific embodiments, administration of said ECM to said individual results in a reduction in the RTOG score (for mucositis) from Grade 4 to Grade 3, from Grade 3 to Grade 2, from Grade 2 to Grade 1, from grade 1 to Grade 0, from Grade 4 to Grade 2, from Grade 4 to Grade 1, from Grade 4 to Grade 0, from Grade 3 to Grade 1, from Grade 3 to Grade 0, or from Grade 2 to Grade 0, e.g., within 1, 2, 3, 4, 5, 6, or 7 days post-administration.

In another embodiment, the oral lesion is caused by, or is associated with, osteonecrosis of the jaw in said individual. In certain specific embodiments, said individual is receiving, or has received, bisphosphonate therapy.

In certain embodiments, the compositions used in the methods described herein comprise ECM, e.g., ECM derived from human placenta. In certain embodiments, the compositions used in the methods described herein consist of ECM, e.g., ECM derived from human placenta. In certain embodiments, the compositions used in the methods described herein comprise one or more ECM components, such as collagen, e.g., telopeptide collagen (i.e., collagen that comprises one or more telopeptide regions), fibronectin, laminin, elastin, and/or glycosaminoglycans. In certain embodiments, the compositions used in the methods described herein consist of one or more ECM components, such as collagen, e.g., telopeptide collagen (i.e., collagen that comprises one or more telopeptide regions), fibronectin, laminin, elastin, and/or glycosaminoglycans, i.e., the compositions consist of one or more isolated/purified ECM components, e.g., collagen.

In one embodiment, the compositions used in the methods described herein comprise collagen (e.g., telopeptide collagen) and are substantially free of cellular debris, subcellular debris, other ECM proteins (e.g., fibronectin and/or laminin), cytokines, and/or growth factors. In certain embodiments, the compositions used in the methods described herein comprise at least 75%, 80%, 85%, 90%, 95%, 96%, 97%, 98%, or 99% total collagen, as compared to the total amount of protein in the composition (e.g., by dry weight). In certain embodiments, the compositions used in the methods described herein comprise between 75%-80%, 80%-85%, 85%-90%, 90%-95%, 95%-98%, or 98%-99.5% total collagen, as compared to the total amount of protein in the composition (e.g., by dry weight).

In certain embodiments, the compositions provided herein comprise collagen but substantially lack other ECM components, such as laminin and fibronectin. In certain embodiments, the collagen comprising compositions used in the methods described herein comprise less than 1%, less than 0.5%, less than 0.1%, less than 0.05%, or less than 0.01% laminin, or comprise between 0.01%-0.05%, 0.05%-0.1%, 0.1%-0.5%, or 0.5%-1.0% laminin, as compared to the total amount of protein in the composition (e.g., by dry weight). In certain embodiments, the collagen comprising compositions provided herein comprise less than 1%, less than 0.5%, less than 0.1%, less than 0.05%, or less than 0.01% fibronectin, or comprise between 0.01%-0.05%, 0.05%-0.1%, 0.1%-0.5%, or 0.5%-1.0% fibronectin, as compared to the total amount of protein in the composition (e.g., by dry weight).

In certain embodiments, the compositions used in the methods described herein comprise collagen (e.g., telopeptide collagen) and elastin, but substantially lack other ECM components, such as laminin and fibronectin. In certain embodiments, the compositions used in the methods described herein comprise about 1%, 2%, 3%, 4%, 5%, 6%, 7%, 8%, 9%, 10%, 11%, 12%, 13%, 14%, or 15% elastin or between 1-5%, 5-10%, or 10-15% elastin, and comprise less than 1%, less than 0.5%, less than 0.1%, less than 0.05%, or less than 0.01% laminin, or comprise between 0.01%-0.05%, 0.05%-0.1%, 0.1%-0.5%, or 0.5%-1.0% laminin; and/or less than 1%, less than 0.5%, less than 0.1%, less than 0.05%, or less than 0.01% fibronectin, or comprise between 0.01%-0.05%, 0.05%-0.1%, 0.1%-0.5%, or 0.5%-1.0% fibronectin, as compared to the total amount of protein in the composition (e.g., by dry weight). In a specific embodiment, the compositions used in the methods described herein comprise greater than 80% collagen, between 10-15% elastin, less than 0.01% laminin, and less than 0.01% fibronectin, as compared to the total amount of protein in the composition (e.g., by dry weight).

In certain embodiments, the ECM (or ECM components) in the compositions used in the methods described herein is detergent-treated. In certain embodiments, the ECM (or ECM components) in the compositions used in the methods described herein is base-treated. In certain embodiments, the ECM (or ECM components) in the compositions used in the methods described herein is detergent-treated and base-treated. In certain embodiments, the ECM (or ECM components) in the compositions used in the methods described herein is detergent-treated, but not base-treated. In certain embodiments, the ECM (or ECM components) in the compositions used in the methods described herein is base-treated, but not detergent-treated.

In certain embodiments, the compositions used in the methods described herein further comprise a plurality of stem cells, e.g., a therapeutically-effective amount of stem cells. In various embodiments, the stem cells are embryonic stem cells, embryonic germ cells, mesenchymal stem cells, bone marrow-derived stem cells, placental stem cells, hematopoietic progenitor cells (e.g., hematopoietic stem cells from peripheral blood, fetal blood, placental blood, umbilical cord blood, placental perfusate, etc.), somatic stem cells, neural stem cells, hepatic stem cells, pancreatic stem cells, endothelial stem cells, cardiac stem cells, muscle stem cells, adipose stem cells, and the like.

In specific embodiments, the stem cells are placental stem cells. In a more specific embodiment, said placental stem cells are CD34- and/or CD200+. In a specific embodiment, the placental stem cells are CD34-, CD10+, CD105+ and CD200+. In certain specific embodiments, the placental stem cells express one or more of CD10, CD73, CD105, CD200, and/or OCT-4, and do not express one or more of CD34, CD38, CD45, and/or HLA-G. In certain other specific embodiments, the placental stem cells can also express HLA-ABC (MHC-1) and do not express HLA-DR. In another specific embodiment, the placental stem cells are CD200+ and HLA-G-. In another specific embodiment, the placental stem cells are CD73+, CD105+, and CD200+. In another specific embodiment, the placental stem cells are CD200+ and OCT-4+. In another specific embodiment, the placental stem cells are CD73+, CD105+ and HLA-G-. In another specific embodiment, the placental stem cells are CD73+ and CD105+, and, when in a population of placental cells, facilitate formation of one or more embryoid-like bodies under conditions that allow formation of embryoid-like bodies. In another specific embodiment, the placental stem cells are OCT-4+ and, when in a population of placental cells, facilitate formation of one or more embryoid-like bodies in a population of isolated placental stem cells comprising said stem cell when cultured under conditions that allow formation of embryoid-like bodies.

In another specific embodiment, the stem cells are adhered to the composition, e.g., when said composition is in the form of a sheet, a patch, or a paste. In a specific embodiment of any of the above embodiments, the stem cells secrete IL-6, IL-8 and/or MCP-1 (monocyte chemotactic protein-1) when contacted with the composition, e.g., when the stem cells adhere to the composition.

In another aspect, provided herein are kits for administering compositions comprising ECM and/or ECM components to an individual having an oral lesion. The kits typically comprise compositions comprising ECM and/or ECM components in a package convenient for distribution to a practitioner of skill in the art.

## 4. BRIEF DESCRIPTION OF THE FIGURES

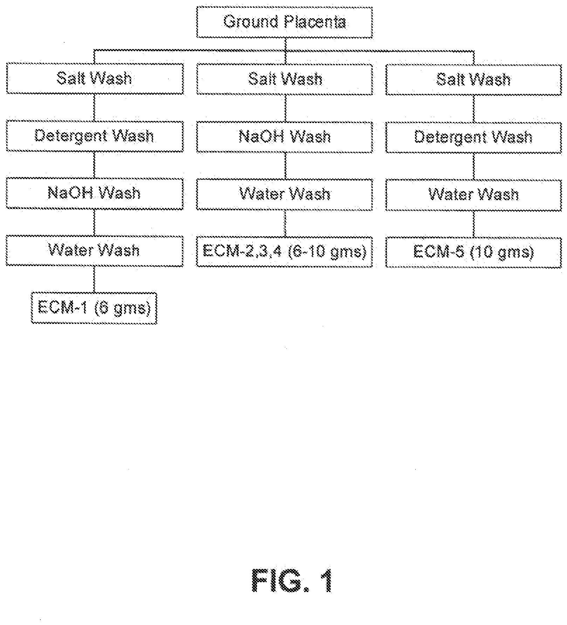

FIG. 1: Flow chart representation of exemplary methods for isolating extracellular matrix (ECM).

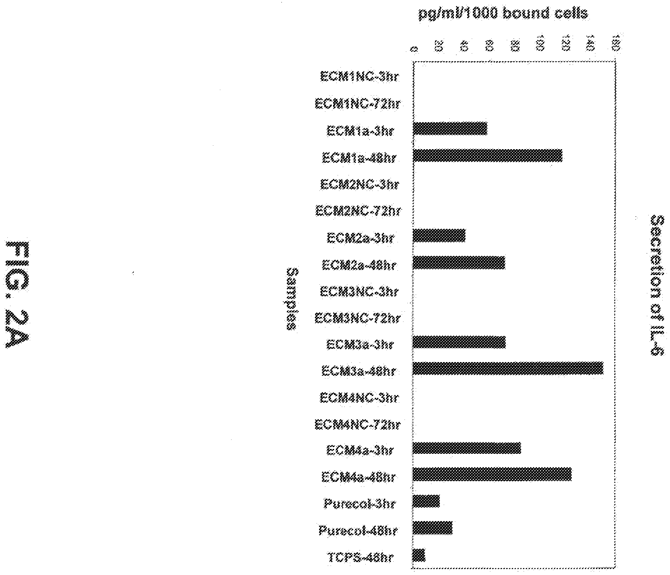

FIG. 2A: Secretion of IL-6 from placental stem cells grown on compositions comprising ECM prepared by various methods. Abscissa: Specific growth conditions by type of composition and time of growth of the cells on the compositions. Ordinate: picograms per milliliter per 1000 ECM-bound cells. NC=no cells. Purecol=purified collagen. TCPS=tissue culture polystyrene.

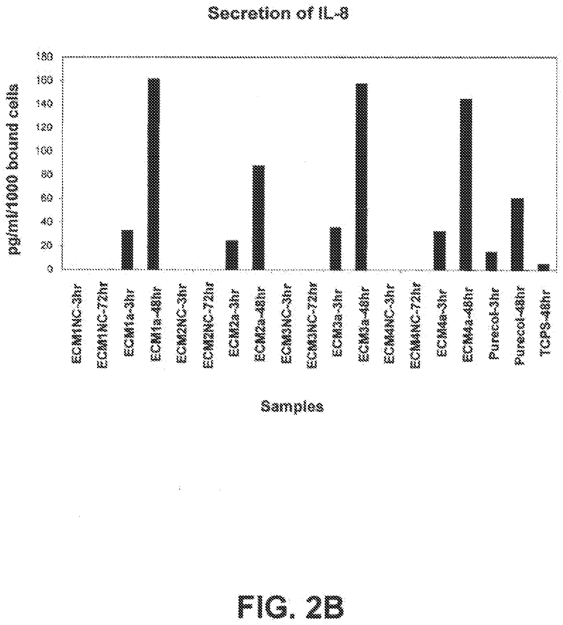

FIG. 2B: Secretion of IL-8 from placental stem cells grown on compositions comprising ECM prepared by various methods. Abscissa: Specific growth conditions by type of composition and time of growth of the cells on the compositions Ordinate: picograms per milliliter per 1000 ECM-bound cells. NC=no cells. Purecol=purified collagen. TCPS=tissue culture polystyrene.

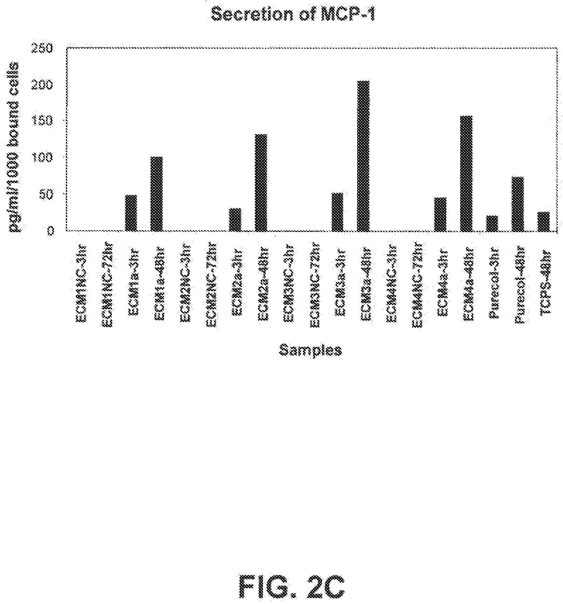

FIG. 2C: Secretion of MCP-1 from placental stem cells grown on compositions comprising ECM prepared by various methods. Abscissa: Specific growth conditions by type of composition and time of growth of the cells on the compositions. Ordinate: picograms per milliliter per 1000 ECM-bound cells. NC=no cells. Purecol=purified collagen. TCPS=tissue culture polystyrene.

## 5. DETAILED DESCRIPTION

### 5.1. Methods of Treating Oral Lesions Using ECM

The compositions comprising ECM and/or ECM components, e.g. placental ECM or components thereof, used in the methods described herein are, in one aspect, used to treat an oral lesion, wherein said lesion is not caused by a dental procedure or by oral surgery. In certain embodiments, provided herein is a method of treating a subject who has an oral lesion comprising administering to the individual, e.g., administering to the oral lesion, a therapeutically-effective amount of a composition described herein. In this context, "therapeutically effective amount" means an amount of a composition comprising ECM and/or ECM components that acts to reduce or eliminate at least one symptom or aspect of the oral lesion. For example, the composition can be administered to a subject in order to repair the lesion, or can be administered as a palliative, e.g., to reduce pain or inflammation caused by or associated with the oral lesion. As used herein, the terms "subject" and "individual" refer to animals such as mammals, including, but not limited to, primates (e.g., humans), cows, sheep, goats, horses, dogs, cats, rabbits, rats, mice and the like. In certain embodiments, the subjects/individuals are human.

In certain embodiments, a composition comprising ECM and/or ECM components is administered directly to an oral lesion of a subject. In other embodiments, a composition comprising ECM and/or ECM components is administered adjacent to or at the periphery of at least a part of an oral lesion of a subject. Such administrations can be, for example, by placement of a composition comprising ECM and/or ECM components that has been formulated as a sheet over at least a portion, or the whole of, the oral lesion. In certain embodiments, the compositions used in the methods described herein are administered to oral lesions as a paste. In certain other embodiments, the compositions used in the methods described herein are administered to oral lesions in the form of a spray or aerosol. In certain other embodiments, the compositions used in the methods described herein are administered to oral lesions in the form of a solution, e.g., in a mouthwash. In certain other embodiments, the compositions used in the methods described herein are administered to oral lesions as a matrix, gel, sheet, or patch.

In a specific embodiment, the oral lesion treated in accordance with the methods described herein is, results from, or is associated with desquamation, e.g., is a desquamating oral disorder. In another specific embodiment, the oral lesion treated in accordance with the methods described herein is an aphthous ulcer. In a specific embodiment, the aphthous ulcer is caused by, or is a part of, Behçet's disease. In another specific embodiment, the aphthous ulcer is idiopathic.

In certain embodiments, a subject having an oral lesion treated in accordance with the methods described herein is undergoing, or has undergone, hematopoietic stem cell therapy. In another specific embodiment, said subject is receiving, or has received, a bone marrow transplant. In certain embodiments, the oral lesion is caused by or is associated with graft-versus-host disease. In a specific embodiment, the oral lesion is caused by or associated with use of a chemotherapy drug, e.g., an alkylating agent, for example a nitrogen mustard alkylating agent, such as melphalan, by the individual having the oral lesion. In a more specific embodiment, said hematopoietic stem cell therapy or bone marrow transplant comprises partial or complete hematopoietic ablation. In a specific embodiment, said ablation comprises partial or full-body radiation.

In another embodiment, a subject having an oral lesion treated in accordance with the methods described herein is undergoing, or has undergone, radiotherapy to the head or neck.

In another embodiment, an oral lesion treated in accordance with the methods described herein is caused by or associated with chemotherapy, e.g., chemotherapy that has been administered to the individual to treat a tumor, blood cancer, or other type of cancer. In a specific embodiment, the oral lesion is caused by post-chemotherapy oral mucositis or chemotherapy-induced oral mucositis. In another specific embodiment, the oral lesion is, or is diagnosed as, aphthous stomatitis, *e.g.*, idiopathic aphthous stomatitis. In specific embodiments, in which the oral lesion is caused by or associated with chemotherapy, e.g., is caused by or associated with use of a chemotherapeutic agent, the chemotherapeutic agent is, e.g., an alkylating agent (e.g., busulfan, cisplatin, carboplatin, cyclophosphamide, dacarbazine, ifosfamide, mechlorethamine or melphalan); an anti-metabolite (e.g., 5-fluorouracil, methotrexate, gemcitabine, cytarabine, or fludarabine); antibiotics having an antitumor effect (e.g., bleomycin, dactinomycin, daunorubicin, doxorubicin, or idarubicin); or mitotic inhibitors (e.g., paclitaxel, docetaxel, etoposide, vinblastine, vincristine or vinorelbine). In a specific embodiment, the oral lesion is caused by or associated with use of an mTOR ("mammalian target of rapamycin") inhibitor by the individual having the oral lesion. In another specific embodiment, the oral lesion is caused by or associated with use of 5-fluorouracil by the individual having the oral lesion.

In another embodiment, an oral lesions treated in accordance with the methods described herein is caused by or associated with administration of an antibody to a subject. In certain specific embodiments, the antibody is an anti-CD20 antibody. In a more specific embodiment, the antibody is rituximab (e.g., RITUXAN®), ofatumumab (e.g., ARZERRA®), veltuzumab or ocrelizumab. In another specific embodiment, the antibody is an anti-tumor necrosis factor antibody. In more specific embodiments, the antibody is adalimumab (e.g., HUMIRA®), etanercept (e.g., ENBREL®), infliximab (e.g., REMICADE®), certolizumab pegol (e.g., CIMZIA®), natalizumab (e.g., TYSABRI®) or golimumab (e.g., SIMPONI®). In another specific embodiment, development of said oral lesion in said individual, wherein said individual is receiving or has received a course antibody therapy has caused a premature termination of said course of antibody therapy. In this context, "premature termination" means termination of the course of antibody therapy prior to what has been prescribed for said individual, partially or wholly as a result of said oral lesion.

In certain embodiments, development of an oral lesion in a subject treated in accordance with the methods described herein, wherein said subject is receiving or has received a course of therapy, *e.g.*, radiotherapy, chemotherapy, or antibody administration, has caused, or is expected to cause, a premature termination of said course of therapy. In this context, "premature termination" means termination of the course of therapy prior to what has been prescribed for said subject, partially or wholly as a result of said oral lesion.

In one embodiment, the individual having an oral lesion is diagnosed or evaluated using the World Health Organization Oral Toxicity (WHO-OT) score. In specific embodiments, administration of said ECM to said individual results in a reduction in the WHO-OT score from Grade 4 to Grade 3, from Grade 3 to Grade 2, from Grade 2 to Grade 1, from Grade 4 to Grade 2, from Grade 4 to Grade 1, or from Grade 3 to Grade 1, *e.g.*, within 1, 2, 3, 4, 5, 6, or 7 days post-administration.

In another embodiment, an individual having an oral lesion treated in accordance with the methods described herein is diagnosed or evaluated using the National Cancer Institute Common Toxicity Criteria (NCI-CTC) for Oral Mucositis score. In specific embodiments, administration of a composition described herein (i.e., a composition comprising ECM (e.g., placental ECM) or ECM components) to said individual results in a reduction in the NCI-CTC score (for stomatitis/pharyngitis [oral/pharyngeal mucositis]) from Grade 4 to Grade 3, from Grade 3 to Grade 2, from Grade 2 to Grade 1, from grade 1 to Grade 0, from Grade 4 to Grade 2, from Grade 4 to Grade 1, from Grade 4 to Grade 0, from Grade 3 to Grade 1, from Grade 3 to Grade 0, or from Grade 2 to Grade 0, *e.g.*, within 1, 2, 3, 4, 5, 6, or 7 days post-administration.

In another embodiment, an individual having an oral lesion treated in accordance with the methods described herein is diagnosed or evaluated using the Oral Mucositis Assessment Scale (OMAS), wherein said OMAS comprises subscores: a mean mucositis score, a weighted mean mucositis score, an extent of mucositis score, and a worst site score. In specific embodiments, administration of a composition described herein (i.e., a composition comprising ECM (e.g., placental ECM) or ECM components) to said individual results in a reduction of one or more of the mean mucositis score, the weighted mean mucositis score, the extent of mucositis score, or the worst site score of at least 1, at least 2, at least 3, at least 4, or at least 5 points, e.g., within 1, 2, 3, 4, 5, 6, or 7 days post-administration. *See* Sonis et al., Cancer 85(10):2103-2113 (1999).

In another embodiment, an individual having an oral lesion treated in accordance with the methods described herein is diagnosed or evaluated using the Western Consortium for Cancer Nursing Research (WCCNR) score. In specific embodiments, administration of a composition described herein (i.e., a composition comprising ECM (e.g., placental ECM) or ECM components) to said individual results in a reduction in the ACCRN score from Stage 3 to Stage 2, from Stage 3 to Stage 1, from Stage 3 to Stage 0, from stage 2 to Stage 1, from Stage 2 to Stage 0, or from stage 1 to Stage 0, *e.g.*, within 1, 2, 3, 4, 5,6, or 7 days post-administration.

In another embodiment, an individual having an oral lesion treated in accordance with the methods described herein is diagnosed or evaluated using the Radiation Therapy Oncology Group (RTOG) score (for mucous membranes). In specific embodiments, administration of a composition described herein (i.e., a composition comprising ECM (e.g., placental ECM) or ECM components) to said individual results in a reduction in the RTOG score (for mucositis) from Grade 4 to Grade 3, from Grade 3 to Grade 2, from Grade 2 to Grade 1, from grade 1 to Grade 0, from Grade 4 to Grade 2, from Grade 4 to Grade 1, from Grade 4 to Grade 0, from Grade 3 to Grade 1, from Grade 3 to Grade 0, or from Grade 2 to Grade 0, *e.g.*, within 1, 2, 3, 4, 5, 6, or 7 days post-administration.

In another embodiment, an oral lesion treated in accordance with the methods described herein is caused by, or is associated with, osteonecrosis of the jaw in a subject. In a specific embodiment, said subject is receiving, or has received, bisphosphonate therapy.

### 5.2. Extracellular Matrix Compositions

Provided herein compositions comprising extracellular matrix (ECM) and/or ECM components, useful in the methods of treatment provided herein, i.e., methods of treating oral lesions. The ECM and ECM components of the compositions used in the methods described herein may, in certain embodiments, be obtained from a mammalian source, e.g., a human, bovine, ovine, sheep, rat source. The ECM and ECM components of the compositions used in the methods described herein be obtained from a marsupial, e.g., a kangaroo. In certain embodiments, the ECM and ECM components of the compositions used in the methods described herein is obtained from a non-mammalian source, e.g., the ECM is obtained from fish.

The ECM and ECM components of the compositions used in the methods described herein can be obtained from any portion of the source from which they are derived. In certain embodiments, the ECM can be obtained from, e.g., bovine skin, calf skin, rat tail, kangaroo tail, or fish skin. In a specific embodiment, the ECM and/or ECM components of the compositions used in the methods described herein is obtained from placenta, e.g., the ECM is bovine placental ECM, ovine placental ECM, or human placental ECM. In a specific embodiment, the ECM and ECM components of the compositions used in the methods described herein are derived from human placenta.

A principal component of ECM, e.g., human placental ECM, is collagen. Accordingly, the compositions comprising ECM used in the methods described herein comprise collagen, e.g., telopeptide collagen and/or atelopeptide collagen. In specific embodiments, the compositions comprising ECM components described herein comprise collagen.

The collagen in the compositions used in the methods described herein can be any type of collagen known to those of skill in the art or a mixture of such collagens. In certain embodiments, the collagen is in the form of a collagen composition that comprises one or more types of collagen. Particular collagens include, for example, type I collagen, type II collagen, type III collagen and type IV collagen. In one embodiment, a collagen comprising composition used in the methods described herein comprises type I collagen and type IV collagen, e.g., the majority of the collagen in the composition is type I and type IV collagen. In certain embodiments, the compositions useful in the methods of treatment provided herein comprise between 1% and 15% type IV collagen, between 2% and 13% type IV collagen, between 3% and 12% type IV collagen or between 4% and 11% type IV collagen by dry weight (i.e., the compositions comprise ECM, wherein the ECM comprises such collagen, and/or the compositions comprise ECM components, one of which is such collagen). In certain embodiments, the compositions useful in the methods of treatment provided herein comprise at least 75%, at least 80%, at least 85%, at least 90%, at least 95%, or at least 99% type I collagen by dry weight (i.e., the compositions comprise ECM, wherein the ECM comprises such collagen, and/or the compositions comprise ECM components, one of which is such collagen). In certain embodiments, the compositions useful in the methods of treatment provided herein comprise between 70% and 95% type I collagen, between 74% and 92% type I collagen or between 80% and 90% type I collagen by dry weight (i.e., the compositions comprise ECM, wherein the ECM comprises such collagen, and/or the compositions comprise ECM components, one of which is such collagen). In certain embodiments, the compositions useful in the methods of treatment provided herein comprise type III collagen, for instance up to 1%, up to 2%, up to 3%, up to 4%, up to 5%, up to 6% or up to 7% type III collagen by dry weight (i.e., the compositions comprise ECM, wherein the ECM comprises such collagen, and/or the compositions comprise ECM components, one of which is such collagen). In certain embodiments, the compositions useful in the methods of treatment provided herein comprise between 2% and 15% type IV collagen, between 70% and 95% type I collagen and up to 6% type III collagen, by dry weight (i.e., the compositions comprise ECM, wherein the ECM comprises such collagen, and/or the compositions comprise ECM components, one of which is such collagen).

In certain embodiments, the collagen comprising compositions used in the methods described herein comprise at least one additional ECM component, e.g., elastin, fibronectin, laminin, and/or glycosaminoglycans. In certain embodiments, the collagen comprising compositions used in the methods described herein lack, or comprise minimal amounts of, one or more components typically associated with the ECM, e.g., the compositions comprising ECM components comprise collagen but lack (or comprise a minimal amount of) one or more of elastin, fibronectin, laminin, and/or glycosaminoglycans. In a specific embodiment, the collagen comprising compositions used in the methods described herein comprise no detectable fibronectin, or no detectable laminin, or no detectable laminin or fibronectin. In a specific embodiment, the collagen comprising compositions used in the methods described herein comprise no detectable glycosaminoglycans.

In a specific embodiment, a composition comprising collagen used in the methods described herein may comprise (i) at least or about 75%, 80%, 85%, 90%, 95%, 96%, 97%, 98%, or 99% total collagen, as compared to the total amount of protein in the composition (e.g., by dry weight); or between 75%-80%, 80%-85%, 85%-90%, 90%-95%, 95%-98%, or 98%-99.5% total collagen, as compared to the total amount of protein in the composition (e.g., by dry weight); (ii) less than 1%, less than 0.5%, less than 0.1%, less than 0.05%, or less than 0.01% laminin, as compared to the total amount of protein in the composition (e.g., by dry weight); or between 0.01%-0.05%, 0.05%-0.1%, 0.1%-0.5%, or 0.5%-1.0% laminin, as compared to the total amount of protein in the composition (e.g., by dry weight); and/or (iii) less than 1%, less than 0.5%, less than 0.1%, less than 0.05%, or less than 0.01% fibronectin, as compared to the total amount of protein in the composition (e.g., by dry weight); or between 0.01%-0.05%, 0.05%-0.1%, 0.1%-0.5%, or 0.5%-1.0% fibronectin, as compared to the total amount of protein in the composition (e.g., by dry weight). In another specific embodiment, the composition comprises at least or about 1%, 2%, 3%, 4%, 5%, 6%, 7%, 8%, 9%, 10%, 11%, 12%, 13%, 14%, or 15% elastin, as compared to the total amount of protein in the composition (e.g., by dry weight); or between 1-5%, 5-10%, or 10-15% elastin, as compared to the total amount of protein in the composition (e.g., by dry weight). In another specific embodiment, the composition comprises about 5% elastin by dry weight. In another specific embodiment, the composition comprises about 10% elastin by dry weight. In another specific embodiment, the composition comprises no more than about 5% elastin by dry weight. In another specific embodiment, the composition comprises more than about 10% elastin by dry weight, e.g., the composition comprises 11%, 12%, 13%, 14%, 15%, or greater than 15% elastin by dry weight.

In certain embodiments, the ECM and ECM components of the compositions used in the methods described herein can be obtained by one of the processes described below.

In certain embodiments, the collagen in the compositions described herein, e.g., the collagen in the ECM of a composition described herein, is cross-linked, e.g., with a cross-linker. In certain embodiments, the cross-linker is glutaraldehyde. See, e.g., U.S. Pat. Nos. 4,852,640, 5,428,022, 5,660,692 and 5,008,116, and in McPherson et al., 1986, J. Biomedical Materials Res. 20:79-92, the contents of which are hereby incorporated by reference in their entirety. Further exemplary cross-linkers and methods of cross-linking collagen are described in U.S. Pat. Nos. 5,880,242 and 6,117,979 and in Zeeman et al., 2000, J Biomed Mater Res. 51(4):541-8, van Wachem et al., 2000, J Biomed Mater Res. 53(1):18-27, van Wachem et al., 1999, J Biomed Mater Res. 47(2):270-7, Zeeman et al., 1999, J Biomed Mater Res. 46(3):424-33, Zeeman et al., 1999, Biomaterials 20(10):921-31, the contents of which are hereby incorporated by reference in their entireties.

In further embodiments, the collagen in the compositions described herein, e.g., the collagen in the ECM of a composition described herein, is cross-linked with 1,4-butanediol diglycidyl ether. In further embodiments, the collagen in the compositions described herein, e.g., the collagen in the ECM of a composition described herein, is cross-linked with genipin, a nontoxic, naturally occurring crosslinking agent. Genipin can be obtained from its parent compound, geniposide, which may be isolated from the fruits of *Gardenia jasminoides.* Genipin may be obtained commercially from Challenge Bioproducts Co., Ltd., 7 Alley 25, Lane 63, TzuChiang St. 404 Taichung Taiwan R.O.C., Tel 886-4-3600852. The use of genipin as a cross-linking reagent is described extensively in U.S. Patent Application Publication No. 2003/0049301, the contents of which are hereby incorporated by reference in their entirety.

The collagen in the compositions described herein, e.g., the collagen in the ECM of a composition described herein, can be cross-linked with a single cross-linker or with a mixture of cross-linkers. In certain embodiments, the ECM in the compositions described herein, or the collagen in a collagen comprising composition described herein (i.e., a composition comprising ECM components, wherein at least one component is collagen) comprises base-treated and/or detergent treated human placental collagen cross-linked with glutaraldehyde. In certain embodiments, the ECM in the compositions described herein, or the collagen in a collagen comprising composition described herein (i.e., a composition comprising ECM components, wherein at least one component is collagen) comprises base-treated, but not detergent treated human placental collagen cross-linked with glutaraldehyde. In certain embodiments, the ECM in the compositions described herein, or the collagen in a collagen comprising composition described herein (i.e., a composition comprising ECM components, wherein at least one component is collagen) comprises detergent-treated, but not base treated human placental collagen cross-linked with glutaraldehyde. In certain embodiments, the ECM in the compositions described herein, or the collagen in a collagen comprising composition described herein (i.e., a composition comprising ECM components, wherein at least one component is collagen) comprises base-treated and detergent treated human placental collagen cross-linked with glutaraldehyde.

The collagen in the compositions described herein, e.g., the collagen in the ECM of a composition described herein, can be cross-linked with any enzyme-mediated crosslinking technique known to those of skill in the art. For instance, the collagen in the compositions described herein, e.g., the collagen in the ECM of a composition described herein, can be cross-linked by transglutaminase, e.g., by the methods described, for example, in Orban et al., 2004, J. Biomedical Materials Res. 68(4):756-62.

**5.3. Processes for Preparation of ECM**

In certain embodiments, the ECM used in the compositions described herein, or from which the ECM components of the compositions comprising ECM components described herein are derived, is prepared from human placenta according to the methods described herein. The placental tissue can be from any part of the placenta including the amnion, whether soluble or insoluble or both, the chorion, the umbilical cord or from the entire placenta. In certain embodiments, the ECM is prepared from whole human placenta without the umbilical cord. In certain other embodiments, the ECM is prepared from whole placenta without the amniotic membrane or umbilical cord.

The placenta from which ECM is obtained is preferably taken as soon as possible after normal delivery, or after cesarean section delivery, of a normal healthy infant. Advantageously, the placenta is collected under aseptic conditions. The placenta, in certain embodiments, is stored for 48 hours from the time of delivery prior to any further treatment. In other embodiments, the placenta is stored for up to 5 days from the time of delivery prior to any further treatment.

The placenta and optionally umbilical cord can be transported from the delivery or birthing room to another location, e.g., a laboratory, for further processing. The placenta can be transported in a sterile, transport device such as a sterile bag or a container, which is optionally thermally insulated. In some embodiments, the placenta is stored at room temperature until further treatment. In other embodiments, the placenta is refrigerated until further treatment, i.e., stored at a temperature of about 2°C to 8°C. The placenta may be stored under sterile conditions for up to 5 days before further treatment. The placenta is preferably handled and processed under aseptic conditions, as known to persons skilled in the art, e.g., in a laboratory can be equipped with an HEPA filtration system (as defined by clean room classification, having a class 1000 or better).

The placenta is preferably exsanguinated, i.e., completely drained of the cord blood remaining after birth, prior to obtaining the ECM. In some embodiments, the placenta is 70% exsanguinated, 80% exsanguinated, 90% exsanguinated, 95% exsanguinated or 99% or greater exsanguinated.

The expectant mother may be screened for known pathogens prior to the time of birth, using standard techniques known to one skilled in the art, for communicable diseases including but not limited to, HIV, HBV, HCV, HTLV, syphilis, CMV, and other viral pathogens known to contaminate placental tissue, e.g., following FDA regulations. The expectant mother may be screened (e.g., a blood sample is taken for diagnostic purposes) within one month of birth, particularly within two weeks of birth, within one week of birth, or at the time of birth. Preferably, only tissues collected from donors who tested negative or non-reactive to the above-mentioned pathogens are used to produce ECM. Advantageously, a thorough paternal and medical and social history of the donor of the placental membrane is obtained, including for example, a detailed family history.

In certain embodiments, the donor is screened using standard serological and bacteriological tests known to persons skilled in the art. For example, donor screening can encompass using standard antigen-detection techniques known to one skilled in the art using, e.g., antibody screen (ATY); alanine amino transferase screening (ALT); Hepatitis Core Antibody (nucleic acid and ELISA); Hepatitis B Surface Antigen; Hepatitis C Virus Antibody; HIV-1 and HIV-2; HTLV-1 and HTLV-2; Syphilis test (RPR); CMV antibody test; and/or Hepatitis C and HIV test. The assays used may be nucleic acid based assays or ELISA based assays as known to one skilled in the art.

Blood from the umbilical cord of the newborn can be tested using standard techniques known to one skilled in the art (See, e.g., Cotorruelo et al., 2002, Clin. Lab. 48(5 6):271 81; Maine et al., 2001, Expert Rev. Mol. Diagn., 1(1):19 29; Nielsen et al., 1987, J. Clin. Microbiol. 25(8): 1406 10). In one embodiment, blood from the umbilical cord of the newborn is tested for bacterial pathogens (including but not limited to gram positive and gram negative bacteria) and/or fungi using standard techniques known to persons skilled in the art. The blood type and Rh factor of the blood of the umbilical cord of the newborn can be determined using standard techniques known to those skilled in the art. In another embodiment, complete blood count (CBC) with differential is obtained from the blood from the umbilical cord of the newborn using standard methods known to one skilled in the art. In yet another embodiment, an aerobic bacterial culture is taken from the blood from the umbilical cord of the newborn, using standard methods known to one skilled in the art. Only tissues collected from donors that have a CBC within a normal limit (e.g., no gross abnormality or deviation from the normal level), test negative for serology and bacteriology, and test negative or non-reactive for infectious disease and contamination are used to produce the ECM.

Once the human placental tissue is obtained, it can, for example, be treated according to the following steps in order to prepare the ECM. Although the following steps are presented in sequential order, one of skill in the art will recognize that the order of several steps can be interchanged. It is assumed that techniques readily apparent to those of skill in the art such as buffer exchange, precipitation, centrifugation, resuspension, dilution and concentration of protein compositions need not be explained in detail. Exemplary preparation is described in the examples below.

Any portion of the placenta, or the entire placenta, can be used in the processes described herein. In certain embodiments, ECM is prepared from whole placenta, or from chorionic or amnionic portions of the placenta.

The umbilical cord may be separated from the placental disc, and the amniotic membrane may be separated from the chorionic membrane. The amniotic membrane may be separated from the chorionic membrane prior to cutting the placental membrane. Separation of the amniotic membrane from the chorionic membrane can be done starting from the edge of the placental membrane, and can be separated from the chorionic membrane using blunt dissection, e.g., with gloved fingers. Following separation of the amniotic membrane from the chorionic membrane and placental disc, the umbilical cord stump may be cut, e.g., with scissors, and detached from the placental disc. In certain embodiments, when separation of the amniotic and chorionic membranes is not possible without tearing the tissue, the amniotic and chorionic membranes may be cut from the placental disc as one piece and then peeled them apart.

The amniotic membrane, chorionic membrane or whole placenta can be stored prior to use in the methods described herein. Exemplary storage techniques are described in U.S. Patent Application Publication Nos. 2004/0048796 and 2003/0187515, the contents of which are hereby incorporated by reference in their entireties.

The placental tissue may be decellularized prior to obtaining the ECM. The placental tissue can be decellularized according to any technique known to those of skill in the art, e.g., techniques described in U.S. Patent Application Publication Nos. 2004/0048796 and 2003/0187515, the contents of which are hereby incorporated by reference in their entireties.

In certain embodiments, the placental tissue is subjected to an osmotic shock, a detergent treatment, and/or a base treatment. Although not intending to be bound by any particular theory of operation, it is believed that the osmotic shock can burst cells in the tissue and thereby facilitating removal of cells, cellular components and blood components. Osmotic shock can be in addition to any clarification step or it can be the sole clarification step.

The osmotic shock can be carried out in any osmotic shock conditions known to those of skill in the art. Such conditions include incubating the tissue in solutions of high osmotic potential, or of low osmotic potential or of alternating high and low osmotic potential. The high osmotic potential solution can be any high osmotic potential solution known to those of skill in the art such as a solution comprising one or more of NaCl (e.g., 0.2M to 1.0M), KCl (e.g., 0.2M to 1.0M or 2.0M), ammonium sulfate, a monosaccharide, a disaccharide (e.g., 20% sucrose), a hydrophilic polymer (e.g., polyethylene glycol), glycerol, etc. In certain embodiments, the high osmotic potential solution is a sodium chloride solution. In some embodiments, the sodium chloride solution is at least 0.25M, 0.5M, 0.75M, 1.0M, 1.25M, 1.5M, 1.75M, 2M, 2.25M or 2.5M NaCl. In some embodiments, the sodium chloride solution is about 0.25M to 5M, about 0.5M to 4M, about 0.75M to 3M, or about 1.0M to 2.0M NaCl.

The low osmotic potential solution can be any low osmotic potential solution known to those of skill in the art, such as water, for example water deionized according to any method known to those of skill. In some embodiments, the osmotic shock solution comprises water with an osmotic shock potential less than that of 50 mM NaCl.

In certain embodiments, the osmotic shock is accomplished using a sodium chloride solution followed by a water solution. In various embodiments, the sodium chloride solution is at least 0.5M NaCl, at least 0.75M NaCl, at least 1.0M NaCl, at least 1.5M NaCl, or at least 2.0M NaCl. In certain embodiments, one 0.5M NaCl treatment is followed by a water wash. In certain embodiments, two consecutive 0.5M NaCl treatments are followed by a water wash. In certain embodiments, one 2M NaCl treatment is followed by a water wash. These sequences can be repeated according to the judgment of one of skill in the art.

In certain embodiments, the placental tissue is treated with a detergent. In certain embodiments, the composition resulting from the osmotic shock is treated with a detergent. The detergent can be any detergent known to those of skill in the art to be capable of disrupting cellular or subcellular membranes. In certain embodiments, the detergent is ionic. For instance, in certain embodiments, the detergent is deoxycholate, deoxycholic acid or sodium dodecylsulfate. In certain embodiments, the detergent is zwitterionic. In certain embodiments, the detergent is nonionic. For instance, in certain embodiments, the detergent can be a TWEEN® detergent, such as TWEEN®-20, or a Triton X detergent, such as Triton X 100. The composition can be contacted with the detergent under conditions judged by one of skill in the art to be suitable for removing unwanted components from the composition. Exemplary conditions are provided in the working examples below.

In certain embodiments, the detergent treatment is carried out at about 0°C to 30°C., about 5°C to 25°C, about 5°C to 20°C, or about 5°C to 15°C. In certain embodiments, the detergent treatment is carried out at about 0°C, about 5°C, about 10°C, about 15°C, about 20°C, about 25°C, or about 30°C. In particular embodiments, the detergent treatment is carried out at about 5°C to 15°C.

In certain embodiments, the detergent treatment can be carried out for about 1-24 hours, about 2-20 hours, about 5-15 hours, about 8-12 hours, or about 2-5 hours.

In certain embodiments, the placental tissue is treated with a base. In certain embodiments, the composition resulting from osmotic shock and/or detergent treatment can be optionally treated one or more times with a base, e.g., by washing the ECM in a basic solution. Exemplary bases for the basic treatment include biocompatible bases, volatile bases or bases known to those of skill in the art to be easily and safely removed from the ECM. The base can be any organic or inorganic bases known to those of skill in the art at a concentration of, for example, 0.2M to 1.0M. In certain embodiments, the base is ammonium hydroxide, potassium hydroxide or sodium hydroxide, e.g., an ammonium hydroxide solution, potassium hydroxide solution or sodium hydroxide solution. The sodium hydroxide solution can, for example, be 0.1M NaOH, 0.25M NaOH, 0.5M NaOH, or 1M NaOH. In particular embodiments, the basic treatment is carried out in 0.1M or 0.5M NaOH.

In certain embodiments, the basic treatment is carried out at about 0°C to 30°C, about 5°C to 25°C, about 5°C to 20°C, or about 5°C to 15°C. In certain embodiments, the base treatment is carried out at about 0°C, about 5°C, about 10°C, about 15°C, about 20°C, about 25°C, or about 30°C. In particular embodiments, the base treatment is carried out at about 5°C to 15°C.

In certain embodiments, the base treatment can be carried out for about 1-24 hours, about 2-20 hours, about 5-15 hours, about 8-12 hours, or about 2-5 hours.

Variations of the detergent and base treatment (e.g., NaOH) steps can be used to generate a number of variations of the final ECM material for use in the compositions used in the methods described herein. For example, in certain embodiments, the ECM-containing tissue can be treated with about 0.1M, 0.2M, 0.3M, 0.4M, or about 0.5M NaOH over about 3, 4, 5, 6, 7, 8, 9, 10, 11, 12, 13, 14, 15, 16, 17, 18, 19, 20, 21, 22, 23, or about 24 hours so as to generate ECM having various amounts of certain components.

In certain other embodiments, the ECM present in the compositions described herein is produced without base treatment. In embodiments where a base treatment step is omitted, the ECM produced comprises higher amounts of elastin, fibronectin and/or laminin than ECM produced using a method that is the same save for inclusion of a base treatment.

In certain embodiments, the ECM is dried. Drying facilitates storage and packaging of the ECM. Drying also makes cellular components more susceptible to removal from the ECM. After any of the above steps, for example, the ECM can be dried prior to the succeeding step. Drying can be carried out according to any technique for drying apparent to those of skill in the art. Useful drying techniques are described in U.S. Patent Publication No. 2004/0048796, the contents of which are hereby incorporated by reference in their entirety. Exemplary drying techniques include, for example, lyophilization, vacuum drying, heat (e.g., below about 50°C), or freeze drying, as demonstrated in the working examples below.

In certain embodiments, any or all steps in ECM preparation are carried out under sterile conditions. In particular embodiments, base treatment, and all subsequent steps, are carried out under sterile conditions. In further embodiments, any ECM composition prepared according to the methods described herein can be further sterilized according to techniques apparent to one of skill in the art.

In certain embodiments, a method of preparing the ECM comprises osmotic shock, freeze dry, detergent treatment, a first water wash, freeze dry, base treatment, a second water wash and dry steps (e.g., freeze drying) described above, carried out in order. In certain embodiments, the detergent is deoxycholate, e.g., 1% deoxycholate. In certain embodiments, the base treatment is 0.5 N NaOH for, e.g., four hours. In certain embodiments, the first water wash is repeated (two total first washes). In certain embodiments, the second water wash is repeated twice (three total second washes). In certain embodiments, the detergent is 1% deoxycholate, the base treatment is 0.5 N NaOH for four hours, the first water wash is repeated (two total washes) and the second water wash is repeated twice (three total washes). In certain embodiments, such a process can provide a composition comprising about 0.5-0.7% (e.g., 0.59% glycosaminoglycans), about 3-5% (e.g., 3.5%) elastin, little or no detectable fibronectin, and little or no detectable laminin, as compared to the total protein in the composition (e.g., by dry weight).

In certain embodiments, a composition comprising ECM is obtained by subjecting tissue, e.g., placental tissue, to osmotic shock, base treatment, and water wash, e.g., as described above. In certain embodiments, these steps are carried out in order. In certain embodiments, the base treatment is NaOH, e.g., 0.5 N NaOH for, e.g., four hours. In certain embodiments, s composition comprising ECM or ECM components resulting from such preparation comprises about 0.2-0.4% or about 0.28% to about 0.38% glycosaminoglycans, about 3-5% or about 3.2% to about 4.7% elastin, little or no (e.g., less than 0.1% or less than 0.01%) fibronectin and little or no (e.g., less than 0.1% or less than 0.01%) laminin, as compared to the total protein in the composition (e.g., by dry weight).

In certain embodiments, a composition comprising ECM is obtained by subjecting tissue, e.g., placental tissue, to osmotic shock, detergent treatment and water wash steps, e.g., as described above. In certain embodiments, these steps are carried out in order. In certain embodiments, the detergent is deoxycholate, e.g., 1% deoxycholate. In certain embodiments, such a process can provide a composition comprising ECM or ECM components, wherein said composition comprises about 0.3%-0.5% (e.g., about 0.4%) glycosaminoglycans, about 10-15% (e.g., about 12%) elastin, about 0.2-1.0 % (e.g., about 0.6%) fibronectin and about 0.1-0.3 % (e.g., about 0.16%) laminin.

## 5.4. Optional Further Treatment

In certain embodiments, the collagen in the ECM of the compositions provided herein (or in the compositions comprising ECM components) comprises telopeptide collagen, i.e., the collagen in the ECM comprises telopeptides. Such telopeptide collagen can be used, in certain embodiments, as a source for compositions comprising atelopeptide collagen (i.e., collagen from which the telopeptides have been removed). The compositions comprising atelopeptide collagen can be used for any purpose apparent to those of skill in the art for atelopeptide collagen.

In such embodiments, the ECM in such compositions comprising telopeptide collagen can be contacted with an enzyme capable of partially or completely removing telopeptides from the collagen contained therein. The enzyme can be any proteolytic enzyme known to those of skill in the art that is capable of removing telopeptides from collagen. In certain embodiments, the enzyme is pepsin or papain. Generally, the enzyme is contacted with the composition under conditions suitable for removal of telopeptide known to those of skill in the art. Methods of treating compositions comprising telopeptide collagen with enzymes to remove telopeptides from such collagen are described in, e.g., U.S. Pat. Nos. 4,511,653, 4,582,640, 5,436,135 and 6,548,077, the contents of which are hereby incorporated by reference in their entireties.

In certain embodiments, the composition comprising telopeptide collagen is contacted with pepsin at about 15°C to 40°C, about 20°C to 35°C, about 25°C to 30°C, about 20°C to 30°C, or about 23°C to 27°C. In particular embodiments, the composition comprising telopeptide collagen is contacted with pepsin at about 23°C to 27°C for a time sufficient to remove telopeptide. The composition may be contacted with the enzyme for a time sufficient to remove telopeptide. In certain embodiments, for example, the composition is contacted with pepsin for at least 5, 10, 15, 20, 25 or 30 hours. In certain embodiments, the composition is contacted with pepsin for about 5 to 30 hours, about 10 to 25 hours or about 20 to 25 hours. In certain embodiments, the composition is contacted with pepsin for about 8, 16, 24 or 32 hours.

The composition is, in certain embodiments, contacted with the enzyme in an amount suitable to remove substantially all telopeptide from the collagen in the ECM. In some embodiments, about 0.1 g, 0.5 g, 1.0 g, 2.0 g or 5.0 g pepsin per kg ECM dry weight is contacted with the ECM comprising telopeptide collagen. In other embodiments, about 0.1 g, 0.5 g, 1.0 g, 2.0 g or 5.0 g pepsin/placenta is contacted with the ECM comprising telopeptide collagen. In certain embodiments, the composition is contacted with about 0.1 to 10.0 g/L, about 0.5 to 5/L, about 1 to 2.5 g/L, or about 0.5 1.5 g/L pepsin. In some embodiments, the composition is contacted with about 0.1 g/L, about 0.2 g/L, about 0.5 g/L, about 1.0 g/L, about 2.0 g/L, 5 g/L or 10 g/L pepsin. In particular embodiments, the \ composition is contacted with about 0.5 to 1.0 g/L pepsin in acetic acid solution with pH about 2-3, at about 23°C to 27°C for about 16-24 hours.

The compositions comprising ECM and/or ECM components, may be contacted with the enzyme in a suitable solution volume:placenta to remove telopeptides from the telopeptide collagen in the compositions. It is observed that a high volume ratio of pepsin to placenta can maximize the effect by pepsin. In certain embodiments, about 1, 2, 4, or 8 volumes of acetic acid solution per placenta is used. In particular embodiments, about 2 volumes of acetic acid solution per placenta is used.

If desired, the compositions comprising ECM and/or ECM components can be further processed by fibrillation, e.g., as described in U.S. Pat. Nos. 4,511,653, 4,582,640 and 5,436,135, the contents of which are hereby incorporated by reference in their entireties. If necessary, the composition can be concentrated according to standard techniques prior to fibrillation.

Where desired, one or more components (e.g., collagen) of the compositions comprising ECM and/or ECM components can be cross-linked. In certain embodiments, the composition is fibrillated prior to cross-linking. The cross-linking can be with any cross-linker known to those of skill in the art, for instance, the cross-linkers described above. In certain embodiments, the cross-linker is glutaraldehyde, and the cross-linking can be carried out according to methods of glutaraldehyde cross-linking of collagen known to those of skill in the art. In other embodiments, the cross-linker is 1,4-butanediol diglycidyl ether or genipin.

In some embodiments, a covalent bond between a cross-linker and a collagen present in a composition described herein can be reduced, for example to improve stability. The reduction can be accomplished by, e.g., contacting the collagen in the composition (e.g., the collagen in the ECM of a composition comprising ECM or the collagen in a composition comprising ECM components, wherein at least one component is collagen), with any reducing agent known to those of skill in the art. In certain embodiments, the reducing agent is sodium borohydride, sodium bisulfite, $\beta$-mercaptoethanol, mercaptoacetic acid, mercaptoethylamine, benzyl mercaptan, thiocresol, dithiothreitol or a phosphine such as tributylphosphine. In certain embodiments, the collagen is cross-linked prior to reduction with the reducing agent. Reduction of collagen, e.g., cross-linked collagen, is described in U.S. Pat. Nos. 4,185,011, 4,597,762, 5,412,076 and 5,763,579, the contents of which are hereby incorporated by reference in their entirety.

In certain embodiments, the compositions comprising ECM and/or ECM components can be further processed by mechanical shearing according to methods known to those of skill in the art. Exemplary shearing techniques are described in U.S. Pat. No. 4,642,117, the contents of which are hereby incorporated by reference in their entirety. In certain embodiments, the compositions comprising ECM and/or ECM components are sheared with a tissue homogenizer.

In certain embodiments, steps can be taken to limit native protease activity in the compositions used in the methods described herein. Suboptimal conditions for proteases may be achieved by formulating the compositions to eliminate or limit the amount of calcium and zinc ions available in solution. Many proteases are active in the presence of calcium and zinc ions and lose much of their activity in calcium and zinc ion free environments. Additives such as metal ion chelators, for example 1,10-phenanthroline and ethylenediaminetetraacetic acid (EDTA), therefore create an environment unfavorable to many proteolytic enzymes. Advantageously, compositions for use in the methods described herein can be prepared selecting conditions of pH, reduced availability of calcium and zinc ions, presence of metal ion chelators and the use of proteolytic inhibitors specific for collagenase. For example, compositions may include a buffered solution of water, pH 5.5 to 8, or pH 7 to 8, free from calcium and zinc ions and including a metal ion chelator such as EDTA. Additionally, control of temperature and time parameters during the treatment of a collagen composition may also be employed to limit the activity of proteases.

### 5.5. Characterization of the Compositions

### 5.5.1. Biochemical Characterization

Biochemical based assays known in the art and exemplified herein may be used to determine the biochemical compositions of the compositions useful in the methods provided herein. Absorbance based assays, e.g., for protein content, include but are not limited to assays that measure absorbance at 280 nm (see, e.g., Layne, E, Spectrophotometric and Turbidimetric Methods for Measuring Proteins, Methods in Enzymology 3: 447-455, (1957); Stoscheck, C M, Quantitation of Protein, Methods in Enzymology 182: 50-69, (1990)), 205 nm, and assays based on the extinction coefficient of the sample (see, e.g., Scopes, R K, Analytical Biochemistry 59: 277, (1974); Stoscheck, C M. Quantitation of Protein, Methods in Enzymology 182: 50-69, (1990)). Compositions may be characterized using known assays for, e.g., one or more of collagen type I, collagen type II, collagen type III, collagen type IV, laminin, elastin, fibronectin, and/or glycosaminoglycan.

Colorimetric based assays may include, but are not limited to, modified Lowry assay, biuret assay, Bradford assay, Bicinchoninic Acid (Smith) assay (see, e.g., Stoscheck, C M, Quantitation of Protein, Methods in Enzymology 182: 50-69 (1990)).

In a specific embodiment, measuring the total protein content of a composition provided herein comprises use of a Bradford dye-binding assay (Bradford, M., Analytical Biochemistry, 72, 248 (1976), which is incorporated herein by reference in its entirety). A Bradford assay may be carried out, e.g., using the Bradford dye-binding assay available through BIO-RAD, Hercules, Calif., USA. The protein assay is based on the change in color of the dye Coomassie Brilliant Blue R-250 in response to different concentrations of protein. The assay may involve, e.g., developing a standard calibration curve by measuring absorbance (at 595 nanometers) of a series of human standards of known concentrations for the protein of interest (e.g., collagen, elastin, laminin, fibronectin, etc.). For example, the concentration of collagen in a composition comprising ECM and/or ECM components, for example, a sample of the amniotic membrane, can be determined by referencing a standard curve. The assay is developed in a standard format that allows measurement of protein concentration in the range of 0.2-1.4 mg/mL and as a microassay that measures protein concentration up to 25 $\mu$g. For the standard assay, e.g., ECM dissolved in 100 mM citric acid (pH 2.4) is aliquoted into 1.5 mL microcentrifuge tubes at concentrations of 0.1-1 mg/mL at a total volume of 0.1 mL. To each tube, 1 mL of the Coomassie blue dye is added. Samples are vortexed and allowed to stand at room temperature for 10 minutes. Absorbance is measured at 595 nanometers (nm). For the microassay, ECM dissolved in 100 mM citric acid (pH 2.4) is aliquoted into wells of a 96-well plate at a total volume of 0.1 mL (2.5-30 $\mu$g/mL). To each well, 10 $\mu$L of dye reagent is added. Samples are vortexed, incubated at room temperature for ten minutes before measuring absorbance in a plate reader at 595 nm. Test samples can be assayed in triplicate. Protein concentrations are determined by referencing to the standard curve. Protein concentration is calculated as a percentage of the total dry weight of the ECM. Within a margin of error of about 10%, the protein content in each of the ECM is essentially 95% or more of the total dry weight of the ECM. Water content may be low and within the experimental error (approximately 10%).

Estimation of the total protein content (e.g., total collagen content) of the ECM in a composition used in the methods described herein may be characterized using methods known to one skilled in the art and exemplified herein. In a specific embodiment the collagen content of ECM is measured using a quantitative dye-based assay kit, e.g., the SIRCOL™ kit manufactured by Biocolor Ltd, UK. The assay utilizes Sirius Red (or Direct Red 80) as a specific collagen binding dye. Dye bound to collagen displays a concentration dependent increase in absorbance at 540 nm in a UV-Vis spectrophotometer. The assay involves developing a standard calibration curve by measuring absorbances of a series of bovine collagen standards of known concentrations. The concentration of collagen in a test sample, for example, amniotic membrane sample, is determined by referencing to the standard curve. In an exemplary assay, collagen (1 mg/mL) is aliquoted into 1.5 mL microcentrifuge tubes at concentrations from 5-100 $\mu$g/100 $\mu$L. Sample volumes are adjusted to a 100 $\mu$L with water. To each sample 1 mL of SIRCOL™ dye reagent is added at room temperature. Sample tubes are capped and allowed to incubate at room temperature with mechanical shaking for 30 mm. The samples are then centrifuged at 12,000Xg for 15 minutes and liquid drained using a pipetter. The reddish precipitate at the bottom of each tube is dissolved in 1 mL of 0.5M NaOH (sodium hydroxide). UV absorbance for the samples is measured at 540 nm using, e.g., a Beckman DU-7400 UV-VIS spectrophotometer. A standard calibration curve is plotted using the concentration of collagen in each sample versus the absorbance (OD) at 540 nm. To determine experimental error the assay may be repeated (n=10) at a single low concentration of collagen standard (10 $\mu$g/100 $\mu$L). The membrane sample is assayed using the same protocol, the sample being added in a total volume of 100 $\mu$L.

In yet other embodiments, collagen types in the compositions used in the methods described herein may be determined using standard methods known in the art and exemplified herein, e.g., ELISA assay. An exemplary assay for determining the types of collagen, e.g., collagen Types I, III and IV, in the ECM comprises using a sandwich ELISA assay provided, for example, as a kit by Arthrogen-CIA® Collagen-I from Chondrex, Inc., Redmond, Wash., USA. For the Type III and Type IV studies, the primary (Capture Antibody) and secondary antibodies (Detection Antibody) and collagen standards may be obtained from Rockland Immunochemicals, Gilbertsville, Pa. The detection antibody is a biotinylated antibody to human collagen Type I, III or IV, which binds streptavidin peroxidase. The enzymatic reaction with a chromogenic substrate and urea and $H_2O_2$ gives a yellow color, which is detected via UV-Vis spectroscopy at 490 nm. To quantitate the amount of collagen-type, a standard calibration curve is developed with a sample of a series of human collagen standards of known concentrations. The concentration of collagen in a test sample of amniotic membrane is determined by referencing to the standard curve. Assay protocols are developed as per the recommendations of the ELISA kit. To develop a standard calibration curve, 10-12 wells in a 96-well tray are coated with the capture antibody (anti-human type-I collagen antibody, unconjugated) by adding 100 $\mu$L of a 100X-diluted Capture Antibody provided with the kit. After overnight incubation, the wells are washed with three times with a wash buffer to remove unbound antibody. Human collagen Type I is then added to the wells in increasing concentration from 0-5 $\mu$g/mL in a 100 $\mu$L volume. After a two hour incubation at room temperature, the wells are washed with the wash buffer three times to remove unbound collagen. The biotinylated Collagen-I antibody is then added to the antibody-collagen complex in the wells in a 100 μL volume and allowed to bind at room temperature for two hours. Unbound antibody is washed out with three washes with the wash buffer. The detection enzyme streptavidin peroxidase is then bound to the antibody-collagen-antibody complex by addition of a 200X-diluted sample of the enzyme provided with the kit and allowing it to incubate at room temperature for one hour. The 96-well plate is washed repeatedly (six times) to remove any unbound enzyme. The chromogenic substrate+urea/$H_2O_2$ is added to each of the wells in a 100 μL volume. The reaction is allowed to proceed for 30 minutes at room temperature. The reaction is terminated by addition of 50 L of 2.5 N sulfuric acid. Absorbance is measured at 490 nm.

In yet other embodiments, total elastin content in the compositions used in the methods described herein may be accomplished using methods known in the art. An exemplary assay for measuring the elastin content of ECM may comprise a quantitative dye-based assay kit (FASTIN) manufactured by Biocolor Ltd, UK. The assay utilizes 5,10,15,20-tetraphenyl-21,23-porphrine (TPPS) as a specific elastin binding dye (see, e.g., Winkleman, J. (1962), Cancer Research, 22, 589-596). Dye bound to elastin displays a concentration dependent increase in absorbance at 513 nm in a UV-Vis spectrophotometer. The assay involves developing a standard calibration curve by measuring absorbances of a series of bovine elastin standards of known concentrations. The concentration of elastin in a test sample, for example, a sample of the ECM, is determined by referencing to the standard curve. ECM (1 mg/mL) is aliquoted into 1.5 mL microcentrifuge tubes at concentrations from 5-100 μg/100 μL. Sample volumes are adjusted to 100 μL with water. To each sample 1 mL of Elastin precipitation Reagent (trichloroacetic acid+arginine) is added at 4°C and stored overnight at the same temperature. Following overnight precipitation, the samples are centrifuged at 12,000Xg for 15 minutes and liquid is drained using a pipetter. To each sample, 1 mL of the FASTIN dye reagent (TPPS) is added with a 100 μL of 90% saturated ammonium sulfate. Sample tubes are capped and allowed to incubate at room temperature with mechanical shaking for 1 hr. The ammonium sulfate serves to precipitate the elastin-dye complex. After the 1 hr mixing step, the samples are centrifuged at 12,000Xg for 15 minutes and liquid is drained using a pipetter. The brown precipitate at the bottom of each tube is dissolved into 1 mL of FASTIN dissociation reagent which is a solution of guanidine-HCl in I-propanol. UV absorbance for the samples is measured at 513 nm using, e.g., a Beckman DU-7400 UV-VIS spectrophotometer. A standard calibration curve is plotted using the concentration of elastin in each sample versus the absorbance (OD) at 513 nm. To determine experimental error in the assay, the assay may be repeated (n=10) at a single low concentration of elastin standard (10 μg/100 μL). The membrane sample is assayed using the same protocol, the sample being added in a total volume of 100 μL. Each sample is assayed in triplicate.

In yet other embodiments, total glycosaminoglycan (GAG) content in the compositions used in the methods described herein may be determined using methods known in the art. The presence of GAGs in ECM may be measured using, e.g., a quantitative dye-based assay kit (BLYSCAN) manufactured by Biocolor Ltd, UK. The assay utilizes 1,9-dimethyl-methylene blue as a specific GAG binding dye. Dye bound to GAG displays a concentration dependent increase in absorbance at 656 nm in a UV-Vis spectrophotometer. The assay involves developing a standard calibration curve by measuring absorbances of a series of bovine GAG standards of known concentrations. The concentration of GAG in a test sample of amniotic membrane is determined by referencing to the standard curve. Bovine GAG (0.1 mg/mL) is aliquoted into 1.5 mL microcentrifuge tubes at concentrations from 0.5-5 μg/100 μL. Sample volumes are adjusted to a 100 μL with water. To each sample 1 mL of the 1,9-dimethyl-methylene dye reagent is added at room temperature. Sample tubes are capped and allowed to incubate at room temperature with mechanical shaking for 30 minutes. The samples are then centrifuged at 12,000Xg for 15 minutes and liquid drained using a pipetter. The reddish precipitate at the bottom of each tube is dissolved in 1 mL of a dye dissociation reagent. UV absorbance for the samples is measured at 656 nm using, e.g., a Beckman DU-7400 UV-VIS spectrophotometer. A standard calibration curve is plotted using the concentration of GAG in each sample versus the absorbance (OD) at 540 nm. To determine experimental error in the assay, the assay may be repeated (n=8) at a single low concentration of GAG standard (1 μg/100 μL). The membrane sample is assayed using the same protocol, the sample being added in a total volume of 100 μL. Each sample is assayed in triplicate.

In yet other embodiments, total laminin content in the compositions used in the methods described herein may be assayed using methods known in the art. An exemplary assay for determining the total laminin content in ECM may comprise, e.g., a sandwich ELISA assay, e.g., as provided as a kit from Takara Bio Inc., Shiga, Japan (Cat # MKIO7). The kit includes a 96-well plate pre-coated with the primary (Capture Antibody), which is a murine monoclonal antibody to human laminin. The secondary antibodies (Detection antibody) and human laminin standards are provided with the kit. The detection antibody is a conjugated human laminin antibody with peroxidase. The enzymatic reaction with a chromogenic substrate tetramethylbenzidine and $H_2O_2$ gives a blue color, which is detected via UV-Vis spectroscopy at 450 nm. To quantitate the amount of laminin, a standard calibration curve is developed with a sample of a series of human laminin standards of known concentrations (provided with kit). The concentration of laminin in a test sample of ECM is determined by referencing to the standard curve. Assay protocols are developed as per the recommendations of the kit. To develop a standard calibration curve, the human laminin standard is added in increasing concentrations of 5 ng/mL to 160 ng/mL in a final volume of 100 μL to individual wells of an antibody pre-coated 96-well tray provided with the kit. After an hour incubation at room temperature, the wells are washed with the wash buffer 3 times (PBS containing 0.05% TWEEN™) to remove unbound laminin. The peroxidase-conjugated laminin antibody is then added to the antibody-laminin complex in the wells in a 100 μL volume and allowed to bind at room temperature for 1 hour. The 96-well plate is washed 4X to remove any unbound enzyme/antibody conjugate. The chromogenic substrate+$H_2O_2$ is added to each of the wells in a 100 μL volume. The reaction is allowed to proceed for 30 minutes at room temperature. The reaction is terminated by addition of 100 μL of 2.5N sulfuric acid. Absorbance is measured at 450 nm. Samples of solubilized membrane are tested at a concentration of 1000 ng/mL. Each membrane sample is tested in triplicate. Laminin concentration is presented as a concentration of total membrane weight as shown below.

In yet other embodiments, total fibronectin content in the compositions used in the methods described herein may be assayed using methods known in the art and exemplified herein. An exemplary assay for determining total fibronectin content of ECM may comprise, e.g., the following: a sandwich ELISA assay provided as a kit from Takara Blo Inc., Shiga, Japan (Cat # MK1 15) may be used. The kit includes a 96-well plate pre-coated with the primary (Capture Antibody), a murine monoclonal antibody to human fibronectin. The secondary antibodies (Detection antibody) and human fibronectin standards are provided with the kit. The detection antibody is a conjugated human fibronectin antibody with horseradish peroxidase. The enzymatic reaction with a chromogenic substrate tetramethylbenzidine and $H_2O_2$ gives a blue color, which is detected via UV-Vis spectroscopy at 450 nm. To quantitate the amount of fibronectin, a standard calibration curve is developed with a sample of a series of human fibronectin standards of known concentrations (provided with kit). The concentration of fibronectin in a test sample is determined by referencing to the standard curve. Assay protocols are developed as per the recommendations of the ELISA kit. To develop a standard calibration curve, the human fibronectin standard is added in increasing concentrations of 12.5 ng/mL to 400 ng/mL in a final volume of 100 μL to individual wells of an antibody pre-coated 96-well tray provided with the kit. After a 1 hr incubation at room temperature, the wells are washed with the wash buffer 3 times (PBS containing 0.05% TWEEN™) to remove unbound fibronectin. The peroxidase-conjugated fibronectin antibody is then added to the antibody-fibronectin complex in the wells in a 100 μL volume and allowed to bind at room temperature for 1 hour. The 96-well plate is washed repeatedly (4X) to remove any unbound enzyme/antibody conjugate. The chromogenic substrate+H202 is added to each of the wells in a 100 μL volume. The reaction is allowed to proceed for 30 minutes at room temperature. The reaction is terminated by addition of 100 μL of 2.5N sulfuric acid. Absorbance is measured at 450 nm. Samples of solubilized membrane are tested at a concentration of 1000 μg/mL. Each membrane sample is tested in triplicate. Those of skill in the art will recognize that the foregoing methods can be used to measure other components of the compositions used in the methods described herein, e.g., the amounts of elastin in such compositions.

### 5.5.2. Biocompatibility Studies

The compositions useful in the methods of treatment provided herein are substantially non-immunogenic. Because non-immunogenic human tissue is inherently biocompatible with other human tissue, it is not necessary to perform several of the standard biocompatibility tests (e.g., dermal irritation and sensitization, acute systemic toxicity). Biocompatibility as used herein refers to the property of being biologically compatible by not producing a toxic, injurious, or immunological response or rejection in living tissue. Bodily response to unknown materials is a principal concern when using artificial materials in the body and hence the biocompatibility of a material is an important design consideration in such materials. Biocompatibility assays include but are not limited to cytotoxicity assays, rabbit eye irritation tests, hemolysis assays and pyrogencity assays. Biocompatibility assays useful for assessing the compositions may be cell-based or cell-free.

Cytotoxicity of the compositions may be determined using an ISO MEM Elution test (see Example 5.4.2.2). The purpose of this study is to evaluate the ability of compositions to elicit a cytotoxic response in cultured mouse fibroblast cells. In an exemplary assay, Eagle's Minimal Essential medium (E-MEM) supplemented with 5% Fetal Bovine Serum (FBS) is used to extract test samples. The medium is also supplemented with one or more of the following: L-glutamine, HEPES, gentamicin, penicillin, vancomycin, and amphotericin B (fungizone). Cultures of L-929 cells (mouse fibroblasts) are grown and used as monolayers in disposable tissue culture labware at $37 \pm 1°C$ in a humidified atmosphere of $5 \pm 1\%$ carbon dioxide in air. Test samples are extracted intact using a ratio equivalent of 120 cm$^2$ sample and 20 ml-E-MEM plus 5% FBS. Test samples are extracted in E-MEM plus 5% FBS at $37 \pm 1°C$ in $5 \pm 1\%$ carbon dioxide for 24-25 hours. After the extraction period, the maintenance culture medium is removed from test culture wells and replaced with 1 ml of the test media/extract and control media/extracts and positive control media spiked with cadmium chloride. Positive, intermediate and negative controls are run in parallel with the test samples. The test media/extract and control media/extract and positive control media spiked with cadmium chloride are plated in triplicate and incubated $72 \pm 4$ hours at $37 \pm 1°C$ in a humidified atmosphere of $5 \pm 1\%$ carbon dioxide in air. Cultures are evaluated for cytotoxic effects by microscopic observation at 24, 48 and $72 \pm 4$ hour incubation periods. Criteria for evaluating cytotoxicity can include morphological changes in cells, such as granulation, crenation or rounding, and loss of viable cells from the monolayer by lysis or detachment. The validity of the test requires that negative control cultures maintain a healthy normal appearance throughout the duration of the test. Degrees of toxicity are scored, as follows:

0 None: Discrete intracytoplasmic granules; no cell lysis

1 Slight: Not more than 20% of the cells are round, loosely attached, and without intracytoplasmic granules; occasional lysed cells are present

2 Mild: Not more than 50% of the cells are round and devoid of intra-cytoplasmic granules; no extensive cell lysis and empty areas between cells

3 Moderate: Not more than 70% of the cell layers contain rounded cells and/or are lysed

4 Severe: Nearly complete destruction of the cell layers.

According to the USP, test articles scoring "0", "1" or "2" will be considered nontoxic. Test articles scoring "3" or "4" will be considered toxic. The positive control sample must have a score of "3" or "4" and the negative control sample must have a score of "0" for a valid test.

The ocular surface of the rabbit is known to be more sensitive than human skin, therefore rabbit eye irritation studies are used to assess the biocompatibility of compositions comprising ECM and/or ECM components. In an exemplary assay, samples are screened for primary ocular irritation. A composition comprising ECM described herein is cleaned using an aqueous solution of 0.05% deoxycholic acid monohydrate sodium salt (D-Cell). The test can be conducted in accordance with the guidelines of the Federal Hazardous Substances Act (FHSA) Regulations, 16 CFR 1500. In an exemplary assay, control eyes are judged clinically normal for rabbits by gross examination with an auxiliary light source. To detect any pre-existing corneal injury the eyes are treated with fluorescein stain, flushed with 0.9% USP physiological saline solution (PSS), and observed with ultraviolet light in a darkened room. A sample is instilled into the lower conjunctival sac of one eye of each rabbit according to standard techniques. The opposite eye of each rabbit remains untreated and serves as the comparative control. Animals are returned to their cages following treatment. At 24, 48, and 72 hours after dosing the test eye of each rabbit is examined with an auxiliary light source and appropriate magnification compared to the untreated control eye, and graded for ocular irritation. To detect or confirm corneal injury the test eyes are treated with fluorescein stain, flushed with PSS, and examined in darkened conditions with an ultraviolet lamp at 24 hours. Reactions are scored in accordance with the FHSA-modified Draize scoring criteria. One of three animals exhibiting a significant positive reaction is a borderline finding. Two of three animals exhibiting a significant positive reaction is a significant positive response and the test article is considered an irritant.

Hemolytic properties of the compositions described herein may be assayed using methods known in the art and exemplified herein (See Example 5.4.2.4). Hemolysis describes the hemolytic properties of a test sample that will contact blood. In an exemplary assay, the procedure involves exposing the test material to a blood cell suspension and then determining the amount of hemoglobin released. The test is run under static conditions with direct contact of the test composition with human blood. The amount of hemoglobin released by the red blood cells is measured spectrophotometrically at 540 nm (following conversion to cyanomethemoglobin) concurrently with negative and positive controls. The hemolytic index for the samples and controls is calculated as follows:

$$\text{Hemolytic Index} = \frac{\text{Hemoglobin Released(mg/mL)} \times 100}{\text{Hemoglobin Present(mg/mL)}}$$

Where: Hemoglobin Released(mg/ml)=(Constant+X Coefficient) X Optical Density X 16 Hemoglobin Present(mg/mL)=Diluted Blood 10±1 mg/mL

Pyrogenicity of the compositions used in the methods described herein may be assayed using methods known in the art and exemplified herein (See Example 5.4.2.5). In one embodiment, the pyrogenicity of a composition is determined by measuring the presence of bacterial endotoxin in the composition using, for example, the Limulus Amebocyte Lysate (LAL) test. This test is an in vitro assay for detection and quantification of bacterial endotoxin. In an exemplary test, ninety-eight samples of a composition (e.g., a composition comprising ECM) (n=1 per lot), each measuring 1x2 cm, are tested individually for extraction. The extractions are performed by washing each sample in 30 mL of extraction fluid for 40 to 60 minutes at 37 to 40°C with intermittent swirling on an orbital shaker. The pH of each sample extract is between 6 and 8 as verified with pH paper. Pyrogen levels are measured by a Kinetic Turbidimetric Colorimetric Test with a test sensitivity of 0.05 Endotoxin Units (EU) per mL. Total endotoxin level per sample is calculated by multiplying the detected endotoxin value (EU/mL) by 30 mL (extraction volume per device) and again by twenty-four (to simulate a 6x8 cm-sized device).

### 5.5.3. Microbiological Studies

The presence of microbiological organisms in the compositions used in the methods described herein, including, but not limited to, *Escherichia coli, Klebsiella pneumoniae, Staphylococcus aureus, Enterococcus faecalis, Candida albicans, Proteus vulgaris, Staphylococcus viridans,* and *Pseudomonas aeruginosa* may be determined by art-known methods. Such methods may be used at any step of the preparation of the compositions. In certain embodiments, steps such as decellularization and rinsing act to reduce the number of microorganisms in the compositions used in the methods described herein.

The amount of contaminating organisms in a composition used in the methods described herein before it undergoes an industrial sterilization process, i.e., the "bioburden" of the compositions can be assayed using art-known methods. In an exemplary method, the minimum E-beam radiation dose that would achieve sterility with a Sterilization Assurance Level of 10-6 is determined. Membranes are extracted by immersion and manual shaking using PEPTONE-TWEEN™ Solution. Plating method is membrane filtration using soybean-casein digest agar. For aerobic conditions, plates are incubated 4 days at 30-35°C, then enumerated. For fungi, plates are incubated four days at 20-25°C, then enumerated. For spore-forming bacteria, the extract portion is heat shocked, filtered and plated as for aerobic bacteria. Plates are incubated 4 days at 30-35°C, then enumerated. For anaerobic bacteria, plates are incubated under anaerobic conditions for 4 days at 30-35°C, then enumerated. Microorganisms utilized can include, e.g., *Clostridium sporogenes, Pseudomonas aeruginosa,* and *Bacillus atrophaeus.*

In particular embodiments, the ECM, e.g., a gram of ECM, has less than 2 colony forming units (cfu) for aerobes and fungi, less than 1, or zero cfu for aerobes and fungi. In yet other embodiments, the ECM has less than 5.1 Colony Forming Units (cfu), less than 2, or less than 1 cfu for anaerobes and spores.

In particular embodiments, the compositions used in the methods described herein are not bacteriostatic or fungistatic as determined using methods exemplified herein and known to one skilled in the art (See Example 5.4.3.2). As used herein bacteriostatic refers to an agent that inhibits bacterial growth or reproduction but does not kill bacteria. As used herein fungistatic refers to an agent that prevents the growth of a fungus by the presence of a non-fungicidal chemical or physical agency.

### 5.5.4. Storage and Handling of Compositions

The compositions used in the methods described herein may be stored at room temperature (e.g., 25°C). In certain embodiments, the compositions used in the methods described herein can be stored at a temperature of at least 0°C, at least 4°C, at least 10°C, at least 15°C, at least 20°C, at least 25°C, at least 30°C, at least 35°C or at least 40°C, no more than 4°C, no more than 10°C, no more than 15°C, no more than 20°C, no more than 25°C, no more than 30°C, no more than 35°C or no more than 40°C. In some embodiments, the compositions used in the methods described herein are not refrigerated. In some embodiments, the compositions used in the methods described herein may be refrigerated at a temperature of about 2 to 8°C. In other embodiments, the compositions used in the methods described herein can be stored at any of the above-identified temperatures for an extended period of time. In a particular embodiment, the compositions used in the methods described herein are stored under sterile and non-oxidizing conditions. In certain embodiments, the compositions used in the methods described herein can be stored at any of the specified temperatures for 12 months or more with no alteration in biochemical or structural integrity (e.g., no degradation), without any alteration of the biochemical or biophysical properties of the collagen composition. In certain embodiments, the compositions used in the methods described herein can be stored for several years with no alteration in biochemical or structural integrity (e.g., no degradation), without any alteration of the biochemical or biophysical properties of the collagen composition. The compositions used in the methods described herein may be stored in any container suitable for long-term storage. In certain embodiments, the compositions used in the methods described herein can be stored in a sterile double peel-pouch package.

### 5.5.5. Sterilization

The compositions used in the methods described herein can be sterilized according to techniques known to those of skill in the art for sterilizing such compositions. In certain embodiments, the compositions are filtered through a filter that allows passage of endotoxins and retains the ECM in the compositions, or retains one or more desired ECM components. Any filter of a size, for example 30 kDa, known to those of skill in the art for filtration of endotoxins can be used. In certain embodiments, the compositions are contacted with the filter under conditions that allow endotoxins to pass through the filter while retaining the ECM or ECM components of the composition. The conditions can be any conditions for filtration known to those of skill in the art, for instance, centrifugation or pumping. The filter should be of a size that retains collagen while allowing endotoxins to pass the filter. In certain embodiments, the filter is between 5 kDa and 100 kDa. In particular embodiments, the filter is about 5 kDa, about 10 kDa, about 15 kDa, about 20 kDa, about 30 kDa, about 40 kDa, about 50 kDa, about 60 kDa, about 70 kDa, about 80 kDa, about 90 kDa or about 100 kDa. The filter can be of any material known to those of skill in the art to be compatible with a composition described herein such as cellulose, polyethersulfone and others apparent to those of skill. The filtration can be repeated as many times as desired by one of skill in the art. Endotoxin can be detected according to standard techniques to monitor clearance.

In certain embodiments, the compositions used in the methods described herein can be filtered to generate ECM free of, or reduced in, viral particles. Advantageously, the filter retains the ECM of the composition while allowing viral particles to pass through. Any filter known to those of skill in the art to be useful for clearing viruses can be used. For instance, a 1000 kDa filter can be used for clearance, or reduction, of parvovirus, hepatitis A virus and HIV. A 750 kDa filter can be used for clearance, or reduction, of parvovirus and hepatitis A virus. A 500 kDa filter can be used for clearance, or reduction, of parvovirus.

The compositions used in the methods described herein can be prepared so that they are free of viral particles, or possess a reduced in number of viral particles, by a method comprising the step of contacting the composition with a filter of a size that allows one or more viral particles to pass through the filter while retaining the ECM in the composition. In certain embodiments, the composition is contacted with the filter under conditions that allow one or more viral particles to pass through the filter while retaining the ECM or one or more desired ECM components of the composition. The conditions can be any conditions for filtration known to those of skill in the art, for instance, centrifugation or pumping. The filter should be of a size that retains the ECM or desired ECM components while allowing one or more viral particles to pass the filter. In certain embodiments, the filter is between 500 kDa and 1000 kDa. In particular embodiments, the filter is about 500 kDa, about 750 kDa or about 1000 kDa. The filter can be of any material known to those of skill in the art to be compatible with the compositions described herein such as cellulose, polyethersulfone and others apparent to those of skill. The filtration can be repeated as many times as desired by one of skill in the art. Viral particles can be detected according to standard techniques to monitor filtration.

Sterilization of the compositions used in the methods described herein can also be carried out by electron beam irradiation using methods known to one skilled in the art, e.g., Gorham, D. Byrom (ed.), 1991, Biomaterials, Stockton Press, New York, 55-122. Any dose of radiation sufficient to kill at least 99.9% of bacteria or other potentially contaminating organisms is within the scope of the method. In a particular embodiment, a dose of at least 18-25 kGy is used to achieve the terminal sterilization of ECM.

### 5.6. Formulations

In certain embodiments, the compositions used in the methods described herein can be formulated in water or phosphate buffered saline, e.g., as a solution or suspension, e.g., a mouthwash. In particular embodiments, the compositions are formulated in phosphate buffered saline. ECM or ECM components can be present in the solution or suspension at any concentration useful to those of skill in the art. In certain embodiments, the formulations provided herein comprise 0.1-100 mg/ml, 1-100 mg/ml, 1-75 mg/ml, 1-50 mg/ml, 1-40 mg/ml, 10-40 mg/ml or 20-40 mg/ml ECM. In certain embodiments, the solution or suspension comprises about 5 mg/ml, 10 mg/ml, 15 mg/ml, 20 mg/ml, 25 mg/ml, 30 mg/ml, 35 mg/ml, 40 mg/ml, 45 mg/ml or 50 mg/ml collagen. In a particular embodiment, provided herein are formulations comprising about 35 mg/ml ECM.

In certain embodiments, the compositions comprising ECM used in the methods described herein are formulated as a paste (generally, ECM as extracted from human placenta, e.g., using the methods described herein, is a white paste). The compositions comprising ECM used in the methods described herein that are formulated as a paste can be used in the methods of treatment provided herein as such a paste, or can be shaped according to any methods known in the art for shaping such materials, e.g., can be shaped to fill an oral lesion in a method of treating the oral lesion. For example, the composition can be formed into a mold, or formed around a mold, to produce specific shapes, and heat-dried, vacuum-dried or freeze-dried. The compositions can also be spread thin and dried on, e.g., a gel dryer, e.g., using vacuum. The shape can be any useful shape including sheets, tubes, plugs, spheres and the like. In specific embodiments, the compositions is shaped to fit an oral lesion.

In certain embodiments, the compositions comprising ECM and/or ECM components may comprise pharmaceutically or cosmetically acceptable carriers for the treatment of oral lesions. Forms of administration of such compositions include, but are not limited to, injections, solutions, creams, gels, implants, pumps, ointments, emulsions, suspensions, microspheres, particles, microparticles, nanoparticles, liposomes, pastes, patches, tablets, transdermal delivery devices, sprays, aerosols, or other means familiar to one of ordinary skill in the art. Such pharmaceutically or cosmetically acceptable carriers are commonly known to one of ordinary skill in the art. Pharmaceutical formulations can be prepared by procedures known in the art using well known and readily available ingredients. For example, the compounds can be formulated with common excipients, diluents, or carriers, and formed into tablets, capsules, suspensions, powders, and the like. Examples of excipients, diluents, and carriers that are suitable for such formulations include the following: fillers and extenders (e.g., starch, sugars, mannitol, and silicic derivatives); binding agents (e.g., carboxymethyl cellulose and other cellulose derivatives, alginates, gelatin, and polyvinyl-pyrrolidone); moisturizing agents (e.g., glycerol); disintegrating agents (e.g., calcium carbonate and sodium bicarbonate); agents for retarding dissolution (e.g., paraffin); resorption accelerators (e.g., quaternary ammonium compounds); surface active agents (e.g., cetyl alcohol, glycerol monostearate); adsorptive carriers (e.g., kaolin and bentonite); emulsifiers; preservatives; sweeteners; stabilizers; coloring agents; perfuming agents; flavoring agents; lubricants (e.g., talc, calcium and magnesium stearate); solid polyethyl glycols; and mixtures thereof.

The terms "pharmaceutically or cosmetically acceptable carrier" or "pharmaceutically or cosmetically acceptable vehicle" are used herein to mean, without limitations, any liquid, solid or semi-solid, including, but not limited to, water or saline, a gel, cream, salve, solvent, diluent, fluid ointment base, ointment, paste, implant, liposome, micelle, giant micelle, and the like, which is suitable for use in contact with living animal or human tissue without causing adverse physiological or cosmetic responses, and which does not interact with the other components of the composition in a deleterious manner. Other pharmaceutically or cosmetically acceptable carriers or vehicles known to one of skill in the art may be employed to make compositions for delivering the molecules.

The compositions used in the methods described herein can be constituted that they release any active ingredient, e.g., an active ingredient in addition to the ECM or ECM components in the composition, only or preferably in a particular location, possibly over a period of time. Such combinations provide yet a further mechanism for controlling release kinetics. The coatings, envelopes, and protective matrices may be made, for example, from polymeric substances or waxes.

Methods of in vivo administration of the compositions used in the methods described herein that comprise ECM and/or ECM components, and optionally other materials such as carriers, that are particularly suitable for various forms include, but are not limited to, oral administration (e.g. buccal or sublingual administration), topical application, aerosol application, transdermal administration, intradermal administration, subdermal administration, intramuscular administration, or surgical administration at the location of a lesion. Techniques useful in the various forms of administrations above include but are not limited to, topical application, surgical administration, injections, sprays, transdermal delivery devices, osmotic pumps, electrodepositing directly on a desired site, or other means familiar to one of ordinary skill in the art.

The compositions used in the methods described herein can be applied in the form of creams, gels, solutions, suspensions, liposomes, particles, or other means known to one of skill in the art of formulation and delivery of therapeutic and cosmetic compounds. Some examples of appropriate formulations for subcutaneous administration include but are not limited to implants, depot, needles, capsules, and osmotic pumps. Some examples of appropriate formulations for oral administration include but are not limited to: pastes, patches, sheets, liquids, syrups, suspensions, aerosols and mists. Accordingly such formulations are, in certain embodiments, used in the treatment or oral lesions in accordance with the methods described herein. Some examples of appropriate formulations for transdermal administration include but are not limited to creams, pastes, patches, sprays, and gels. Some examples of appropriate delivery mechanisms for subcutaneous administration include but are not limited to implants, depots, needles, capsules, and osmotic pumps.

Embodiments in which the compositions comprise, for example, one or more pharmaceutically or cosmetically acceptable carriers or excipients may conveniently be presented in unit dosage form and may be prepared by conventional pharmaceutical techniques. Such techniques include the step of bringing into association the compositions containing the active ingredient and the pharmaceutical carrier(s) or excipient(s). In general, the formulations are prepared by uniformly and intimately bringing into association the active ingredient with, e.g., a liquid carrier. Particular unit dosage formulations are those containing a dose or unit, or an appropriate fraction thereof, of the administered ingredient. It should be understood that in addition to the ingredients particularly mentioned above, formulations comprising the compositions provided herein may include other agents commonly used by one of ordinary skill in the art. The volume of administration will vary depending on the route of administration.

The compositions used in the methods described herein may be administered to persons or animals in any dose range that will produce desired physiological or pharmacological results, e.g., treatment of an oral lesion. Dosage will depend upon the substance or substances administered, the therapeutic endpoint desired, the desired effective concentration at the site of action or in a body fluid, and the type of administration. Information regarding appropriate doses of substances are known to persons of ordinary skill in the art and may be found in references such as L. S. Goodman and A. Gilman, eds, The Pharmacological Basis of Therapeutics, Macmillan Publishing, New York, and Katzung, Basic & Clinical Pharmacology, Appleton & Lang, Norwalk, Conn., (6th Ed. 1995). A clinician skilled in the art of the desired therapy may chose specific dosages and dose ranges, and frequency of administration, as required by the circumstances and the substances to be administered.

The compositions used in the methods described herein may comprise one or more compounds or substances, e.g., active ingredients or active agents, that are not ECM or ECM components, e.g., collagen, elastin, laminin and/or glycosaminoglycan. For example, the composition may be impregnated, either during production or during preparation for surgery, with a biomolecule. Such biomolecules include but are not limited to, antibiotics (such as Clindamycin, Minocycline, Doxycycline, Gentamycin), hormones, growth factors, anti-tumor agents, anti-fungal agents, anti-viral agents, pain medications, anti-histamines, anti-inflammatory agents, anti-infectives including but not limited to silver (such as silver salts, including but not limited to silver nitrate and silver sulfadiazine), elemental silver, antibiotics, bactericidal enzymes (such as lysozome), wound healing agents (such as cytokines including but not limited to PDGF, TGF; thymosin), hyaluronic acid as a wound healing agent, wound sealants (such as fibrin with or without thrombin), cellular attractant and scaffolding reagents (such as fibronectin) and the like. In a specific example, the composition may be impregnated with at least one growth factor, for example, fibroblast growth factor, epithelial growth factor, etc. The composition may also be impregnated with small organic molecules such as specific inhibitors of particular biochemical processes e.g., membrane receptor inhibitors, kinase inhibitors, growth inhibitors, anticancer drugs, antibiotics, etc.

In yet other embodiments, the compositions used in the methods described herein may be combined with a hydrogel. Any hydrogel known to one skilled in the art may be used, e.g., any of the hydrogel compositions disclosed in Graham, 1998, Med. Device Technol. 9(1): 18-22; Peppas et al., 2000, Eur. J. Pharm. Biopharm. 50(1): 27-46; Nguyen et al., 2002, Biomaterials, 23(22): 4307-14; Henincl et al., 2002, Adv. Drug Deliv. Rev 54(1): 13-36; Skelhorne et al., 2002, Med. Device. Technol. 13(9): 19-23; or Schmedlen et al., 2002, Biomaterials 23: 4325-32. In a specific embodiment, the hydrogel is applied onto the composition, i.e., discharged on the surface of the composition, if the composition is formulated in a solid form. The hydrogel for example, may be sprayed onto the composition, saturated on the surface of the composition, soaked with the composition, bathed with the composition, or coated onto the surface of the composition. The hydrogels useful in the methods and compositions provided herein can be made from any water-interactive, or water soluble polymer known in the art, including but not limited to, polyvinylalcohol (PVA), polyhydroxyehthyl methacrylate, polyethylene glycol, polyvinyl pyrrolidone, hyaluronic acid, dextran or derivatives and analogs thereof.

In some embodiments, the composition is further impregnated with one or more biomolecules prior to being combined with a hydrogel. In other embodiments, the hydrogel is further impregnated with one or more biomolecules prior to being combined with composition. Such biomolecules include but are not limited to, antibiotics (such as Clindamycin, Minocycline, Doxycycline, Gentamycin), hormones, growth factors, anti-tumor agents, anti-fungal agents, antiviral agents, pain medications, anti-histamines, anti-inflammatory agents, anti-infectives including but not limited to silver (such as silver salts, including but not limited to silver nitrate and silver sulfadiazine), elemental silver, antibiotics, bactericidal enzymes (such as lysozome), wound healing agents (such as cytokines including but not limited to PDGF, TGF; thymosin), hyaluronic acid as a wound healing agent, wound sealants (such as fibrin with or without thrombin), cellular attractant and scaffolding reagents (such as fibronectin) and the like. In a specific example, the composition or the hydrogel may be impregnated with at least one growth factor, for example, fibroblast growth factor, epithelial growth factor, etc. Advantageously, the biomolecule can be a therapeutic agent.

In some embodiments, the hydrogel is combined with a laminate comprising the compositions.

In some embodiments, the hydrogel/composition is applied topically to a subject, i.e., on the surface of the oral mucosa, e.g., at the site of a lesion. In some embodiments, the hydrogel formulated to be non-biodegradable. In yet other embodiments, the hydrogel is formulated to be biodegradable. In a specific embodiment, the hydrogel is formulated to degrade within days, e.g., less than 7 days, less than 14 days, less than 21 days, or less than 28 days after administration to an individual. In another specific embodiment, the hydrogel composition is formulated to degrade within months after administration to an individual.

In some embodiments, the compositions described herein comprise cells. For example, cells may be in solution with a composition described herein, or may be populated on a composition described herein when that composition is in solid form. Cells that can be used in the compositions described herein include, but are not limited to, stem cells (e.g., human stem cells), human differentiated adult cells, totipotent stem cells, pluripotent stem cells, multipotent stem cells, tissue specific stem cells, embryonic like stem cells, committed progenitor cells, fibroblastoid cells, and the like. In other embodiments, the compositions may comprise specific classes of progenitor cells including but not limited to chondrocytes, hepatocytes, hematopoietic cells, pancreatic parenchymal cells, neuroblasts, and muscle progenitor cells.

**5.7. Stem Cells**

In certain embodiments, the compositions used in the methods described herein comprise a plurality of stem cells. The stem cells can be any stem cells suitable for a given purpose, and can be totipotent or pluripotent stem cells, or can be progenitor cells. Preferably, the composition comprises placental stem cells such as those described in U.S. Patent Nos. 7,045,148; 7,468,276; 8,057,788 and 8,202,703, the disclosures of which are hereby incorporated by reference in their entireties. However, the compositions can comprise stem or progenitor cells, preferably mammalian stem or progenitor cells, from any tissue source, e.g., embryonic stem cells, embryonic germ cells, mesenchymal stem cells, bone marrow-derived stem cells, hematopoietic progenitor cells (e.g., hematopoietic stem cells from peripheral blood, fetal blood, placental blood, umbilical cord blood, placental perfusate, etc.), somatic stem cells, neural stem cells, hepatic stem cells, pancreatic stem cells, endothelial stem cells, cardiac stem cells, muscle stem cells, adipose stem cells, and the like. The compositions can comprise any combination of types of stem cells. In certain embodiments, the stem cells are human stem cells, e.g., human placental stem cells. In specific embodiments, the cells are autologous to the source of the composition, e.g., the cells are placental stem cells derived from the same source (i.e., placenta) from which the ECM or ECM components in the compositions are derived.

In certain embodiments, the compositions, when in solid form, are contacted with a plurality of stem or progenitor cells for a time sufficient for a plurality of said stem or progenitor cells to attach to the compositions. In preferred embodiments, the compositions are shaped into a useful configuration, e.g., sheet, plug, tube, or other configuration, prior to contacting with the stem or progenitor cells. Contacting the stem or progenitor cells with the compositions can be effected by any method known in the art, and may comprise, e.g., dispensing medium comprising the stem or progenitor cells onto the surface of the compositions; immersing a part or a whole of the compositions in a suspension of the stem or progenitor cells; culturing a plurality of the stem or progenitor cells on the surface of the compositions for a time sufficient for the plurality to proliferate for at least one cell division; and the like. The stem cells, preferably placental stem cells, can be present on the compositions, e.g., a shaped form of the compositions, on the entirety or a portion of the compositions surface, e.g., can be present randomly on the surface, present confluently, etc.

The number of stem or progenitor cells contacted with the compositions in any embodiment may vary, but in various embodiments can be at least $1 \times 10^6$, $3 \times 10^6$, $1 \times 10^7$, $3 \times 10^7$, $1 \times 10^8$, $3 \times 10^8$, $1 \times 10^9$, $3 \times 10^9$, $1 \times 10^{10}$, $3 \times 10^{10}$, $1 \times 10^{11}$, $3 \times 10^{11}$, or $1 \times 10^{12}$; or may be no more than $1 \times 10^6$, $3 \times 10^6$, $1 \times 10^7$, $3 \times 10^7$, $1 \times 10^8$, $3 \times 10^8$, $1 \times 10^9$, $3 \times 10^9$, $1 \times 10^{10}$, $3 \times 10^{10}$, $1 \times 10^{11}$, $3 \times 10^{11}$, or $1 \times 10^{12}$ stem or progenitor cells.

In certain other embodiments, the compositions comprise one or more types of extracellular matrix protein deposited by a stem cell or population of stem cells. In one embodiment, for example, a composition is made to comprise extracellular matrix proteins by contacting the composition with a plurality of stem cells; culturing the stem cells on the composition for a time sufficient for the stem cells to deposit a detectable amount of at least one type of extracellular matrix protein; and decellularizing the composition to produce a composition comprising at least one type of extracellular matrix protein. In one embodiment, therefore, the composition comprises a decellularized extracellular matrix, wherein the decellularized extracellular matrix is deposited or produced by stem cells. In various embodiments, the extracellular matrix protein comprises one or more of collagen (e.g., one or more of Type I, II, III, and/or IV collagen), elastin and/or fibronectin. In another embodiment, the extracellular matrix protein is produced by a plurality of stem cells that are proliferating and not differentiating. In another embodiment, the extracellular matrix is produced by a plurality of stem cells that are differentiating, or by a plurality of cells that have differentiated from a plurality of stem cells.

### 5.7.1. Placental Stem Cells

In one embodiment, the composition comprises a plurality of CD34- placental stem cells. CD34- placental stem cells are stem cells, obtainable from placental tissue, that adhere to a tissue culture substrate and have the capacity to differentiate into non-placental cell types. Placental stem cells can be either fetal or maternal in origin (that is, can have the genotype of either the mother or fetus). Populations of placental stem cells, or populations of cells comprising placental stem cells, can comprise placental stem cells that are solely fetal or maternal in origin, or can comprise a mixed population of placental stem cells of both fetal and maternal origin. The placental stem cells, and populations of cells comprising the placental stem cells, can be identified and selected by the morphological, marker, and culture characteristic discussed below.

The placental stem cells, when cultured in primary cultures or in cell culture, adhere to the tissue culture substrate, e.g., tissue culture container surface (e.g., tissue culture plastic). Placental stem cells in culture assume a generally fibroblastoid, stellate appearance, with a number of cytoplasmic processes extending from the central cell body. The placental stem cells are, however, morphologically differentiable from fibroblasts cultured under the same conditions, as the placental stem cells exhibit a greater number of such processes than do fibroblasts. Morphologically, placental stem cells are also distinguishable from hematopoietic stem cells, which generally assume a more rounded, or cobblestone, morphology in culture.

The placental stem cells generally express the markers CD10, CD73, CD105, CD200, HLA-G, and/or OCT-4, and do not express CD34, CD38, or CD45. Placental stem cells can also express HLA-ABC (MHC-1) and HLA-DR. Thus, in one embodiment, the stem cells that can be combined with the ECM are CD200+ or HLA-G+. In another embodiment, the placental stem cells are CD73+, CD105+, and CD200+. In another embodiment, the placental stem cells are CD200+ and OCT-4+. In another embodiment, the placental stem cells are CD73+, CD105+ and HLA-G+. In another embodiment, the placental stem cells are CD73+ and CD105+, and, when in a population of placental cells, facilitate formation of one or more embryoid-like bodies under conditions that allow formation of embryoid-like bodies. In another embodiment, the placental stem cells are OCT-4+ and, when in a population of placental cells, facilitate formation of one or more embryoid-like bodies in a population of isolated placental stem cells comprising said stem cell when cultured under conditions that allow formation of embryoid-like bodies.

In certain embodiments, the isolated placental stem cells are isolated placental stem cells. In certain other embodiments, the isolated placental stem cells are isolated placental multipotent cells. In one embodiment, the isolated placental stem cells, e.g., PDACs, are CD34-, CD10+ and CD105+ as detectable by flow cytometry. In another specific embodiment, the isolated CD34-, CD10+, CD105+ placental cells have the potential to differentiate into cells of a neural phenotype, cells of an osteogenic phenotype, and/or cells of a chondrogenic phenotype. In another specific embodiment, the isolated CD34-, CD10+, CD105+ placental cells are additionally CD200+. In another specific embodiment, the isolated CD34-, CD10+. CD105+ placental cells are additionally CD45- or CD90+. In another specific embodiment, the isolated CD34-, CD10+, CD105+ placental cells are additionally CD45- and CD90+, as detectable by flow cytometry. In another specific embodiment, the isolated CD34-, CD10+, CD105+, CD200+ placental cells are additionally CD90+ or CD45-, as detectable by flow cytometry. In another specific embodiment, the isolated CD34-, CD10+, CD105+, CD200+ placental cells are additionally CD90+ and CD45-, as detectable by flow cytometry, i.e., the cells are CD34-, CD10+, CD45-, CD90+, CD105+ and CD200+. In another specific embodiment, said CD34-, CD10+, CD45-, CD90+, CD105+, CD200+ cells are additionally CD80- and CD86-.

In certain embodiments, said placental stem cells are CD34-, CD10+, CD105+ and CD200+, and one or more of CD38-, CD45-, CD80-, CD86-, CD133-, HLA-DR,DP,DQ-, SSEA3-, SSEA4-, CD29+, CD44+, CD73+, CD90+, CD105+, HLA-A,B,C+, PDL1+, ABC-p+, and/or OCT-4+, as detectable by flow cytometry. In other embodiments, any of the CD34-, CD10+, CD105+ cells described above are additionally one or more of CD29+, CD38-, CD44+, CD54+, SH3+ or SH4+. In another specific embodiment, the cells are additionally CD44+. In another specific embodiment of any of the isolated CD34-, CD10+, CD105+ placental cells above, the cells are additionally one or more of CD117-, CD133-, KDR- (VEGFR2-), HLA-A,B,C+, HLA-DP,DQ,DR-, or Programmed Death-1 Ligand (PDL1) +, or any combination thereof.

In another embodiment, the CD34-, CD10+, CD105+ cells are additionally one or more of CD13+, CD29+, CD33+, CD38-, CD44+, CD45-, CD54+, CD62E-, CD62L-, CD62P-, SH3+ (CD73+), SH4+ (CD73+), CD80-, CD86-, CD90+, SH2+ (CD105+), CD106/VCAM+, CD117-, CD144/VE-cadherin$_{low}$, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, or Programmed Death-1 Ligand (PDL1)+, or any combination thereof. In another embodiment, the CD34-, CD10+, CD105+ cells are additionally CD13+, CD29+, CD33+, CD38-, CD44+, CD45-, CD54/ICAM+, CD62E-, CD62L-, CD62P-, SH3+ (CD73+), SH4+ (CD73+), CD80-, CD86-, CD90+, SH2+ (CD105+), CD106/VCAM+, CD117-, CD144/VE-cadherin$^{low}$, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, and Programmed Death-1 Ligand (PDL1)+.

In another specific embodiment, any of the placental stem cells described herein are additionally ABC-p+, as detectable by flow cytometry, or OCT-4+ (POU5F1), as determined by reverse-transcriptase polymerase chain reaction (RT-PCR), wherein ABC-p is a placenta-specific ABC transporter protein (also known as breast cancer resistance protein (BCRP) and as mitoxantrone resistance protein (MXR)), and OCT-4 is the Octamer-4 protein (POU5F1). In another specific embodiment, any of the placental stem cells described herein are additionally SSEA3- or SSEA4-, as determined by flow cytometry, wherein SSEA3 is Stage Specific Embryonic Antigen 3, and SSEA4 is Stage Specific Embryonic Antigen 4. In another specific embodiment, any of the placental stem cells described herein are additionally SSEA3- and SSEA4-.

In another specific embodiment, any of the placental cells described herein are additionally one or more of MHC-I+ (e.g., HLA-A,B,C+), MHC-II- (e.g., HLA-DP,DQ,DR-) or HLA-G-. In another specific embodiment, any of the placental stem cells described herein are additionally one or more of MHC-I+ (e.g., HLA-A,B,C+), MHC-IV (e.g., HLA-DP,DQ,DR-) and HLA-G-.

Also provided herein are populations of the isolated placental stem cells, or populations of cells, e.g., populations of placental cells, comprising, e.g., that are enriched for, the isolated placental stem cells, that are useful in the methods and compositions disclosed herein. Preferred populations of cells comprising the isolated placental stem cells, wherein the populations of cells comprise, e.g., at least 10%, 15%, 20%, 25%, 30%, 35%, 40%, 45%, 50%, 55%, 60%, 65%, 70%, 75%, 80%, 85%, 90%, 95% or 98% isolated placental stem cells that are CD10+, CD105+ and CD34-; that is, at least 10%, 15%, 20%, 25%, 30%, 35%, 40%, 45%, 50%, 55%, 60%, 65%, 70%, 75%, 80%, 85%, 90%, 95% or 98% of cells in said population are isolated placental stem cells that are CD10+, CD105+ and CD34-. In a specific embodiment, the isolated CD34-, CD10+, CD105+ placental stem cells are additionally CD200+. In another specific embodiment, the isolated CD34-, CD10+, CD105+, CD200+ placental stem cells are additionally CD90+ or CD45-, as detectable by flow cytometry. In another specific embodiment, the isolated CD34-, CD10+, CD105+, CD200+ placental stem cells are additionally CD90+ and CD45-, as detectable by flow cytometry. In another specific embodiment, any of the isolated CD34-, CD10+, CD105+ placental stem cells described above are additionally one or more of CD29+, CD38-, CD44+, CD54+, SH3+ or SH4+. In another specific embodiment, the isolated CD34-, CD10+, CD105+ placental stem cells, or isolated CD34-, CD10+, CD105+, CD200+ placental stem cells, are additionally CD44+. In a specific embodiment of any of the populations of cells comprising isolated CD34-, CD10+, CD105+ placental stem cells above, the isolated placental stem cells are additionally one or more of CD13+, CD29+, CD33+, CD38-, CD44+, CD45-, CD54-, CD62E-, CD62L-, CD62P-, SH3+ (CD73+), SH4+ (CD73+), CD80-, CD86-, CD90+, SH2+ (CD105+), CD106/VCAM+, CD117-, CD144NE-cadherin$^{low}$, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, or Programmed Death-1 Ligand (PDL1) +, or any combination thereof. In another specific embodiment, the CD34-, CD10+, CD105+ placental stem cells are additionally CD13+, CD29+, CD33+, CD38-, CD44+, CD45-, CD54/ICAM+, CD62E-, CD62L-, CD62P-, SH3+ (CD73+), SH4+ (CD73+), CD80-, CD86-, CD90+, SH2+ (CD105+), CD106/VCAM+, CD117-, CD144/VE-cadherin$^{low}$, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, and Programmed Death-1 Ligand (PDL1)+.

In certain embodiments, the isolated placental stem cells useful in the methods and compositions described herein are one or more, or all, of CD10+, CD29+, CD34-, CD38-, CD44+, CD45-, CD54+, CD90+, SH2+, SH3+, SH4+, SSEA3-, SSEA4-, OCT-4+, and ABC-p+, wherein said isolated placental stem cells are obtained by physical and/or enzymatic disruption of placental tissue. In a specific embodiment, the isolated placental stem cells are OCT-4+ and ABC-p+. In another specific embodiment, the isolated placental stem cells are OCT-4+ and CD34-, wherein said isolated placental stem cells have at least one of the following characteristics: CD10+, CD29+, CD44+, CD45-, CD54+, CD90+, SH3+, SH4+, SSEA3-, and SSEA4-. In another specific embodiment, the isolated placental stem cells are OCT-4+, CD34-, CD10+, CD29+, CD44+, CD45-, CD54+, CD90+, SH3+, SH4+, SSEA3-, and SSEA4-. In another embodiment, the isolated placental stem cells are OCT-4+, CD34-, SSEA3-, and SSEA4-. In another specific embodiment, the isolated placental stem cells are OCT-4+ and CD34-, and either SH2+ or SH3+. In another specific embodiment, the isolated placental stem cells are OCT-4+, CD34-, SH2+, and SH3+. In another specific embodiment, the isolated placental stem cells are OCT-4+, CD34-, SSEA3-, and SSEA4-, and are either SH2+ or SH3+. In another specific embodiment, the isolated placental stem cells are OCT-4+ and CD34-, and either SH2+ or SH3+, and are at least one of CD10+, CD29+, CD44+, CD45-, CD54+, CD90+, SSEA3-, or SSEA4-. In another specific embodiment, the isolated placental stem cells are OCT-4+, CD34-, CD10+, CD29+, CD44+, CD45-, CD54+, CD90+, SSEA3-, and SSEA4-, and either SH2+ or SH3+.

In another embodiment, the isolated placental stem cells useful in the methods and compositions disclosed herein are SH2+, SH3+, SH4+ and OCT-4+. In another specific embodiment, the isolated placental stem cells are CD10+, CD29+, CD44+, CD54+, CD90+, CD34-, CD45-, SSEA3-, or SSEA4-. In another embodiment, the isolated placental stem cells are SH2+, SH3+, SH4+. SSEA3- and SSEA4-. In another specific embodiment, the isolated placental stem cells are SH2+, SH3+, SH4+, SSEA3- and SSEA4-, CD10+, CD29+, CD44+, CD54+, CD90+, OCT-4+, CD34- or CD45-.

In another embodiment, the isolated placental stem cells useful in the methods and compositions disclosed herein are CD10+, CD29+, CD34-, CD44+, CD45-, CD54+, CD90+, SH2+, SH3+, and SH4+; wherein said isolated placental stem cells are additionally one or more of OCT-4+, SSEA3- or SSEA4-.

In certain embodiments, isolated placental stem cells useful in the methods and compositions disclosed herein are CD200+ or HLA-G-. In a specific embodiment, the isolated placental stem cells are CD200+ and HLA-G-. In another specific embodiment, the isolated placental stem cells are additionally CD73+ and CD105+. In another specific embodiment, the isolated placental stem cells are additionally CD34-, CD38- or CD45-. In another specific embodiment, the isolated placental stem cells are additionally CD34-, CD38- and CD45-. In another specific embodiment, said placental stem cells are CD34-, CD38-, CD45-, CD73+ and CD105+. In another specific embodiment, said isolated CD200+ or HLA-G- placental cells facilitate the formation of embryoid-like bodies in a population of placental cells comprising the isolated placental stem cells, under conditions that allow the formation of embryoid-like bodies. In another specific embodiment, the isolated placental stem cells are isolated away from placental cells that are not stem or multipotent cells. In another specific embodiment, said isolated placental stem cells are isolated away from placental cells that do not display these combination of markers.

In another embodiment, a cell population useful in the methods of treatment and ECM compositions described herein is a population of cells comprising, e.g., that is enriched for, CD200+, HLA-G- placental stem cells. In a specific embodiment, said population is a population of placental cells. In various embodiments, at least about 10%, at least about 20%, at least about 30%, at least about 40%, at least about 50%, or at least about 60% of cells in said cell population are isolated CD200+, HLA-G- placental stem cells. Preferably, at least about 70% of cells in said cell population are isolated CD200+, HLA-G- placental stem cells. More preferably, at least about 90%, 95%, or 99% of said cells are isolated CD200+, HLA-G- placental stem cells. In a specific embodiment of the cell populations, said isolated CD200+, HLA-G- placental stem cells are also CD73+ and CD105+. In another specific embodiment, said isolated CD200+, HLA-G- placental stem cells are also CD34-, CD38- or CD45-. In another specific embodiment, said isolated CD200+, HLA-G- placental stem cells are also CD34-, CD38-, CD45-, CD73+ and CD105+. In another embodiment, said cell population produces one or more embryoid-like bodies when cultured under conditions that allow the formation of embryoid-like bodies. In another specific embodiment, said cell population is isolated away from placental cells that are not stem cells. In another specific embodiment, said isolated CD200+, HLA-G- placental stem cells are isolated away from placental cells that do not display these markers.

In another embodiment, the isolated placental stem cells useful in the methods and compositions described herein are CD73+, CD105+, and CD200+. In another specific embodiment, the isolated placental stem cells are HLA-G-. In another specific embodiment, the isolated placental stem cells are CD34-, CD38- or CD45-. In another specific embodiment, the isolated placental stem cells are CD34-, CD38- and CD45-. In another specific embodiment, the isolated placental stem cells are CD34-, CD38-, CD45-, and HLA-G-. In another specific embodiment, the isolated CD73+, CD105+, and CD200+ placental stem cells facilitate the formation of one or more embryoid-like bodies in a population of placental cells comprising the isolated placental stem cells, when the population is cultured under conditions that allow the formation of embryoid-like bodies. In another specific embodiment, the isolated placental stem cells are isolated away from placental cells that are not the isolated placental stem cells. In another specific embodiment, the isolated placental stem cells are isolated away from placental cells that do not display these markers.

In another embodiment, a cell population useful in the methods and compositions described herein is a population of cells comprising, e.g., that is enriched for, isolated CD73+, CD105+, CD200+ placental stem cells. In various embodiments, at least about 10%, at least about 20%, at least about 30%, at least about 40%, at least about 50%, or at least about 60% of cells in said cell population are isolated CD73+, CD105+, CD200+ placental stem cells. In another embodiment, at least about 70% of said cells in said population of cells are isolated CD73+, CD105+, CD200+ placental stem cells. In another embodiment, at least about 90%, 95% or 99% of cells in said population of cells are isolated CD73+, CD105+, CD200+ placental stem cells. In a specific embodiment of said populations, the isolated placental stem cells are HLA-G-. In another specific embodiment, the isolated placental stem cells are additionally CD34-, CD38- or CD45-. In another specific embodiment, the isolated placental stem cells are additionally CD34-, CD38- and CD45-. In another specific embodiment, the isolated placental stem cells are additionally CD34-, CD38-, CD45-, and HLA-G. In another specific embodiment, said population of cells comprising said placental stem cells produces one or more embryoid-like bodies when cultured under conditions that allow the formation of embryoid-like bodies. In another specific embodiment, said population of placental stem cells is isolated away from placental cells that are not stem cells. In another specific embodiment, said population of placental cells is isolated away from placental cells that do not display these characteristics.

In certain other embodiments, the isolated placental stem cells are one or more of CD10+, CD29+, CD34-, CD38-, CD44+, CD45-, CD54+, CD90+, SH2+, SH4+, SSEA3-, SSEA4-, OCT-4+, HLA-G- or ABC-p+. In a specific embodiment, the isolated placental stem cells are CD10+, CD29+. CD34-, CD38-, CD44+, CD45-, CD54+, CD90+, SH2+, SH3+, SH4+, SSEA3-, SSEA4-, and OCT-4+. In another specific embodiment, the isolated placental stem cells are CD10+, CD29+, CD34-, CD38-, CD45-, CD54+, SH2+, SH3+, and SH4+. In another specific embodiment, the isolated placental stem cells are CD10+, CD29+, CD34-, CD38-, CD45-, CD54+, SH2+, SH3+, SH4+ and OCT-4+. In another specific embodiment, the isolated placental stem cells are CD10+, CD29+, CD34-, CD38-, CD44+, CD45-, CD54+, CD90+, HLA-G-, SH2+, SH3+, and SH4+. In another specific embodiment, the isolated placental stem cells are OCT-4+ and ABC-p+. In another specific embodiment, the isolated placental stem cells are SH2+, SH3+, SH4+ and OCT-4+. In another embodiment, the isolated placental stem cells are OCT-4+, CD34-, SSEA3-, and SSEA4-. In a specific embodiment, said isolated OCT-4+, CD34-, SSEA3-, and SSEA4- placental cells are additionally CD10+, CD29+, CD34-, CD44+, CD45-, CD54+, CD90+, SH2+, SH3+, and SH4+. In another embodiment, the isolated placental stem cells are OCT-4+ and CD34-, and either SH3+ or SH4+. In another embodiment, the isolated placental stem cells are CD34- and either CD10+, CD29+, CD44+, CD54+, CD90+, or OCT-4+.

In another embodiment, the isolated placental stem cells useful in the methods and compositions described herein are CD200+ and OCT-4+. In a specific embodiment, the isolated placental stem cells are CD73+ and CD105+. In another specific embodiment, said isolated placental stem cells are HLA-G-. In another specific embodiment, said isolated CD200+, OCT-4+ placental stem cells are CD34-, CD38- or CD45-. In another specific embodiment, said isolated CD200+, OCT-4+ placental stem cells are CD34-, CD38- and CD45-. In another specific embodiment, said isolated CD200+, OCT-4+ placental stem cells are CD34-, CD38-, CD45-, CD73+, CD105+ and HLA-G-. In another specific embodiment, the isolated CD200+, OCT-4+ placental stem cells facilitate the production of one or more embryoid-like bodies by a population of placental cells that comprises the isolated cells, when the population is cultured under conditions that allow the formation of embryoid-like bodies. In another specific embodiment, said isolated CD200+, OCT-4+ placental stem cells are isolated away from placental cells that are not stem cells. In another specific embodiment, said isolated CD200+, OCT-4+ placental cells are isolated away from placental cells that do not display these characteristics.

In another embodiment, a cell population useful in the methods and compositions described herein is a population of cells comprising, e.g., that is enriched for, CD200+, OCT-4+ placental stem cells. In various embodiments, at least about 10%, at least about 20%, at least about 30%, at least about 40%, at least about 50%, or at least about 60% of cells in said cell population are isolated CD200+, OCT-4+ placental stem cells. In another embodiment, at least about 70% of said cells are said isolated CD200+, OCT-4+ placental stem cells. In another embodiment, at least about 80%, 90%, 95%, or 99% of cells in said cell population are said isolated CD200+, OCT-4+ placental stem cells. In a specific embodiment of the isolated populations, said isolated CD200+, OCT-4+ placental cells are additionally CD73+ and CD105+. In another specific embodiment, said isolated CD200+, OCT-4+ placental stem cells are additionally HLA-G-. In another specific embodiment, said isolated CD200+, OCT-4+ placental stem cells are additionally CD34-, CD38- and CD45-. In another specific embodiment, said isolated CD200+, OCT-4+ placental cells are additionally CD34-, CD38-, CD45-, CD73+, CD105+ and HLA-G-. In another specific embodiment, the cell population produces one or more embryoid-like bodies when cultured under conditions that allow the formation of embryoid-like bodies. In another specific embodiment, said cell population is isolated away from placental cells that are not isolated CD200+, OCT-4+ placental cells. In another specific embodiment, said cell population is isolated away from placental cells that do not display these markers.

In another embodiment, the isolated placental stem cells useful in the methods and compositions described herein are CD73+, CD105+ and HLA-G-. In another specific embodiment, the isolated CD73+, CD105+ and HLA-G- placental stem cells are additionally CD34-, CD38- or CD45-. In another specific embodiment, the isolated CD73+, CD105+, HLA-G- placental cells are additionally CD34-, CD38- and CD45-. In another specific embodiment, the isolated CD73+, CD105+, HLA-G- placental stem cells are additionally OCT-4+.

In another specific embodiment, the isolated CD73+, CD105+, HLA-G- placental stem cells are additionally CD200+. In another specific embodiment, the isolated CD73+, CD105+, HLA-G- placental stem cells are additionally CD34-, CD38-, CD45-, OCT-4+ and CD200+. In another specific embodiment, the isolated CD73+, CD105+, HLA-G- placental stem cells facilitate the formation of embryoid-like bodies in a population of placental cells comprising said cells, when the population is cultured under conditions that allow the formation of embryoid-like bodies. In another specific embodiment, said the isolated CD73+, CD105+, HLA-G- placental stem cells are isolated away from placental cells that are not the isolated CD73+, CD105+, HLA-G- placental stem cells. In another specific embodiment, said the isolated CD73+, CD105+, HLA-G- placental stem cells are isolated away from placental cells that do not display these markers.

In another embodiment, a cell population useful in the methods and compositions described herein is a population of cells comprising, e.g., that is enriched for, isolated CD73+, CD105 and HLA-G- placental stem cells. In various embodiments, at least about 10%, at least about 20%, at least about 30%, at least about 40%, at least about 50%, or at least about 60% of cells in said population of cells are isolated CD73+, CD105+, HLA-G- placental stem cells. In another embodiment, at least about 70% of cells in said population of cells are isolated CD73+, CD105+, HLA-G- placental stem cells. In another embodiment, at least about 90%, 95% or 99% of cells in said population of cells are isolated CD73+, CD105+, HLA-G- placental stem cells. In a specific embodiment of the above populations, said isolated CD73+, CD105+, HLA-G- placental stem cells are additionally CD34-, CD38- or CD45-. In another specific embodiment, said isolated CD73+, CD105+, HLA-G- placental stem cells are additionally CD34-, CD38- and CD45-. In another specific embodiment, said isolated CD73+, CD105+, HLA-G- placental stem cells are additionally OCT-4+. In another specific embodiment, said isolated CD73+, CD105+, HLA-G- placental stem cells are additionally CD200+. In another specific embodiment, said isolated CD73+, CD105+, HLA-G- placental stem cells are additionally CD34-, CD38-, CD45-, OCT-4+ and CD200+. In another specific embodiment, said cell population is isolated away from placental cells that are not said CD73+, CD105+, HLA-G- placental stem cells. In another specific embodiment, said cell population is isolated away from placental cells that do not display these markers.

In another embodiment, the isolated placental stem cells useful in the methods and compositions described herein are CD73+ and CD105+ and facilitate the formation of one or more embryoid-like bodies in a population of isolated placental cells comprising said CD73+, CD105+ placental stem cells when said population is cultured under conditions that allow formation of embryoid-like bodies. In another specific embodiment, said isolated CD73+, CD105+ placental stem cells are additionally CD34-, CD38- or CD45-. In another specific embodiment, said isolated CD73+, CD105+ placental stem cells are additionally CD34-, CD38- and CD45-. In another specific embodiment, said isolated CD73+, CD105+ placental stem cells are additionally OCT-4+. In another specific embodiment, said isolated CD73+, CD105+ placental stem cells are additionally OCT-4+, CD34-, CD38- and CD45-. In another specific embodiment, said isolated CD73+, CD105+ placental cells are isolated away from placental cells that are not said cells. In another specific embodiment, said isolated CD73+, CD105+ placental cells are isolated away from placental cells that do not display these characteristics.

In another embodiment, a cell population useful in the methods and compositions described herein is a population of cells comprising, e.g., that is enriched for, isolated placental stem cells that are CD73+, CD105+ and facilitate the formation of one or more embryoid-like bodies in a population of isolated placental cells comprising said cells when said population is cultured under conditions that allow formation of embryoid-like bodies. In various embodiments, at least about 10%, at least about 20%, at least about 30%, at least about 40%, at least about 50%, or at least about 60% of cells in said population of cells are said isolated CD73+, CD105+ placental cells. In another embodiment, at least about 70% of cells in said population of cells are said isolated CD73+, CD105+ placental stem cells. In another embodiment, at least about 90%, 95% or 99% of cells in said population of cells are said isolated CD73+, CD105+ placental stem cells. In a specific embodiment of the above populations, said isolated CD73+, CD105+ placental stem cells are additionally CD34-, CD38- or CD45-. In another specific embodiment, said isolated CD73+, CD105+ placental stem cells are additionally CD34-, CD38- and CD45-. In another specific embodiment, said isolated CD73+, CD105+ placental stem cells are additionally OCT-4+. In another specific embodiment, said isolated CD73+, CD105+ placental stem cells are additionally CD200+. In another specific embodiment, said isolated CD73+, CD105+ placental stem cells are additionally CD34-, CD38-, CD45-, OCT-4+ and CD200+. In another specific embodiment, said cell population is isolated away from placental cells that are not said isolated CD73+, CD105+ placental stem cells. In another specific embodiment, said cell population is isolated away from placental cells that do not display these markers.

In another embodiment, the isolated placental stem cells useful in the methods and compositions described herein are OCT-4+ and facilitate formation of one or more embryoid-like bodies in a population of isolated placental cells comprising said cells when cultured under conditions that allow formation of embryoid-like bodies. In a specific embodiment, said isolated OCT-4+ placental stem cells are additionally CD73+ and CD105+. In another specific embodiment, said isolated OCT-4+ placental stem cells are additionally CD34-, CD38-, or CD45-. In another specific embodiment, said isolated OCT-4+ placental stem cells are additionally CD200+. In another specific embodiment, said isolated OCT-4+ placental stem cells are additionally CD73+, CD105+, CD200+, CD34-, CD38-, and CD45-. In another specific embodiment, said isolated OCT-4+ placental stem cells are isolated away from placental cells that are not OCT-4+ placental stem cells. In another specific embodiment, said isolated OCT-4+ placental cells are isolated away from placental cells that do not display these characteristics.

In another embodiment, a cell population useful in the methods and compositions described herein is a population of cells comprising, e.g., that is enriched for, isolated placental stem cells that are OCT-4+ and facilitate the formation of one or more embryoid-like bodies in a population of isolated placental cells comprising said cells when said population is cultured under conditions that allow formation of embryoid-like bodies. In various embodiments, at least about 10%, at least about 20%, at least about 30%, at least about 40%, at least about 50%, or at least about 60% of cells in said population of cells are said isolated OCT-4+ placental stem cells. In another embodiment, at least about 70% of cells in said population of cells are said isolated OCT-4+ placental cells. In another embodiment, at least about 80%, 90%, 95% or 99% of cells in said population of cells are said isolated OCT-4+ placental stem cells. In a specific embodiment of the above populations, said isolated OCT-4+ placental stem cells are additionally CD34-, CD38- or CD45-. In another specific embodiment, said isolated OCT-4+ placental stem cells are additionally CD34-, CD38- and CD45-. In another specific embodiment, said isolated OCT-4+ placental stem cells are additionally CD73+ and CD105+. In another specific embodiment, said isolated OCT-4+ placental stem cells are additionally CD200+. In another specific embodiment, said isolated OCT-4+ placental stem cells are additionally CD73+, CD105+, CD200+, CD34-, CD38-, and CD45-. In another specific embodiment, said cell population is isolated away from placental cells that are not said placental stem cells. In another specific embodiment, said cell population is isolated away from placental cells that do not display these markers.

In another embodiment, the isolated placental stem cells useful in the methods and compositions described herein are isolated HLA-A,B,C+, CD45-, CD133- and CD34-placental stem cells. In another embodiment, a cell population useful in the methods and compositions described herein is a population of cells comprising isolated placental stem cells, wherein at least about 70%, at least about 80%, at least about 90%, at least about 95% or at least about 99% of cells in said population of cells are isolated HLA-A,B,C+, CD45-, CD133- and CD34- placental stem cells. In a specific embodiment, said isolated placental stem cell or population of isolated placental stem cells is isolated away from placental cells that are not HLA-A,B,C+, CD45-, CD133- and CD34- placental stem cells. In another specific embodiment, said isolated placental stem cells are non-maternal in origin. In another specific embodiment, said population of isolated placental stem cells are substantially free of maternal components; e.g., at least about 40%, 45%, 5-0%, 55%, 60%, 65%, 70%, 75%, 90%, 85%, 90%, 95%, 98% or 99% of said cells in said population of isolated placental cells are non-maternal in origin.

In another embodiment, the isolated placental stem cells useful in the methods and compositions described herein are isolated CD10+, CD13+, CD33+, CD45-, CD117- and CD 133- placental stem cells. In another embodiment, a cell population useful in the methods and compositions described herein is a population of cells comprising isolated placental stem cells, wherein at least about 70%, at least about 80%, at least about 90%, at least about 95% or at least about 99% of cells in said population of cells are isolated CD10+, CD13+, CD33+, CD45-, CD117- and CD133- placental stem cells. In a specific embodiment, said isolated placental stem cells or population of isolated placental stem cells is isolated away from placental cells that are not said isolated placental stem cells. In another specific embodiment, said isolated CD10+, CD13+, CD33+, CD45-, CD117- and CD133- placental cells are non-maternal in origin, i.e., have the fetal genotype. In another specific embodiment, at least about 40%, 45%, 50%, 55%, 60%, 65%, 70%, 75%, 90%, 85%, 90%, 95%, 98% or 99% of said cells in said population of isolated placental stem cells, are non-maternal in origin. In another specific embodiment, said isolated placental stem cells or population of isolated placental stem cells are isolated away from placental cells that do not display these characteristics.

In another embodiment, the isolated placental stem cells useful in the methods and compositions described herein are isolated CD10+ CD33-, CD44+, CD45-, and CD117-placental stem cells. In another embodiment, a cell population useful for the in the methods and compositions described herein is a population of cells comprising, e.g., enriched for, isolated placental stem cells, wherein at least about 70%, at least about 80%, at least about 90%, at least about 95% or at least about 99% of cells in said population of cells are isolated CD10+ CD33-, CD44+, CD45-, and CD117- placental stem cells. In a specific embodiment, said isolated placental cell or population of isolated placental stem cells is isolated away from placental cells that are not said cells. In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental stem cell or population of isolated placental stem cells is isolated away from placental cells that do not display these markers.

In another embodiment, the isolated placental stem cells useful in the methods and compositions described herein are isolated CD10+ CD13-, CD33-, CD45-, and CD117-placental stem cells. In another embodiment, a cell population useful in the methods and compositions described herein is a population of cells comprising, e.g., enriched for, isolated CD10+, CD13-, CD33-, CD45-, and CD117- placental stem cells, wherein at least about 70%, at least about 80%, at least about 90%, at least about 95% or at least about 99% of cells in said population are CD10+ CD13-, CD33-, CD45-, and CD117- placental cells. In a specific embodiment, said isolated placental stem cells or population of isolated placental stem cells are isolated away from placental cells that are not said cells. In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental stem cell or population of isolated placental stem cells is isolated away from placental cells that do not display these characteristics.

In another embodiment, the isolated placental stem cells useful in the methods and compositions described herein are HLA A,B,C+, CD45-, CD34-, and CD133-, and are additionally CD10+, CD13+, CD38+, CD44+, CD90+, CD105+, CD200+ and/or HLA-G-, and/or negative for CD117. In another embodiment, a cell population useful in the methods described herein is a population of cells comprising isolated placental stem cells, wherein at least about 20%, 25%, 30%, 35%, 40%, 45%, 50%, 55%, 60%, 65%, 70%, 75%, 80%, 85%, 90%, 95%, 98% or about 99% of the cells in said population are isolated placental stem cells that are HLA A,B,C+, CD45-, CD34-, CD133-, and that are additionally positive for CD10, CD13, CD38, CD44, CD90, CD105, CD200, and/or negative for CD117 and/or HLA-G. In a specific embodiment, said isolated placental stem cells or population of isolated placental stem cells are isolated away from placental cells that are not said cells. In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental stem cells or population of isolated placental stem cells are isolated away from placental cells that do not display these markers.

In another embodiment, the isolated placental stem cells useful in the methods and compositions described herein are isolated placental stem cells that are CD200+ and CD10+, as determined by antibody binding, and CD117-, as determined by either antibody binding or RT-PCR. In another embodiment, the isolated placental stem cells useful in the methods and compositions described herein are isolated placental stem cells, e.g., placental stem cells or placental multipotent cells, that are CD10+, CD29+, CD54+, CD200+, HLA-G-, MHC class I+ and $\beta$-2-microglobulin+. In another embodiment, isolated placental stem cells useful in the methods and compositions described herein are placental cells wherein the expression of at least one cellular marker is at least two-fold higher than for a mesenchymal stem cell (e.g., a bone marrow-derived mesenchymal stem cell). In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin.

In another embodiment, the isolated placental stem cells useful in the methods and compositions described herein are isolated placental stem cells, e.g., placental stem cells or placental multipotent cells, that are one or more of CD10+, CD29+, CD44+, CD45-, CD54/ICAM+, CD62E-. CD62L-, CD62P-, CD80-, CD86-, CD103-, CD104-, CD105+, CD106/VCAM+, CD144/VE-cadherin$^{low}$, CD184/CXCR4-, $\beta$-microglobulin$^{low}$, MHC-I$^{low}$, MHC-II-, HLA-G$^{low}$, and/or PDL1$^{low}$. In a specific embodiment, the isolated placental stem cells are at least CD29+ and CD54+. In another specific embodiment, the isolated placental stem cells are at least CD44+ and CD106+. In another specific embodiment, the isolated placental stem cells are at least CD29+.

In another embodiment, a cell population useful in the methods and compositions described herein comprises isolated placental stem cells, wherein at least 50%, 60%, 70%, 80%, 90%, 95%, 98% or 99% of the cells in said cell population are isolated placental stem cells that are one or more of CD10+, CD29+, CD44+, CD45-, CD54/ICAM+, CD62-E-, CD62-L-, CD62-P-, CD80-, CD86-, CD103-, CD104-, CD105+, CD106/VCAM+, CD144/VE- cadherin$^{dim}$, CD184/CXCR4-, $\beta$-microglobulin$^{dim}$, HLA-I$^{dim}$, HLA-II-, HLA-G$^{dim}$, and/or PDL1$^{dim}$. In another specific embodiment, at least 50%, 60%, 70%, 80%, 90%, 95%, 98% or 99% of cells in said cell population are CD10+, CD29+, CD44+, CD45-, CD54/ICAM+, CD62-E-, CD62-L-, CD62-P-, CD80-, CD86-, CD103-, CD104-, CD105+, CD106/VCAM+, CD144/VE-cadherin$^{dim}$, CD184/CXCR4-, $\beta$-microglobulin$^{dim}$, MHC-I$^{dim}$, MHC-II-, HLA-G$^{dim}$, and PDL1$^{dim}$. In certain embodiments, the placental cells express HLA-II markers when induced by interferon gamma (IFN-$\gamma$).

In another embodiment, the isolated placental stem cells useful in the methods and compositions described herein are isolated placental stem cells that are one or more, or all, of CD10+, CD29+, CD34-, CD38-, CD44+, CD45-, CD54+, CD90+, SH2+, SH3+, SH4+, SSEA3-, SSEA4-, OCT-4+, and ABC-p+, where ABC-p is a placenta-specific ABC transporter protein (also known as breast cancer resistance protein (BCRP) and as mitoxantrone resistance protein (MXR)), wherein said isolated placental stem cells are obtained, e.g., by perfusion of a mammalian, e.g., human, placenta that has been drained of cord blood and perfused to remove residual blood.

In another specific embodiment of any of the above characteristics, expression of the cellular marker (e.g., cluster of differentiation or immunogenic marker) is determined by flow cytometry; in another specific embodiment, expression of the marker is determined by RT-PCR.

Gene profiling confirms that isolated placental stem cells, and populations of isolated placental stem cells, are distinguishable from other cells, e.g., mesenchymal stem cells, e.g., bone marrow-derived mesenchymal stem cells. The isolated placental stem cells described herein can be distinguished from, e.g., mesenchymal stem cells on the basis of the expression of one or more genes, the expression of which is significantly higher in the isolated placental stem cells in comparison to bone marrow-derived mesenchymal stem cells. In particular, the isolated placental stem cells, useful in the methods of treatment provided herein, can be distinguished from mesenchymal stem cells on the basis of the expression of one or more genes, the expression of which is significantly higher (that is, at least twofold higher) in the isolated placental stem cells than in an equivalent number of bone marrow-derived mesenchymal stem cells, wherein the one or more genes are ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, ZC3H12A, or a combination of any of the foregoing, when the cells are grown under equivalent conditions. See, e.g., U.S. Patent Application Publication No. 2007/0275362, the disclosure of which is incorporated herein by reference in its entirety. In certain specific embodiments, said expression of said one or more genes is determinable, e.g., by RT-PCR or microarray analysis, e.g., using a U133-A microarray (Affymetrix). In another specific embodiment, said isolated placental stem cells express said one or more genes when cultured for a number of population doublings, e.g., anywhere from about 3 to about 35 population doublings, in a medium comprising DMEM-LG (e.g., from Gibco); 2% fetal calf serum (e.g., from Hyclone Labs.); 1X insulin-transferrin-selenium (ITS); 1X linoleic acid-bovine serum albumin (LA-BSA); $10^{-9}$ M dexamethasone (e.g., from Sigma); $10^{-4}$ M ascorbic acid 2-phosphate (e.g., from Sigma); epidermal growth factor 10 ng/mL (e.g., from R&D Systems); and platelet-derived growth factor (PDGF-BB) 10 ng/mL (e.g., from R&D Systems). In another specific embodiment, the isolated placental cell-specific gene is CD200.

Specific sequences for these genes can be found in GenBank at accession nos. NM-001615 (ACTG2), BC065545 (ADARB1), (NM_181847 (AMIGO2), AY358590 (ARTS-1), BC074884 (B4GALT6), BC008396 (BCHE), BCO20196 (C11orf9), BCO31103 (CD200), NM_001845 (COL4A1), NM_001846 (COL4A2), BCO52289 (CPA4), BC094758 (DMD), AF293359 (DSC3), NM_001943 (DSG2), AF338241 (ELOVL2), AY336105 (F2RL1), NM_018215 (FLJ10781), AY416799 (GATA6), BC075798 (GPR126), NM_016235 (GPRC5B), AF340038 (ICAM1), BC000844 (IER3), BC066339 (IGFBP7), BC013142 (ILIA), BT019749 (IL6), BC007461 (IL18), (BC072017) KRT18, BC075839 (KRT8), BC060825 (LIPG), BC065240 (LRAP), BC010444 (MATN2), BC011908 (MEST), BC068455 (NFE2L3), NM_014840 (NUAK1), AB006755 (PCDH7), NM_014476 (PDLIM3), BC126199 (PKP-2), BC090862 (RTN1), BC002538 (SERPINB9), BCO23312 (ST3GAL6), BC001201 (ST6GALNAC5), BC126160 or BC065328 (SLC12A8), BCO25697 (TCF21), BC096235 (TGFB2), BC005046 (VTN), and BC005001 (ZC3H12A) as of March 2008.

In certain specific embodiments, said isolated placental stem cells express each of ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, and ZC3H12A at a detectably higher level than an equivalent number of bone marrow-derived mesenchymal stem cells, when the cells are grown under equivalent conditions.

In specific embodiments, the placental cells express CD200 and ARTS 1 (aminopeptidase regulator of type 1 tumor necrosis factor); ARTS-1 and LRAP (leukocyte-derived arginine aminopeptidase); IL6 (interleukin-6) and TGFB2 (transforming growth factor, beta 2); IL6 and KRT18 (keratin 18); IER3 (immediate early response 3), MEST (mesoderm specific transcript homolog) and TGFB2; CD200 and IER3; CD200 and IL6; CD200 and KRT18; CD200 and LRAP; CD200 and MEST; CD200 and NFE2L3 (nuclear factor (erythroid-derived 2)-like 3); or CD200 and TGFB2 at a detectably higher level than an equivalent number of bone marrow-derived mesenchymal stem cells (BM-MSCs) wherein said bone marrow-derived mesenchymal stem cells have undergone a number of passages in culture equivalent to the number of passages said isolated placental stem cells have undergone. In other specific embodiments, the placental cells express ARTS-1, CD200, IL6 and LRAP; ARTS-1, IL6, TGFB2, IER3, KRT18 and MEST; CD200, IER3, IL6, KRT18, LRAP, MEST, NFE2L3, and TGFB2; ARTS-1, CD200, IER3, IL6, KRT18, LRAP, MEST, NFE2L3, and TGFB2; or IER3, MEST and TGFB2 at a detectably higher level than an equivalent number of bone marrow-derived mesenchymal stem cells BM-MSCs, wherein said bone marrow-derived mesenchymal stem cells have undergone a number of passages in culture equivalent to the number of passages said isolated placental stem cells have undergone.

Expression of the above-referenced genes can be assessed by standard techniques. For example, probes based on the sequence of the gene(s) can be individually selected and constructed by conventional techniques. Expression of the genes can be assessed, e.g., on a microarray comprising probes to one or more of the genes, e.g. an Affymetrix GENECHIP™ Human Genome U133A 2.0 array, or an Affymetrix GENECHIP™ Human Genome U133 Plus 2.0 (Santa Clara, Calif.). Expression of these genes can be assessed even if the sequence for a particular GenBank accession number is amended because probes specific for the amended sequence can readily be generated using well-known standard techniques.

The level of expression of these genes can be used to confirm the identity of a population of isolated placental stem cells, to identify a population of cells as comprising at least a plurality of isolated placental stem cells, or the like. Populations of isolated placental stem cells, the identity of which is confirmed, can be clonal, e.g., populations of isolated placental stem cells expanded from a single isolated placental cell, or a mixed population of stem cells, e.g., a population of cells comprising solely isolated placental stem cells that are expanded from multiple isolated placental stem cells, or a population of cells comprising isolated placental stem cells, as described herein, and at least one other type of cell.

The placental stem cells can be obtained by perfusion, e.g., produced according to a method comprising perfusing a mammalian placenta that has been drained of cord blood and perfused to remove residual blood; perfusing said placenta with a perfusion solution; and collecting said perfusion solution, wherein said perfusion solution after perfusion comprises a population of placental cells that comprises placental stem cells; and isolating a plurality of said placental stem cells from said population of cells. In a specific embodiment, the perfusion solution is passed through both the umbilical vein and umbilical arteries and collected after it exudes from the placenta. Populations of placental stem cells produced by this method typically comprise a mixture of fetal and maternal cells. In another specific embodiment, the perfusion solution is passed through the umbilical vein and collected from the umbilical arteries, or passed through the umbilical arteries and collected from the umbilical vein. Populations of placental stem cells produced by this method typically are substantially exclusively fetal in origin; that is, e.g., greater than 90%, 95%, 99%, or 99.5% of the placental stem cells in the population are fetal in origin.

In various embodiments, the placental stem cells, contained within a population of cells obtained from perfusion of a placenta, are at least 50%, 60%, 70%, 80%, 90%, 95%, 99% or at least 99.5% of said population of placental cells. In another specific embodiment, the placental stem cells collected by perfusion comprise fetal and maternal cells. In another specific embodiment, the placental stem cells collected by perfusion are at least 50%, 60%, 70%, 80%, 90%, 95%, 99% or at least 99.5% fetal cells.

Placental stem cells can also be isolated from a mammalian placenta by physical disruption, e.g., enzymatic digestion, of the organ or a portion thereof. For example, the placenta, or a portion thereof, may be, e.g., crushed, sheared, minced, diced, chopped, macerated or the like, and the tissue subsequently digested with one or more enzymes. The placenta, or a portion thereof, may also be physically disrupted and digested with one or more enzymes, and the resulting material then immersed in, or mixed into, e.g., medium or buffer. Any method of physical disruption can be used, provided that the method of disruption leaves a plurality, more preferably a majority, and more preferably at least 60%, 70%, 80%, 90%, 95%, 98%, or 99% of the cells in said organ viable, as determined by, e.g., trypan blue exclusion.

The placenta can be dissected into components prior to physical disruption and/or enzymatic digestion and stem cell recovery. For example, placental stem cells can be obtained from the amniotic membrane, chorion, umbilical cord, placental cotyledons, or any combination thereof. Typically, placental stem cells can be obtained by disruption of a small block of placental tissue, e.g., a block of placental tissue that is about 1, 2, 3, 4, 5, 6, 7, 8, 9, 10, 20, 30, 40, 50, 60, 70, 80, 90, 100, 200, 300, 400, 500, 600, 700, 800, 900 or about 1000 cubic millimeters in volume.

One stem cell collection composition, useful in placental stem cells by perfusion or physical/enzymatic disruption of placental tissue, comprises one or more tissue-disruptive enzyme(s), e.g., collagenase, dispase, hyaluronidase, LIBERASE (Boehringer Mannheim Corp., Indianapolis, Ind.), papain, deoxyribonucleases, serine proteases, such as trypsin, chymotrypsin, or elastase, or the like. Any combination of tissue digestion enzymes can be used. Typical concentrations for tissue digestion enzymes include, e.g., 50-200 U/mL for collagenase I and collagenase IV, 1-10 U/mL for dispase, and 10-100 U/mL for elastase. Proteases can be used in combination, that is, two or more proteases in the same digestion reaction, or can be used sequentially in order to liberate placental stem cells. For example, in one embodiment, a placenta, or part thereof, is digested first with an appropriate amount of collagenase I at 2 mg/ml for 30 minutes, followed by digestion with trypsin, 0.25%, for 10 minutes, at 37°C Serine proteases are preferably used consecutively following use of other enzymes.

In another embodiment, the tissue can further be disrupted by the addition of a chelator, e.g., ethylene glycol bis(2-aminoethyl ether)-N,N,N'N'-tetraacetic acid (EGTA) or ethylenediaminetetraacetic acid (EDTA) to the stem cell collection composition comprising the stem cells, or to a solution in which the tissue is disrupted and/or digested prior to isolation of the stem cells with the stem cell collection composition.

Where an entire placenta, or portion of a placenta comprising both fetal and maternal cells (for example, where the portion of the placenta comprises the chorion or cotyledons), the placental stem cells collected will comprise a mix of placental stem cells derived from both fetal and maternal sources. Where a portion of the placenta that comprises no, or a negligible number of, maternal cells (for example, amnion), the placental stem cells collected will comprise almost exclusively fetal placental stem cells.

**5.7.2. Isolation and Characterization of Placental Stem Cells**

Stem cells from mammalian placenta, whether obtained by perfusion or enzymatic digestion, can initially be purified from (i.e., be isolated from) other cells by, e.g., Ficoll gradient centrifugation. Such centrifugation can follow any standard protocol for centrifugation speed, etc. In one embodiment, for example, cells collected from the placenta are recovered from perfusate by centrifugation at 5000 X g for 15 minutes at room temperature, which separates cells from, e.g., contaminating debris and platelets. In another embodiment, placental perfusate is concentrated to about 200 ml, gently layered over Ficoll, and centrifuged at about 1100 X g for 20 minutes at 22°C, and the low-density interface layer of cells is collected for further processing.

Cell pellets can be resuspended in fresh stem cell collection composition, or a medium suitable for stem cell maintenance, e.g., IMDM serum-free medium containing 2 U/ml heparin and 2 mM EDTA (GibcoBRL, NY). The total mononuclear cell fraction can be isolated, e.g., using Lymphoprep (Nycomed Pharma, Oslo, Norway) according to the manufacturer's recommended procedure.

Placental cells obtained by perfusion or digestion can, for example, be further, or initially, isolated by differential trypsinization using, e.g., a solution of 0.05% trypsin with 0.2% EDTA (Sigma, St. Louis, Missouri). Differential trypsinization is possible because placental stem cells typically detach from plastic surfaces within about five minutes whereas other adherent populations typically require more than 20-30 minutes incubation. The detached placental stem cells can be harvested following trypsinization and trypsin neutralization, using, e.g., Trypsin Neutralizer Solution (Cascade Biologics, Portland, Oregon). In one embodiment of isolation of adherent cells, aliquots of, for example, about 5-10 X $10^6$ cells are placed in each of several T-75 flasks, preferably fibronectin-coated T75 flasks. In such an embodiment, the cells can be cultured with, e.g., a commercially available mesenchymal stem cell culture medium such as MESENCULT® (Stemcell Technologies, Vancouver, BC, Canada), and placed in a tissue culture incubator (37°C, 5% $CO_2$). After 10 to 15 days, non-adherent cells are removed from the flasks by washing with PBS. The PBS is then replaced by MESENCULT® or similar medium. Flasks are preferably examined daily for the presence of various adherent cell types and in particular, for identification and expansion of clusters of fibroblastoid cells.

The number and type of cells collected from a mammalian placenta can be monitored, for example, by measuring changes in morphology and cell surface markers using standard cell detection techniques such as flow cytometry, cell sorting, immunocytochemistry (e.g., staining with tissue specific or cell-marker specific antibodies) fluorescence activated cell sorting (FACS), magnetic activated cell sorting (MACS), by examination of the morphology of cells using light or confocal microscopy, and/or by measuring changes in gene expression using techniques well known in the art, such as PCR and gene expression profiling. These techniques can be used, too, to identify cells that are positive for one or more particular markers. For example, using antibodies to CD34, one can determine, using the techniques above, whether a cell comprises a detectable amount of CD34; if so, the cell is CD34+. Likewise, if a cell produces enough OCT-4 RNA to be detectable by RT-PCR, or significantly more OCT-4 RNA than an adult cell, the cell is OCT-4+. Antibodies to cell surface markers (e.g., CD markers such as CD34) and the sequence of stem cell-specific genes, such as OCT-4, are well-known in the art.

Placental stem cells, particularly cells that have been isolated by Ficoll separation, differential adherence, or a combination of both, may be sorted using a fluorescence activated cell sorter (FACS). Fluorescence activated cell sorting (FACS) is a well-known method for separating particles, including cells, based on the fluorescent properties of the particles (see, e.g., Kamarch, 1987, Methods Enzymol, 151:150-165). Laser excitation of fluorescent moieties in the individual particles results in a small electrical charge allowing electromagnetic separation of positive and negative particles from a mixture. In one embodiment, cell surface marker-specific antibodies or ligands are labeled with distinct fluorescent labels. Cells are processed through the cell sorter, allowing separation of cells based on their ability to bind to the antibodies used. FACS sorted particles may be directly deposited into individual wells of 96-well or 384-well plates to facilitate separation and cloning. Antibodies linked to magnetic beads may also be used to sort cells.

In one sorting scheme, stem cells from placenta are sorted on the basis of expression of the markers CD34, CD38, CD44, CD45, CD73, CD105, OCT-4 and/or HLA-G. This can be accomplished in connection with procedures to select stem cells on the basis of their adherence properties in culture. For example, an adherence selection stem can be accomplished before or after sorting on the basis of marker expression. In one embodiment, for example, cells are sorted first on the basis of their expression of CD34; CD34- cells are retained, and cells that are CD200+HLA-G-, are separated from all other CD34- cells. In another embodiment, cells from placenta are based on their expression of CD200 and/or lack of expression of HLA-G; for example, cells displaying either of these markers are isolated for further use. Cells that express, e.g., CD200 and/or lack expression of HLA-G can, in a specific embodiment, be further sorted based on their expression of CD73 and/or CD105, or epitopes recognized by antibodies SH2, SH3 or SH4, or lack of expression of CD34, CD38 or CD45. For example, in one embodiment, placental cells are sorted by expression, or lack thereof, of CD200, HLA-G, CD73, CD105, CD34, CD38 and CD45, and placental cells that are CD200+, HLA-G-, CD73+, CD105+, CD34-, CD38- and CD45- are isolated from other placental cells for further use.

In another embodiment, magnetic beads can be used to separate cells. The cells may be sorted using a magnetic activated cell sorting (MACS) technique, a method for separating particles based on their ability to bind magnetic beads (0.5-100 $\mu$m diameter). A variety of useful modifications can be performed on the magnetic microspheres, including covalent addition of antibody that specifically recognizes a particular cell surface molecule or hapten. The beads are then mixed with the cells to allow binding. Cells are then passed through a magnetic field to separate out cells having the specific cell surface marker. In one embodiment, these cells can then isolated and re-mixed with magnetic beads coupled to an antibody against additional cell surface markers. The cells are again passed through a magnetic field, isolating cells that bound both the antibodies. Such cells can then be diluted into separate dishes, such as microtiter dishes for clonal isolation.

Placental stem cells can be assessed for viability, proliferation potential, and longevity using standard techniques known in the art, such as trypan blue exclusion assay, fluorescein diacetate uptake assay, propidium iodide uptake assay (to assess viability); and thymidine uptake assay, MTT cell proliferation assay (to assess proliferation). Longevity may be determined by methods well known in the art, such as by determining the maximum number of population doubling in an extended culture.

Placental stem cells can also be separated from other placental cells using other techniques known in the art, e.g., selective growth of desired cells (positive selection), selective destruction of unwanted cells (negative selection); separation based upon differential cell agglutinability in the mixed population as, for example, with soybean agglutinin; freeze-thaw procedures; filtration; conventional and zonal centrifugation; centrifugal elutriation (counter-streaming centrifugation); unit gravity separation; countercurrent distribution; electrophoresis; and the like.

### 5.7.3. Culture of Placental Stem Cells

Placental stem cells can be isolated as described above and immediately contacted with ECM. Placental stem cells can also be cultured, e.g., in cell culture, for a number of generations prior to contacting with ECM. For example, isolated placental stem cells, or placental stem cell population, or cells or placental tissue from which placental stem cells grow out, can be used to initiate, or seed, cell cultures. Cells are generally transferred to sterile tissue culture vessels either uncoated or coated with extracellular matrix or ligands such as laminin, collagen (e.g., native or denatured), gelatin, fibronectin, ornithine, vitronectin, and/or commercially-available extracellular membrane protein.

In certain embodiments, the placental stem cells are cultured on ECM, e.g., the ECM provided herein. In certain embodiments, the ECM comprises detectable amounts of fibronectin and laminin. In other embodiments, the ECM comprises no detectable amount of fibronectin or laminin. In other embodiments, the ECM comprises at least about 5%, or at least about 10%, elastin by dry weight. In another embodiment, the ECM comprises no more than about 5% elastin by dry weight.

In certain embodiments, placental stem cells are cultured for the production of specific cytokines that are collectable from the culture medium. In specific embodiments, the cytokine is IL-6, IL-8, and/or monocyte chemotactic protein-1 (MCP-1). In certain other embodiments, the placental stem cells are cultured for the production of fibronectin. In a specific embodiment, the placental stem cells are cultured on ECM which comprises less than about 5% fibronectin.

As noted above, ECM can be shaped into any shape that is useful, e.g., medically useful. These compositions, once shaped and dried, are typically stable in aqueous solution, e.g., tissue culture medium or buffer. Thus, stem cells, such as placental stem cells, can be cultured directly on the shaped compositions. Such culturing can be done in cell culture dishes or other liquid containers, e.g., flasks, suitable for cell culture.

Placental stem cells can be cultured in any medium, and under any conditions, recognized in the art as acceptable for the culture of stem cells. In certain embodiments, the culture medium comprises serum, e.g., human serum or bovine calf serum/fetal calf serum. In certain other embodiments, the culture medium is serum-free. Placental stem cells can be cultured in, for example, DMEM-LG (Dulbecco's Modified Essential Medium, low glucose)/MCDB 201 (chick fibroblast basal medium) containing ITS (insulin-transferrin-selenium), LA+BSA (linoleic acid-bovine serum albumin), dextrose, L-ascorbic acid, PDGF, EGF, IGF-1, and penicillin/streptomycin; DMEM-HG (high glucose) comprising 10% fetal bovine serum (FBS); DMEM-HG comprising 15% FBS; IMDM (Iscove's modified Dulbecco's medium) comprising 10% FBS, 10% horse serum, and hydrocortisone; M199 comprising 10% FBS, EGF, and heparin; {umlaut over (.gamma.)}-MEM (minimal essential medium) comprising 10% FBS, GLUTAMAX.TM. and gentamicin; DMEM comprising 10% FBS, GLUTAMAX.TM. and gentamicin, etc. In one embodiment, the medium is DMEM-LG/MCDB-201 comprising 2% FBS, ITS, LA+BSA, dextrose, L-ascorbic acid, PDGF, EGF, and penicillin/streptomycin.

Other media in that can be used to culture placental stem cells include DMEM (high or low glucose), Eagle's basal medium, Ham's F10 medium (F10), Ham's F-12 medium (F12), Iscove's modified Dulbecco's medium, Mesenchymal Stem Cell Growth Medium (MSCGM), Liebovitz's L-15 medium, MCDB, DMEM/F12, RPMI 1640, advanced DMEM (Gibco), DMEM/MCDB201 (Sigma), and CELL-GRO FREE.

The culture medium can be supplemented with one or more components including, for example, serum (e.g., fetal bovine serum (FBS), preferably about 2-15% (v/v); equine (horse) serum (ES); human serum (HS)); beta-mercaptoethanol (BME), preferably about 0.001% (v/v); one or more growth factors, for example, platelet-derived growth factor (PDGF), epidermal growth factor (EGF), basic fibroblast growth factor (bFGF), insulin-like growth factor-1 (IGF-1), leukemia inhibitory factor (LIF), vascular endothelial growth factor (VEGF), and erythropoietin (EPO); amino acids, including L-valine; and one or more antibiotic and/or antimycotic agents to control microbial contamination, such as, for example, penicillin G, streptomycin sulfate, amphotericin B, gentamicin, and nystatin, either alone or in combination.

Placental stem cells can be cultured in standard tissue culture conditions, e.g., in tissue culture dishes or multiwell plates. Placental stem cells can also be cultured using a hanging drop method. In this method, placental stem cells are suspended at about 1 X $10^4$ cells per mL in about 5 mL of medium, and one or more drops of the medium are placed on the inside of the lid of a tissue culture container, e.g., a 100 mL Petri dish. The drops can be, e.g., single drops, or multiple drops from, e.g., a multichannel pipetter. The lid is carefully inverted and placed on top of the bottom of the dish, which contains a volume of liquid, e.g., sterile PBS sufficient to maintain the moisture content in the dish atmosphere, and the stem cells are cultured.

Once an isolated placental stem cell, or isolated population of stem cells comprising placental stem cells (e.g., a stem cell or population of stem cells separated from at least 50% of the placental cells with which the stem cell or population of stem cells is normally associated in vivo) is obtained, the placental stem cells or population of cells can be proliferated and expanded in vitro. For example, placental stem cells can be cultured in tissue culture containers, e.g., dishes, flasks, multiwell plates, or the like, for a sufficient time for the stem cells to proliferate to 70-90% confluence, that is, until the stem cells and their progeny occupy 70-90% of the culturing surface area of the tissue culture container.

Placental stem cells can be seeded in culture vessels at a density that allows cell growth. For example, the cells may be seeded at low density (e.g., about 1,000 to about 5,000 cells/cm$^2$) to high density (e.g., about 50,000 or more cells/cm$^2$). In a preferred embodiment, the cells are cultured at about 0 to about 5 percent by volume $CO_2$ in air. In some preferred embodiments, the cells are cultured at about 2 to about 25 percent $O_2$ in air, preferably about 5 to about 20 percent $O_2$ in air. The cells preferably are cultured at about 25°C to about 40°C, preferably 37°C. The cells are preferably cultured in an incubator. The culture medium can be static or agitated, for example, using a bioreactor. Placental stem cells may be grown under low oxidative stress (e.g., with addition of glutathione, ascorbic acid, catalase, tocopherol, N-acetylcysteine, or the like).

Once 70%-90% confluence is obtained, the cells may be passaged. For example, the cells can be enzymatically treated, e.g., trypsinized, using techniques well-known in the art, to separate them from the tissue culture surface. After removing the cells by pipetting and counting the cells, about 20,000-100,000 stem cells, preferably about 50,000 stem cells, are passaged to a new culture container containing fresh culture medium. Typically, the new medium is the same type of medium from which the stem cells were removed. Placental stem cells that have been passaged at least 1, 2, 3, 4, 5, 6, 7, 8, 9, 10, 12, 14, 16, 18, or 20 times, or more, can be used in combination with the ECM.

## 5.8. Non-Stem Cells

The compositions used in the methods described herein can, in certain embodiments, comprise one or more types of non-stem cells. As used herein, "non-stem cell" indicates a terminally-differentiated cell. For example, in one embodiment, a composition comprises a plurality of fibroblasts. Non-stem cells that can be combined with the compositions include, without limitation, fibroblasts or fibroblast-like cells, dermal cells, endothelial cells, epithelial cells, muscle cells, cardiac cells, pancreatic cells, and the like. In certain other embodiments, the composition comprises at least two types of stem cells and at least two types of non-stem cells.

For any of the above embodiments in which a composition is combined with stem cells or non-stem cells, the cells and the composition can be administered to an individual together, e.g., as a unitary composition. For example, in one embodiment, the composition can comprise stem cells that have been contacted with the composition immediately prior (e.g., within 10-20 minutes) of administering the composition to the individual. In another embodiment, the stem cells can be contacted with the composition at a time prior to administration sufficient to allow the stem cells to attach to the composition, typically at least 1 hour prior to administration. In a more specific embodiment, the time prior to administration is a time sufficient for the stem cells to attach and proliferate, typically at least 24 hours to 48 hours, or more, prior to administration. In another more specific embodiment, the time is a time sufficient for the stem cells to attach to, and proliferate on, a solid formed composition, and to deposit a detectable amount of an extracellular matrix protein, e.g., fibronectin.

The compositions, and stem cells or non-stem cells, can be administered to the individual separately, as well. For instance, in one embodiment, the composition can be administered to an individual, e.g., at the site of a wound or tissue needing repair, and the stem cells can be subsequently administered. In another embodiment, the stem cells are contacted with the site of an oral lesion, and the wound or tissue needing repair is subsequently contacted with a composition described herein.

In one embodiment, therefore, provided herein is a method of promoting the healing of an oral lesion, comprising contacting the lesion with a composition described herein comprising stem cells, e.g., placental stem cells, wherein the stem cells secrete IL-6, IL-8 or MCP-1, or a any combination thereof, or secrete fibronectin, into at least a portion of the lesion. Where the stem cells are to secrete fibronectin, it is preferred that the composition comprise an undetectable amount of fibronectin. In a specific embodiment, the composition is shaped or formed approximately to the shape of the oral lesion.

## 5.9. Kits

In another aspect provided herein are kits comprising the compositions described herein, and additional components, to facilitate treatment of an oral lesion. In certain embodiments, the kit comprises one or more packages of a composition described herein for distribution to a practitioner of skill in the art. The kits can comprise a label or labeling with instructions on using the composition in the treatment of an oral lesion. In certain embodiments, the kits can comprise components useful for carrying out the methods such as means for administering a collagen composition such as one or more spray bottles, tweezers, spatula (for applying paste), cannulas, catheters, etc. In certain embodiments, the kit can comprise one or more components useful for the safe disposal of means for administering the composition (e.g. a 'sharps' container). In certain embodiments, the kits can comprise compositions (e.g., compositions comprising ECM and/or ECM components) in pre-filled syringes, unit-dose or unit-of-use packages.

In certain other embodiments, the kit comprises a composition described herein and one or more other components for the culture of a population of stem cells or non-stem cells. For example, the kit can comprise a composition described herein in one or more configurations suitable for the culture of stem cells, e.g., placental stem cells, e.g., a composition in the form of a sheet, tube, mesh, and the like. The kit can comprise one or more items to be used for the culture of stem cells, e.g., culture dishes that are able to contain a composition described herein during cell culture; plasticware, syringes, pipet tips, cell culture media, one or more cytokines or growth factors, disposables, and the like.

In other embodiments, the kit can comprise one or more components that facilitate the collection of stem cells from placental tissue. In various specific embodiments, the kit comprises components that facilitate perfusion of a placenta to collect stem cells, e.g., perfusion solution; one or more trays large enough to contain a placenta, glassware or plasticware for collection of perfusion solution; one or more bags for collection of perfusion solution, needs and/or canulae for canalizing umbilical vessels; proteases suitable for tissue digestion, implements for macerating or otherwise rendering placental tissue, and the like. In other specific embodiments, the kit comprises one or more components that facilitate enzymatic digestion of placental tissue to isolate placental stem cells, e.g., one or more tissue-digesting enzymes (e.g., trypsin, chymotrypsin, or the like); plasticware suitable for cell culture (e.g., culture dishes, multiwell culture plates, and the like).

## 6. EXAMPLES

In the sections below, those of skill in the art will recognize that the phrase "at approximately 23°C" can refer to room temperature.

### 6.1. Example 1: Isolation of ECM from Placentas

This example illustrates isolation of ECM from placentas.

Frozen placentas are obtained according to the methods described herein. The placentas are thawed by wrapping in a Nalgene tray with water for 1-4 hrs. They are then removed from plastic wrap and placed in water for further thawing.

Thawed placentas are placed on the stainless steel tray of a meat grinder. The umbilical cord fragment is cut from each placenta, and each placenta is sliced into about 4 strips at approximately 23°C The strips are ground with the meat grinder at approximately 23°C.

Osmotic Shock: The resulting ground placentas are added to a Nalgene tank with 0.5 M NaCl (5 liters/placenta) and mixed using a motorized mixer at 75-100 rpm (24 hrs at 4-6°C).

After 24 hrs, the mixer is stopped, allowing tissue to settle to the bottom of the mixer at approximately 23°C Tissue and fluid are pumped out using a peristaltic pump with #36 TYGON™ tubing and filtered through a #40 sieve at approximately 23°C, and isolated tissue is placed back into the mixing tank.

Fresh 0.5 M NaCl (5 L/placenta) is added to the mixture and mixed for 24 hrs at 4-6°C (motorized mixer, 75-100 rpm). After 24 hrs, the tissue is isolated using the method described above.

Tissue is washed with water (5-L/placenta) and mixed for 24 hrs at 4-6°C (motorized mixer, 75-100 rpm). After 24 hrs, the tissue is isolated using the method described above.

The tissue is further washed again with 0.5 M NaCl, again with 0.5 M NaCl and then water according to the above four paragraphs.

Freeze-drying: The resulting sample is shelled in 200-400 g amounts in a freeze-dryer vessel and frozen at -70°C for 1-2 hrs. The frozen sample is freeze-dried for 24-48 hrs in a freeze-drier and then removed. The freeze-dried sample is mixed to a smooth powder in a blender and then transferred to a clean mixing tank.

Detergent treatment: A 1% deoxycholic acid solution (IL/placenta) is added to the mixing tank with the blended, freeze-dried sample. The sample and 1% deoxycholic acid solution are mixed for 24 hrs at 4-6°C (motorized mixer, 75-100 rpm). After 24 hrs, the mixer is stopped, and tissue is isolated with a #40 sieve as described above.

The detergent treatment is repeated for 24 hrs at 4-6°C (motorized mixer, 75-100 rpm). After 24 hrs, the mixer is stopped, and tissue is isolated with a #40 sieve as described above.

Water wash: Tissue is washed with water (5 L/placenta) and mixed for 24 hrs at 4-6°C (motorized mixer, 75-100 rpm). After 24 hrs, the tissue is isolated using the method described above.

Tissue is again washed with water (5 L/placenta) and mixed for 24 hrs at 4-6°C (motorized mixer, 100-150 rpm). After 24 hrs, the tissue is isolated using the method described above.

Tissue is again washed with water (5 L/placenta) and mixed for 24 hrs at 4-6°C (motorized mixer, 150 rpm). After 24 hrs, the tissue is isolated using the method described above.

Optionally, tissue is washed with water (5 L/placenta) a fourth time and mixed for 24 hrs at 4-6°C (motorized mixer, 150 rpm). After 24 hrs, the tissue is isolated using the method described above.

Freeze-drying: The resulting sample is added to a blender in 200 g amounts. 200 mL deionized water is added to the sample, and the sample is mixed to a smooth paste with the blender. Blended samples are pooled and rinsed with water (1 L/placenta).

Sample in 200-400 g amounts is added to a freeze-dryer vessel. Samples are shelled and frozen at -70°C. Shelled samples are freeze dried for 24-48 hrs.

Sterile basic treatment: Freeze-dried samples are pooled. Sodium hydroxide solution (0.5 M, IL) is added to an autoclaved, sterile flask. Low endotoxin water (IL) is added to the pooled, freeze-dried samples. The samples and sodium hydroxide solution are mixed on a shaker at 250 rpm for 4 hrs at approximately 23°C.

Sterile water wash: The sample is recovered by filtration through a sterile #70 filter and rinsed with 1 L endotoxin free water. Endotoxin free water (1 L) is added, and sample is mixed on a shaker at 250 rpm for 18-24 hrs at approximately 23°C.

The sample is recovered by filtration through a sterile #70 filter. Endotoxin free water (1 L) is added, and sample is mixed on a shaker at 250 rpm for 18-24 hrs at approximately 23°C.

The sample is recovered by filtration through a sterile #70 filter and rinsed with 1 L endotoxin free water. Endotoxin free water (1 L) is added, and sample is mixed on a shaker at 250 rpm for 18-24 hrs at approximately 23°C.

If the pH is greater than 9, the sample is washed again with endotoxin-free water and mixed on a shaker at about 250 RPM for about 18-24 hours.

If the pH is less than or equal to 9, the sample is ready for formulation. The yield can be 10 g/placenta or more.

The resulting ECM can be freeze-dried for storage. For use, the sample can be suspended in phosphate-buffered saline at 300-1000 mg/mL in a blender for use as a paste in, for example, a syringe. The sample can also be molded in phosphate buffered saline at 500-1000 mg/mL and shaped for use as for example, sheets, tubes, plugs, or the like.

## 6.2. Example 2: Preparation of ECM Comprising Telopeptide Collagen

7.5 g of ECM comprising telopeptide collagen was prepared according to the osmotic shock, freeze-drying, detergent treatment, water wash, freeze-drying, basic treatment, water wash and freeze-drying steps of Example 1.

11.8 g of ECM comprising telopeptide collagen was prepared according to the osmotic shock, freeze-drying, detergent treatment, water wash, basic treatment, water wash and freeze-drying steps of Example 1.

12.0 g of ECM comprising telopeptide collagen was prepared according to the osmotic shock, freeze-drying, detergent treatment, water wash, basic treatment, water wash and freeze-drying steps of Example 1.

11.8 g of ECM comprising telopeptide collagen was prepared according to the osmotic shock, detergent treatment, water wash, basic treatment, water wash and freeze-drying steps of Example 1.

## 6.3. Example 3: Biochemical Analysis

ECM was prepared according to Examples 1 and 2. Biochemical analysis by standard techniques showed by dry weight 80.40% collagen, 1.00% water and less than 0.01% fibronectin, laminin and glycosaminoglycans. Elastin content was not determined.

Amino acid analysis of samples prepared according to Examples 1 and 2 showed 34-35% glycine, about 11% hydroxyproline and 10-11% proline.

Immunoanalysis of samples prepared according to Examples 1 and 2 showed that, of total collagen, 74-92% was type I collagen, 4-6% was type III collagen and 2-15% was type IV collagen.

## 6.4. Example 4: Alternate Methods of Making ECM, and Culture of Stem Cells on the ECM

This Example demonstrates alternate methods of making ECM, and provides an analysis of the composition of the materials made by those methods.

Materials and Methods

Isolation of Extracellular Matrix (ECM): ECM was isolated as follows. Briefly, a frozen human placenta was thawed in 0.5M sodium chloride, ground in a meat grinder and washed repeatedly in 0.5M sodium chloride and water in a incubator shaker at 23°C, followed by a detergent such as 1% SDS or 0.5% deoxycholic acid. Exsanguinated placental tissue was treated with 0.1-0.5N sodium hydroxide for times varying between 3 hours and 24 hours to solubilize the cotyledonous tissue, following by rinsing with phosphate-buffered saline (PBS) to neutralize the pH. The material produced as such was a stable paste and was stored at 4°C.

Biochemical Analysis: To determine the biochemical composition of the isolated ECM, a 1 gram sample was freeze-dried and dry weight determined. The ECM was solubilized by either dissolving in 100 mM HCl at 70°C or by pepsin treatment (1 mg/gm) of the ECM in 10 mM HCl at 23°C for 18 hrs. The tissue dissolved in 100 mM HCl was used to determine content of fibronectin, laminin, GAGs and elastin. The pepsin-solubilized tissue was used to determine collagen content.

Fibronectin and laminin concentrations were determined using a sandwich ELISA. Elastin and glycosaminoglycan (GAG) content were determined using a dye based assay. For Determination of collagen I content was performed using a sandwich ELISA (Chondrex). Collagen III and IV content were determined using in-house ELISAs using primary antibodies for Type II and Type IV collagen and HRP-conjugated secondary antibodies.

Preparation of ECM Constructs: to Prepare Sheets of the ECM, a layer of hydrated ECM paste was sandwiched between two medical grade TYVEK™ sheets. This construct was loaded into a gel drier and vacuum was applied overnight at 23°C until the ECM film was dry. Sheets were cut to an appropriate size for cell culture studies. To prepare 3D structures of the ECM, ECM paste was filled into various molds and freeze-dried. To study the stability of the ECM sheets and 3D molds in media or water, the constructs were incubated at 37°C up to 1 week, in water, saline or cell culture media.

Cell culture: Placental stem cells were subcultured in 60% low-glucose DMEM (Invitrogen, Carlsbad, Calif.), 40% MCDB-201 (Sigma, St. Louis, Mo.), 2% fetal bovine serum (Hyclone, Logan, Utah), 1X insulin-transferrin-selenium supplement (Invitrogen), 0.02% linoleic acid/bovine serum albumin (Sigma), 10 ng/mL epidermal growth factor (Sigma), 10 ng/mL platelet-derived growth factor (R&D Systems, Minneapolis, Minn.), 0.05M dexamethasone (Sigma), 0.1 mM ascorbic acid 2-phosphate (Sigma), and 100 U penicillin/1000 U streptomycin (Invitrogen). Placental stem cells (30,000 per well) were seeded onto ECM films that had been positioned into 24 multi-well cluster plates. Placental stem cells were also seeded at equivalent density on Labtek chamber slides (Nalgene Nunc International, Rochester, N.Y.) pre-coated with collagen (Inamed, Fremont, Calif.). Cells were incubated at 37°C for 3 and 48 hours and processed for immunofluorescence microscopy.

Immunofluorescence microscopy: After 3 or 48 hr incubation with ECM films, placental stem cell-ECM constructs were fixed with 3.7% formaldehyde for 10 minutes and permeabilized with 0.5% Triton-X 100 for 20 minutes. Placental stem cells were incubated with AlexaFluor 488-conjugated phalloidin to visualize F-actin. For fibronectin staining, samples incubated with a rabbit anti-human fibronectin antibody (Sigma) in blocking buffer (3% bovine serum albumin/IX phosphate-buffered saline) for 1 hour, washed with phosphate-buffered saline, and further incubated with an AlexaFluor 594-conjugated anti-rabbit antibody in blocking buffer for 30 minutes. Samples were again washed with phosphate-buffered saline, mounted on slides, and observed with a fluorescent microscope.

Cytokine secretion analysis: Media samples (100 µl) were removed from cell cultures ECM sheets containing placental stem cells, as well as from tissue culture treated plates containing placental stem cell, at 0, 3, 24 and 48 hrs of culture. Samples were diluted into 1 mL PBS and analyzed for the presence of cytokines. Concentration of each cytokine was calculated from a standard plot of known concentrations of cytokines.

Results

Isolation of ECM: The dry weight of a typical placenta is about 30 g, corresponding to a wet weight of about 300 g per placenta. As shown in FIG. 1, the osmotic shock step and detergent washing step can be used to remove a considerable amount of non-extracellular matrix tissue, with a final residual weight of about 10 g. The use of a combination of solubilization using NaOH and detergent results in a further decrease in the residual weight to about 6 g. It was found that the time of exposure to NaOH, and the concentration of NaOH, affected the total mass of ECM isolated from the placenta. Variations of our detergent and NaOH wash steps were used to generate 5 variations of the final ECM material. Typically, a single placenta yielded between about 6 g to about 10 g of ECM material.

Biochemical Composition of ECM. Biochemical analysis of the 5 variations of the ECMs (designated ECM-1 to ECM-5) showed that they were composed essentially of collagens; Type I being the major collagen (about 74% to about 90% of total collagen), and Type III (about 4% to about 6% of total collagen) and Type IV (about 2% to about 15% of total collagen) being minor components. The other major extracellular matrix protein found in the placental ECM was elastin. As shown in Table 1, elastin represented about 3-5% of the total dry weight of ECM-1 to ECM-4. However, ECM-5, which was generated without the use of NaOH, contained approximately 12% elastin. While glycosaminoglycans were identified in ECM made by all five methods, the percent of dry weight appeared to be unaffected by the use of NaOH in the isolation methods. The presence of the important adhesion proteins fibronectin and laminin, conversely, was dramatically sensitive to the use of NaOH. Fibronectin and laminin did not survive the NaOH treatment, and could not be found in ECM-1 through ECM-4. However, ECM-5, which was isolated without the use of NaOH, has a composition that is richer in the adhesion proteins (Table 1).

TABLE 1 Extracellular matrix components present in placental collagen compositions made by different methods.

|  | Fibronectin | Laminin | GAGs | Elastin |
|---|---|---|---|---|
| ECM-5 | 0.6% | 0.16% | 0.40% | 12% |
| ECM-4 | 0 | 0 | 0.28% | 4.7% |
| ECM-3 | 0 | 0 | 0.34% | 3.2% |
| ECM-2 | 0 | 0 | 0.38% | 4.4% |
| ECM-1 | 0 | 0 | 0.59% | 3.5% |
| % = percent dry weight | | | | |

Cell Binding Studies: Three hours after seeding, similar levels of attachment of placental stem cells were observed on all ECMs (#1-5). The levels of stem cell binding to ECMs were slightly less than that observed on purified collagen. Immunostaining for fibronectin at this time revealed abundant intracellular staining, with no detectable extracellular fibronectin. By 48 hours of culture, placental stem cells were observed to increase in number and to adopt similar well-spread morphologies on purified collagen, ECM-2, and ECM-4. In contrast, placental stem cells cultured on ECM-1 did not thrive. Not only were fewer cells observed, but their morphologies were rounded and not well-spread. Placental stem cells on ECM-5 appeared more elongated and polarized than placental stem cells on other ECMs or on collagen.

Determination of cell attachment on ECM-3 was somewhat compromised due to the heterogeneity of the surface of the material upon drying. Because it was difficult to image along one plane of focus, it initially appeared that very few cells attached; however observation in different planes of focus revealed some cell attachment on ECM-3.

Immunostaining for fibronectin at the 48 hr timepoint revealed an extensive network of extracellular fibronectin matrix fibrils on ECM-1 through ECM-4. These fibronectin matrix fibrils were assembled by placental stem cells, as controls in which placental stem cells were not cultured on ECM did not show evidence of fibronectin fibrils. In contrast to ECM-1 through ECM-4, ECM-5 and collagen did not support fibronectin matrix assembly by placental stem cells; no extracellular fibrillar fibronectin was detected on these surfaces.

Cytokine array studies: the secretion of key cytokines/chemokines from the placental stem cells as a consequence of binding and proliferation on the ECM was investigated. Cytokine secretion on ECM was compared to that from placental stem cells incubated on tissue culture treated cell culture plates. A standard a 25-multiplex cytokine array, which includes several interleukins and cytokines (Biosource), was used. The cytokines included IL-1$\beta$, IL-IRa, IL-2R, IL-4, IL-5, IL-6, IL-7, IL-8, IL-10, IL-12p40/p70, IL-13, IL-15, IL-17, TNF-$\beta$, IFN-$\beta$, IFN-$\gamma$, GM-CSF, MIP-1$\alpha$, MIP-1$\beta$, IP-10, MIG, Eotaxin, Rantes, and MCP-1. Of the 25 cytokines studied, increased secretion of 3 cytokines, IL-6, IL-8 and MCP-1 were observed when placental stem cells were cultured on the ECM sheets, over and above secretion by placental stem cells cultured on tissue culture treated plates. FIGS. 2A-2C show a time-dependent increase in cytokine secretion (IL-6, IL-8 & MCP-1) by placental stem cells on the five ECM constructs. All data was normalized for 1000 cells bound/cm$^2$. ECM-5 was anomalous in that there was no apparent increase in MCP-1 secretion, suggesting a change in cellular physiology of the placental stem cells when cultured on this extra-cellular matrix. As previously shown, ECM-5 did not support the expression of fibronectin, unlike ECM-1 through-4. It is interesting to note that ECM-5 was the only matrix generated without the use of NaOH and had a biochemical composition that maintained the 2 key cell adhesion proteins fibronectin and laminin.

All publications and patent applications cited in this specification are herein incorporated by reference as if each individual publication or patent application were specifically and individually indicated to be incorporated by reference. Although the foregoing has been described in some detail by way of illustration and example for purposes of clarity of understanding, it will be readily apparent to those of ordinary skill in the art in light of the teachings provided herein that certain changes and modifications may be made thereto without departing from the spirit or scope of the appended claims.

The following numbered paragraphs (paras.) contain further statements of various aspects of the present invention:-

1. A method of treating an individual having an oral lesion, wherein said oral lesion is not caused by a dental procedure, comprising administering to the oral lesion a therapeutically effective amount of extracellular matrix.

2. The method of para 1, wherein said extracellular matrix is human placental extracellular matrix that has not been chemically modified or contacted with an exogenous protease.

3. The method of para 2, wherein said human placental extracellular matrix has been prepared prior to said administration by treatment with a detergent but not with a base.

4. The method of para 2, wherein said human placental extracellular matrix has been prepared prior to said administration by treatment with a detergent and with a base.

5. The method of para 2, wherein said human placental extracellular matrix comprises collagen and one or more of laminin, fibronectin, elastin, and glycosaminoglycan.

6. The method of para 2, wherein collagen in said human placental extracellular matrix comprises between 74% and 92% Type I collagen by dry weight.

7. The method of para 2, wherein collagen in said human placental extracellular matrix comprises between 4% to 6% Type III collagen by dry weight.

8. The method of para 2, wherein collagen in said human placental extracellular matrix comprises between 2% to 15% Type IV collagen by dry weight.

9. The method of para 2, wherein said human placental extracellular matrix comprises less than 0.01% laminin or 0.01% fibronectin by dry weight.

10. The method of para 2, wherein said human placental extracellular matrix comprises between 3% and 5% elastin by dry weight.

11. The method of para 2, wherein collagen said human placental extracellular matrix is preparable by a method comprising, in order, the steps of: (a) macerating placental tissue; (b) suspending the placental tissue in a hypotonic saline solution; (c) treating the placental tissue with a detergent; and (d) treating the placental tissue with a base.

12. The method of para 11, wherein said base is ammonium hydroxide, potassium hydroxide, or sodium hydroxide.

13. The method of para 11, wherein said hypertonic saline solution comprises sodium chloride.

14. The method of para 11, wherein said detergent is or comprises deoxycholate or deoxycholic acid.

15. The method of para 1, wherein said oral lesion is caused by or is associated with administration of a chemotherapeutic agent to said individual.

16. The method of para 15, wherein said chemotherapeutic agent is an alkylating agent.

17. The method of para 16, wherein said alkylating agent is one or more of melphalan, busulfan, cisplatin, carboplatin, cyclophosphamide, dacarbazine, ifosfamide, or mechlorethamine.

18. The method of para 15, wherein said chemotherapeutic agent is an anti-metabolite.

19. The method of para 18, wherein said anti-metabolite is one or more of 5-fluorouracil, methotrexate, gemcitabine, cytarabine, or fludarabine.

20. The method of para 15, wherein said chemotherapeutic agent is an antibiotic having anti-tumor effect.

21. The method of para 20, wherein said antibiotic having anti-tumor effect is one or more of bleomycin, dactinomycin, daunorubicin, doxorubicin, or idarubicin.

22. The method of para 15, wherein said chemotherapeutic agent is a mitotic inhibitor.

23. The method of para 22, wherein said mitotic inhibitor is one or more of paclitaxel, docetaxel, etoposide, vinblastine, vincristine or vinorelbine.

24. The method of para 15, wherein said oral lesion, or a plurality of said oral lesions, has caused or is expected to cause premature termination of a course of therapy comprising said chemotherapeutic agent.

25. The method of para 1, wherein said oral lesion is caused by or is associated with administration of an antibody to said individual.

26. The method of para 25, wherein said antibody is one or more of rituximab, ofatumumab, veltuzumab, ocrelizumab, adalimumab, etanercept, infliximab, certolizumab pegol, natalizumab or golimumab.

27. The method of para 1, wherein said oral lesion is caused by or is associated with hematopoietic stem cell transplantation, or bone marrow transplantation, to said individual.

28. The method of para 1, wherein said oral lesion is caused by or is associated with graft-versus-host disease in said individual.

29. The method of para 1, wherein said oral lesion is caused by or is associated with radiation that has been administered to said individual.

30. The method of para 1, wherein said oral lesion is caused by a desquamating oral disorder.

31. The method of para 1, wherein said oral lesion is an aphthous ulcer.

32. The method of any of paras 1-31, wherein said ECM comprises a plurality of stem cells.

33. The method of claim 32, wherein said stem cells are CD10+, CD34-CD105+, CD100+ placental stem cells.

34. The method of any of paras 1 to 33, wherein said administration of said ECM results in an improvement of said oral lesion of at least one Grade according to the World Health Organization Oral Toxicity (WHO-OT) score within 7 days post-administration.

35. The method of any of paras 1 to 33, wherein said administration of said ECM results in an improvement of said oral lesion of at least one Grade according to the National Cancer Institute Common Toxicity Criteria (NCI-CTC) for Oral Mucositis within 7 days post-administration.

36. The method of any of paras 1 to 33, wherein said administration of said ECM results in an improvement of said oral lesion of at least 1 point in any subscore of the Oral Mucositis Assessment Scale (OMAS) 7 days post-administration.

37. The method of any of paras 1 to 33, wherein said administration of said ECM results in an improvement of said oral lesion of at least one Stage according to the Western Consortium for Cancer Nursing Research (WCCNR) score within 7 days post-administration.

**Claims**

1. An extracellular matrix for use in a method of treating an individual having an oral lesion, wherein said oral lesion is not caused by a dental procedure, the method comprising administering to the oral lesion a therapeutically effective amount of the extracellular matrix.

2. The extracellular matrix for the use of claim 1, wherein said extracellular matrix is human placental extracellular matrix that has not been chemically modified or contacted with an exogenous protease, optionally:

   (a) wherein said human placental extracellular matrix has been prepared prior to said administration by treatment with a detergent but not with a base; or
   (b) wherein said human placental extracellular matrix has been prepared prior to said administration by treatment with a detergent and with a base.

3. The extracellular matrix for the use of claim 2, wherein said human placental extracellular matrix comprises collagen and one or more of laminin, fibronectin, elastin, and glycosaminoglycan, optionally wherein collagen in said human placental extracellular matrix comprises between 74% and 92% Type I collagen by dry weight, or wherein collagen in said human placental extracellular matrix comprises between 4% to 6% Type III collagen by dry weight, or wherein collagen in said human placental extracellular matrix comprises between 2% to 15% Type IV collagen by dry weight.

4. The extracellular matrix for the use of claim 2, wherein said human placental extracellular matrix comprises:

   (a) less than 0.01% laminin or 0.01% fibronectin by dry weight; and/or
   (b) between 3% and 5% elastin by dry weight.

5. The extracellular matrix for the use of claim 2, wherein said human placental extracellular matrix is preparable by a method comprising, in order, the steps of: (a) macerating placental tissue; (b) suspending the placental tissue in a hypotonic saline solution; (c) treating the placental tissue with a detergent; and (d) treating the placental tissue with a base.

6. The extracellular matrix for the use of claim 5, wherein:

   (a) said base is ammonium hydroxide, potassium hydroxide, or sodium hydroxide; and/or
   (b) said hypertonic saline solution comprises sodium chloride; and/or
   (c) said detergent is or comprises deoxycholate or deoxycholic acid.

7. The extracellular matrix for the use of claim 1, wherein said oral lesion is caused by or is associated with administration of a chemotherapeutic agent to said individual, optionally wherein:

   (a) said chemotherapeutic agent is an alkylating agent; optionally wherein said alkylating agent is one or more of melphalan, busulfan, cisplatin, carboplatin, cyclophosphamide, dacarbazine, ifosfamide, or mechlorethamine; or
   (b) said chemotherapeutic agent is an antimetabolite; optionally wherein said anti-metabolite is one or more of 5-fluorouracil, methotrexate, gemcitabine, cytarabine, or fludarabine; or
   (c) said chemotherapeutic agent is an antibiotic having anti-tumor effect; optionally wherein said antibiotic having anti-tumor effect is one or more of bleomycin, dactinomycin, daunorubicin, doxorubicin, or idarubicin; or
   (d) said chemotherapeutic agent is a mitotic inhibitor; optionally wherein said mitotic inhibitor is one or more of paclitaxel, docetaxel, etoposide, vinblastine, vincristine or vinorelbine.

8. The extracellular matrix for the use of claim 7, wherein said oral lesion, or a plurality of said oral lesions, has caused or is expected to cause premature termination of a course of therapy comprising said chemotherapeutic agent.

9. The extracellular matrix for the use of claim 1, wherein said oral lesion is caused by or is associated with:

(a) administration of an antibody to said individual; optionally wherein said antibody is one or more of rituximab, ofatumumab, veltuzumab, ocrelizumab, adalimumab, etanercept, infliximab, certolizumab pegol, natalizumab or golimumab;
(b) hematopoietic stem cell transplantation, or bone marrow transplantation, to said individual;
(c) graft-versus-host disease in said individual; or
(d) radiation that has been administered to said individual.

10. The extracellular matrix for the use of claim 1, wherein said oral lesion is caused by a desquamating oral disorder; optionally wherein said oral lesion is an aphthous ulcer.

11. The extracellular matrix for the use of any of claims 1-10, wherein said ECM comprises a plurality of stem cells; optionally wherein said stem cells are CD 10+, CD34-CD105+, CD 100+ placental stem cells.

12. The extracellular matrix for the use of any of claims 1 to 11, wherein said administration of said ECM results in:

(a) an improvement of said oral lesion of at least one Grade according to the World Health Organization Oral Toxicity (WHO-OT) score within 7 days post-administration; or
(b) an improvement of said oral lesion of at least one Grade according to the National Cancer Institute Common Toxicity Criteria (NCI-CTC) for Oral Mucositis within 7 days post-administration; or
(c) an improvement of said oral lesion of at least 1 point in any subscore of the Oral Mucositis Assessment Scale (OMAS) 7 days post-administration; or
(d) an improvement of said oral lesion of at least one Stage according to the Western Consortium for Cancer Nursing Research (WCCNR) score within 7 days post-administration.

13. A method for preparing human placental extracellular matrix suitable for use according to any one of claims 1-12 comprising, in order, the steps of: (a) macerating placental tissue; (b) suspending the placental tissue in a hypotonic saline solution; (c) treating the placental tissue with a detergent; and (d) treating the placental tissue with a base.

14. The method of claim 13, wherein:

(a) said base is ammonium hydroxide, potassium hydroxide, or sodium hydroxide;
(b) said hypertonic saline solution comprises sodium chloride; and/or
(c) said detergent is or comprises deoxycholate or deoxycholic acid.

```
                    ┌─────────────────────┐
                    │   Ground Placenta   │
                    └─────────────────────┘
          ┌────────────────┼─────────────────┐
 ┌─────────────┐   ┌─────────────┐   ┌─────────────┐
 │  Salt Wash  │   │  Salt Wash  │   │  Salt Wash  │
 └─────────────┘   └─────────────┘   └─────────────┘
        │                 │                 │
 ┌─────────────┐   ┌─────────────┐   ┌─────────────┐
 │Detergent Wash│  │  NaOH Wash  │   │Detergent Wash│
 └─────────────┘   └─────────────┘   └─────────────┘
        │                 │                 │
 ┌─────────────┐   ┌─────────────┐   ┌─────────────┐
 │  NaOH Wash  │   │ Water Wash  │   │ Water Wash  │
 └─────────────┘   └─────────────┘   └─────────────┘
        │                 │                 │
 ┌─────────────┐  ┌──────────────────┐ ┌──────────────────┐
 │ Water Wash  │  │ECM-2,3,4 (6-10 gms)│ │ ECM-5 (10 gms)  │
 └─────────────┘  └──────────────────┘ └──────────────────┘
        │
 ┌──────────────┐
 │ ECM-1 (6 gms)│
 └──────────────┘
```

# FIG. 1

40

FIG. 2A

FIG. 2B

Secretion of MCP-1

FIG. 2C

Europäisches
Patentamt

European
Patent Office

Office européen
des brevets

# EUROPEAN SEARCH REPORT

Application Number

EP 20 20 4692

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/104539 A1 (DANIEL JOHN [US] ET AL) 29 April 2010 (2010-04-29) | 1-6, 10-14 | INV. A61K35/50 A61K35/12 A61P17/02 C07K14/78 |
| Y | * the whole document * | 7-9 | |
| Y | ALTERIO DANIELA ET AL: "CANCER TREATMENT-INDUCED ORAL MUCOSITIS", ANTICANCER RESEARCH - INTERNATIONAL JOURNAL OF CANCER RESEARCH AND TREATMENT, INTERNATIONAL INSTITUTE OF ANTICANCER RESEARCH, GR, vol. 27, no. 2, 1 March 2007 (2007-03-01), pages 1105-1125, XP008079451, ISSN: 0250-7005 * the whole document * | 7-9 | |
| X | WO 2008/057162 A2 (ANTHROGENESIS CORP [US]; BHATIA MOHIT [US] ET AL.) 15 May 2008 (2008-05-15) | 1-6, 10-12,14 | |
| Y | * the whole document * | 7-9 | |
| X | US 2012/128626 A1 (SMITH JEFFERY T L [US]) 24 May 2012 (2012-05-24) * the whole document * | 13,14 | TECHNICAL FIELDS SEARCHED (IPC) A61K A61P |
| X | US 2004/048796 A1 (HARIRI ROBERT J [US] ET AL) 11 March 2004 (2004-03-11) * the whole document * | 13,14 | |

The present search report has been drawn up for all claims

| Place of search | Date of completion of the search | Examiner |
|---|---|---|
| Munich | 12 May 2021 | Greif, Gabriela |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...........................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 20 20 4692

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2021

| Patent document cited in search report | Publication date | Patent family member(s) | Publication date |
|---|---|---|---|
| US 2010104539 A1 | 29-04-2010 | CA 2736663 A1 | 12-03-2009 |
| | | EP 2197270 A1 | 23-06-2010 |
| | | EP 3189731 A1 | 12-07-2017 |
| | | EP 3689421 A1 | 05-08-2020 |
| | | US 2010104539 A1 | 29-04-2010 |
| | | US 2012078378 A1 | 29-03-2012 |
| | | US 2012294908 A1 | 22-11-2012 |
| | | US 2012294909 A1 | 22-11-2012 |
| | | US 2012294910 A1 | 22-11-2012 |
| | | US 2013129836 A1 | 23-05-2013 |
| | | US 2013197665 A1 | 01-08-2013 |
| | | US 2013224159 A1 | 29-08-2013 |
| | | US 2013317624 A1 | 28-11-2013 |
| | | US 2014214176 A1 | 31-07-2014 |
| | | US 2014234387 A1 | 21-08-2014 |
| | | US 2014290837 A1 | 02-10-2014 |
| | | US 2014322289 A1 | 30-10-2014 |
| | | US 2016030633 A1 | 04-02-2016 |
| | | US 2016038638 A1 | 11-02-2016 |
| | | US 2018085404 A1 | 29-03-2018 |
| | | US 2021093672 A1 | 01-04-2021 |
| | | WO 2009033160 A1 | 12-03-2009 |
| WO 2008057162 A2 | 15-05-2008 | AU 2007318210 A1 | 15-05-2008 |
| | | CA 2665369 A1 | 15-05-2008 |
| | | CN 101622007 A | 06-01-2010 |
| | | CN 104623642 A | 20-05-2015 |
| | | CN 104623643 A | 20-05-2015 |
| | | EP 2076279 A2 | 08-07-2009 |
| | | EP 2664341 A2 | 20-11-2013 |
| | | EP 2664342 A2 | 20-11-2013 |
| | | ES 2516698 T3 | 31-10-2014 |
| | | HK 1127561 A1 | 02-10-2009 |
| | | IL 198015 A | 29-08-2013 |
| | | IL 216687 A | 31-10-2013 |
| | | IL 227835 A | 31-07-2016 |
| | | IL 227836 A | 31-05-2016 |
| | | JP 5769925 B2 | 26-08-2015 |
| | | JP 2010505857 A | 25-02-2010 |
| | | JP 2015211858 A | 26-11-2015 |
| | | JP 2017070761 A | 13-04-2017 |
| | | JP 2019130384 A | 08-08-2019 |
| | | KR 20090120451 A | 24-11-2009 |
| | | KR 20140070667 A | 10-06-2014 |
| | | KR 20160093739 A | 08-08-2016 |
| | | KR 20180086533 A | 31-07-2018 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 20 20 4692

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2021

| Patent document cited in search report | | Publication date | Patent family member(s) | | Publication date |
|---|---|---|---|---|---|
| | | | KR | 20190112868 A | 07-10-2019 |
| | | | KR | 20210018526 A | 17-02-2021 |
| | | | MX | 354253 B | 20-02-2018 |
| | | | NZ | 576372 A | 24-02-2012 |
| | | | NZ | 597223 A | 27-09-2013 |
| | | | NZ | 612094 A | 27-02-2015 |
| | | | US | 2008181935 A1 | 31-07-2008 |
| | | | US | 2011306755 A1 | 15-12-2011 |
| | | | US | 2012225484 A1 | 06-09-2012 |
| | | | US | 2013071362 A1 | 21-03-2013 |
| | | | US | 2013172531 A1 | 04-07-2013 |
| | | | US | 2013231288 A1 | 05-09-2013 |
| | | | US | 2014011743 A1 | 09-01-2014 |
| | | | US | 2017096473 A1 | 06-04-2017 |
| | | | US | 2019089124 A1 | 21-03-2019 |
| | | | WO | 2008057162 A2 | 15-05-2008 |
| | | | ZA | 200902485 B | 28-07-2010 |
| US 2012128626 | A1 | 24-05-2012 | AU | 2011332810 A1 | 13-06-2013 |
| | | | AU | 2011332817 A1 | 13-06-2013 |
| | | | AU | 2017203536 A1 | 15-06-2017 |
| | | | AU | 2019250228 A1 | 07-11-2019 |
| | | | CA | 2818813 A1 | 31-05-2012 |
| | | | CA | 2818814 A1 | 31-05-2012 |
| | | | DK | 2643018 T3 | 18-01-2021 |
| | | | EP | 2643016 A2 | 02-10-2013 |
| | | | EP | 2643018 A2 | 02-10-2013 |
| | | | US | 2012128626 A1 | 24-05-2012 |
| | | | US | 2012189629 A1 | 26-07-2012 |
| | | | US | 2015368337 A1 | 24-12-2015 |
| | | | US | 2016340423 A1 | 24-11-2016 |
| | | | US | 2019023780 A1 | 24-01-2019 |
| | | | US | 2020140539 A1 | 07-05-2020 |
| | | | WO | 2012071554 A2 | 31-05-2012 |
| | | | WO | 2012071561 A2 | 31-05-2012 |
| US 2004048796 | A1 | 11-03-2004 | AU | 2003226018 A1 | 13-10-2003 |
| | | | AU | 2008202604 A1 | 03-07-2008 |
| | | | AU | 2011201662 A1 | 12-05-2011 |
| | | | CA | 2479903 A1 | 09-10-2003 |
| | | | CN | 1791331 A | 21-06-2006 |
| | | | DK | 2700309 T3 | 16-09-2019 |
| | | | DK | 2702871 T3 | 18-02-2019 |
| | | | EP | 1575572 A2 | 21-09-2005 |
| | | | EP | 2700309 A1 | 26-02-2014 |
| | | | EP | 2702871 A1 | 05-03-2014 |

EPO FORM P0459

For more details about this annex : see Official Journal of the European Patent Office, No. 12/82

page 2 of 3

**ANNEX TO THE EUROPEAN SEARCH REPORT
ON EUROPEAN PATENT APPLICATION NO.**

EP 20 20 4692

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2021

| Patent document cited in search report | Publication date | Patent family member(s) | Publication date |
|---|---|---|---|
| | | EP 3412322 A1 | 12-12-2018 |
| | | ES 2526088 T3 | 05-01-2015 |
| | | ES 2710024 T3 | 22-04-2019 |
| | | HK 1085384 A1 | 25-08-2006 |
| | | IL 164220 A | 31-10-2012 |
| | | JP 4615223 B2 | 19-01-2011 |
| | | JP 5931327 B2 | 08-06-2016 |
| | | JP 5993387 B2 | 14-09-2016 |
| | | JP 2006507851 A | 09-03-2006 |
| | | JP 2010265298 A | 25-11-2010 |
| | | JP 2014138590 A | 31-07-2014 |
| | | KR 20050002906 A | 10-01-2005 |
| | | MX PA04009188 A | 20-06-2005 |
| | | NZ 535799 A | 25-06-2010 |
| | | PT 2702871 T | 01-02-2019 |
| | | US 2003187515 A1 | 02-10-2003 |
| | | US 2004048796 A1 | 11-03-2004 |
| | | WO 03082201 A2 | 09-10-2003 |
| | | ZA 200407650 B | 26-07-2006 |

EPO FORM P0459

For more details about this annex : see Official Journal of the European Patent Office, No. 12/82

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61734665
- US 4852640 A
- US 5428022 A
- US 5660692 A
- US 5008116 A
- US 5880242 A
- US 6117979 A
- US 20030049301
- US 20040048796
- US 20030187515 A
- US 20040048796 A
- US 4511653 A
- US 4582640 A
- US 5436135 A
- US 6548077 B
- US 4185011 A
- US 4597762 A
- US 5412076 A
- US 5763579 A
- US 4642117 A
- US 7045148 B
- US 7468276 B
- US 8057788 B
- US 8202703 B

**Non-patent literature cited in the description**

- **SONIS et al.** *Cancer,* 1999, vol. 85 (10), 2103-2113
- **MCPHERSON et al.** *J. Biomedical Materials Res.,* 1986, vol. 20, 79-92
- **ZEEMAN et al.** *J Biomed Mater Res.,* 2000, vol. 51 (4), 541-8
- **WACHEM et al.** *J Biomed Mater Res.,* 2000, vol. 53 (1), 18-27
- **VAN WACHEM et al.** *J Biomed Mater Res.,* 1999, vol. 47 (2), 270-7
- **ZEEMAN et al.** *J Biomed Mater Res.,* 1999, vol. 46 (3), 424-33
- **ZEEMAN et al.** *Biomaterials,* 1999, vol. 20 (10), 921-31
- **ORBAN et al.** *J. Biomedical Materials Res.,* 2004, vol. 68 (4), 756-62
- **COTORRUELO et al.** *Clin. Lab.,* 2002, vol. 48 (5 6), 271-81
- **MAINE et al.** *Expert Rev. Mol. Diagn.,* 2001, vol. 1 (1), 19-29
- **NIELSEN et al.** *J. Clin. Microbiol.,* 1987, vol. 25 (8), 1406-10
- **LAYNE, E.** Spectrophotometric and Turbidimetric Methods for Measuring Proteins. *Methods in Enzymology,* 1957, vol. 3, 447-455
- **STOSCHECK, C M.** Quantitation of Protein. *Methods in Enzymology,* 1990, vol. 182, 50-69
- **SCOPES, R K.** *Analytical Biochemistry,* 1974, vol. 59, 277
- **STOSCHECK, C M.** Quantitation of Protein. *Methods in Enzymology,* 1990, vol. 182, 50-69
- **BRADFORD, M.** *Analytical Biochemistry,* 1976, vol. 72, 248
- **WINKLEMAN, J.** *Cancer Research,* 1962, vol. 22, 589-596
- Biomaterials. Stockton Press, 1991, 55-122
- The Pharmacological Basis of Therapeutics. Macmillan Publishing
- **KATZUNG.** Basic & Clinical Pharmacology. Appleton & Lang, 1995
- **GRAHAM.** *Med. Device Technol.,* 1998, vol. 9 (1), 18-22
- **PEPPAS et al.** *Eur. J. Pharm. Biopharm.,* 2000, vol. 50 (1), 27-46
- **NGUYEN et al.** *Biomaterials,* 2002, vol. 23 (22), 4307-14
- **HENINCL et al.** *Adv. Drug Deliv. Rev,* 2002, vol. 54 (1), 13-36
- **SKELHORNE et al.** *Med. Device. Technol.,* 2002, vol. 13 (9), 19-23
- **SCHMEDLEN et al.** *Biomaterials,* 2002, vol. 23, 4325-32
- **KAMARCH.** *Methods Enzymol,* 1987, vol. 151, 150-165